# EUROPEAN PATENT APPLICATION

(11) **EP 4 582 804 A1**
(43) Date of publication of application: **09.07.2025**
(21) Application number: 23859335.4
(22) Date of filing: 29.08.2023
(51) Int. Cl.: G01N 33/53, C12Q 1/02

(54) **METHOD FOR SCREENING AND PREPARING IMMUNOMODULATOR**

(30) Priority: 29.08.2022 CN 202211043007
(71) Applicant: Jiangsu Hengrui Pharmaceuticals Co., Ltd., Lianyungang, Jiangsu 222047 (CN); Shanghai Shengdi Pharmaceutical Co., Ltd, Shanghai 201210 (CN)
(72) Inventor: ZHANG, Chongqi, Shanghai 201210 (CN); WANG, Huan, Shanghai 201210 (CN); GAO, Han, Shanghai 201210 (CN); LIN, Yuan, Shanghai 201210 (CN); LIN, Kan, Shanghai 201210 (CN); LIAO, Cheng, Shanghai 201210 (CN)
(74) Representative: Dragotti & Associati S.R.L.
(86) International application number: PCT/CN2023/115456
(87) International publication number: WO 2024/046305

(57) **Abstract**

The present disclosure provides a method for screening and preparing an immunomodulator. The present disclosure further relates to a use of an immunosuppressant in the treatment of immune-related diseases.

## Description

The present disclosure claims priority to Chinese Patent Application No. CN202211043007.2, titled "METHOD FOR SCREENING AND PREPARING IMMUNOMODULATOR" and filed on August 29, 2022, which is incorporated herein by reference in its entirety.

### TECHNICAL FIELD

The present disclosure relates to the field of biopharmaceuticals and particularly to screening and preparation methods for an immunomodulator and use of the immunomodulator in the treatment of diseases (particularly autoimmune diseases).

### BACKGROUND

Autoimmune diseases such as systemic lupus erythematosus (SLE), lupus nephritis, and multiple sclerosis mostly involve the production of autoantibodies against the body's own tissue antigens. The autoantibodies induce systemic immune responses that finally affect different organs, thereby causing corresponding disease symptoms. From a biological mechanism perspective, a series of important biological processes-the differentiation of B cells into plasma cells, the production and maintenance of plasma cells, and the secretion of antibodies by plasma cells-play a central role in the development and progression of autoimmune diseases. Thus, numerous drugs targeting B cells and plasma cells have entered clinical trials. Belimumab (trade name Benlysta), which has been approved, is a specific inhibitor of the B-lymphocyte stimulator (BLyS, also known as BAFF), and it is used to treat active, autoantibody-positive systemic lupus erythematosus in patients undergoing standard therapy. In addition, telitacicept (RC18, trade name: Tai'ai) is a TACI-Ig fusion protein that targets both BLyS and APRIL to inhibit B cells and plasma cells, and it was approved by the Chinese National Medical Products Administration in 2021 for the treatment of systemic lupus erythematosus.

B cell differentiation is mainly divided into several stages, including five stages: precursor B cells, immature B cells, mature B cells, activated B cells, and plasma cells. The first three stages are antigen-independent and primarily occur in the bone marrow. Once B cells mature, they migrate to peripheral immune organs and, after antigen stimulation, are activated, thereby proliferating and further differentiating into memory B cells or plasma cells.

In the development of new drugs for autoimmune diseases, particularly SLE, related therapies targeting B cells are one of the major focuses. Establishing experimental methods for evaluating B cell-related biological functions is a key step in the development of related drugs for autoimmune diseases. Therefore, establishing a convenient, efficient, and stable experimental system for evaluating B cell-related functions is helpful in facilitating the development of new drugs for autoimmune diseases.

Existing experiments for evaluating B cell-related functions primarily involve the 3H-labeled thymidine (TdR) method, in which TdR is labeled with the isotope 3H. As a precursor in DNA synthesis, 3H-TdR can be incorporated in the DNA synthesis and metabolism processes. As a result, every newly synthesized double-stranded DNA gives radioactive signals. By measuring the radioactivity of cells, one can assess DNA metabolism and cell proliferation. However, this method is mainly used to evaluate B cell proliferation and must be carried out in a certified, safe laboratory, and the laboratory technicians must also undergo strict specialized training. The equipment for measuring radioactivity is relatively expensive, and there are also radionuclide contamination problems. Therefore, these B cell-related proliferation experiments cannot be carried out in common laboratories, thus limiting the widespread use of the method.

In the present disclosure, a simple and efficient experimental system for the differentiation of B cells into plasma cells was developed through a series of explorations of the experimental conditions. As demonstrated by related drugs that are already on the market or in late-phase clinical trials, this method is convenient and feasible and can be used for screening for immunomodulators that can be used in drug preparation.

### SUMMARY

The present disclosure provides screening and preparation methods for an immunomodulator and use of the immunomodulator in the preparation of a medicament for treating or ameliorating diseases.

### Screening and Preparation Methods for Immunomodulator

The present disclosure provides a method for promoting B cell differentiation. In addition, the present disclosure provides screening and preparation methods for an immunomodulator. The methods may be performed *in vitro* or *in vivo.*

In some embodiments, a method for promoting B cell differentiation and screening and preparation methods for an immunomodulator are provided, the methods comprising administering to B cells a TLR agonist, and/or an interleukin, and/or retinoic acid (RA), and/or a tumor necrosis factor family member. For example, the TLR agonist is selected from the group consisting of a TLR1 agonist, a TLR2 agonist, a TLR3 agonist, a TLR4 agonist, a TLR5 agonist, a TLR6 agonist, a TLR7 agonist, a TLR8 agonist (e.g., R848), and a TLR9 agonist (e.g., a CpG-ODN, e.g., CpG-A or CpG-B). For example, the interleukin is selected from the group consisting of IL-3, IL-4, IL-6, IL-10, and IL-21. For example, the tumor necrosis factor family member is BAFF or APRIL.

In some embodiments, the TLR agonist may be selected from the group consisting of Heplisav, SD-101, resiquimod, Dynavax, DV-281, imiquimod, cobitolimod, entolimod, lefitolimod, Poly-ICLC, Grass MATA MPL, G-100, AST-008, GSK-1795091, tilsotolimod, KMRC-011, CMB-305, rintatolimod, AZD-1419, influenza-PAL, SAR-439794, MIS-416, MGN-1601, GSK-2245035, VTX-1463, motolimod, GS-9688, LHC-165, BDB-001, PGV-001, AV-7909, DSP-0509, DPX-E7, RG-7854, telratolimod, vesatolimod, poly-ICLC adjuvanted vaccines, MVAME-03, Riboxxim, G-305, PUL-042, litenimod, DRibbles vaccine, TMX-202, ISA-201, PEPA-10, AL-034, CpG-ODN(K3), Vaxart, CBLB-612, Pseudomonas aeruginosa, IR-103, VAX-161, VAX-125, MEDI19197, R848, CpG-A, and CpG-B and complexes thereof and pharmaceutically acceptable salts thereof.

In a first aspect, in some embodiments of the present disclosure, a method for promoting B cell differentiation is provided, the method comprising administering to B cells:
a) IL-3 and CpG-A;
b) IL-3 and CpG-A and IL-21;
c) IL-3 and CpG-A and IFNα; or
d) IL-3 and CpG-A and IFNα and IL-21;
the method can increase the proportion of B cells differentiating into plasma cells, and/or increase B cell viability.

In some embodiments, each of a)-d) can increase the proportion of B cells differentiating into plasma cells, and d) can increase B cell viability.

In the aforementioned method, IL-3 is at a concentration of 0.1-50 ng/mL, 1-30 ng/mL, or 5-20 ng/mL, e.g., about 5, about 6, about 7, about 8, about 9, about 10, about 11, about 12, about 13, about 14, or about 15 ng/mL;
CpG-A is at a concentration of 0.01-10 µM, 0.05-5 µM, 0.1-2 µM, or 0.2-1 µM, e.g., about 0.2, about 0.3, about 0.4, about 0.5, about 0.6, about 0.7, about 0.8, about 0.9, or about 1 µM;
IFNα is at a concentration of 1-10,000 U/mL, 100-5000 U/mL, or 500-2000 U/mL, e.g., about 500, about 600, about 700, about 800, about 900, about 1000, about 1100, about 1200, about 1300, about 1400, about 1500 U/mL;
IL-21 is at a concentration of 0.1-1000 ng/mL, 1-200 ng/mL, 10-100 ng/mL, or 20-50 ng/mL, e.g., about 6.6, about 16.5, about 20, about 25, about 30, about 33, about 35, about 40, about 45, about 50, or about 66 ng/mL.

In some embodiments, the method comprises administering to B cells:
a-1) 5-20 ng/mL IL-3 and 0.2-1 µM CpG-A;
b-1) 5-20 ng/mL IL-3 and 0.2-1 µM CpG-A and 16.5-66 ng/mL IL-21;
c-1) 5-20 ng/mL IL-3 and 0.2-1 µM CpG-A and 500-2000 U/mL IFNα; or
d-1) 5-20 ng/mL IL-3 and 0.2-1 µM CpG-A and 500-2000 U/mL IFNα and 16.5-66 ng/mL IL-21.

In some embodiments, the method comprises administering to B cells:
a-2) about 10 ng/mL IL-3 and about 0.5 µM CpG-A;
b-2) about 10 ng/mL IL-3 and about 0.5 µM CpG-A and about 33 ng/mL IL-21;
c-2) about 10 ng/mL IL-3 and about 0.5 µM CpG-A and about 1000 U/mL IFNα; or
d-2) about 10 ng/mL IL-3 and about 0.5 µM CpG-A and about 1000 U/mL IFNα and about 33 ng/mL IL-21.

In some embodiments, a screening method for an immunomodulator is provided, the method comprising the following steps:
step 1) administering to B cells a TLR agonist and culturing for M days, wherein M is selected from an integer of 1-28;
step 2) removing the TLR agonist;
step 3) adding one or more interleukins and one or more TNF family members and culturing for N days, wherein N is selected from an integer of 1-28;
step 4) adjusting concentrations of the TNF family members and continuing culturing; step 5) adding a test sample; and
step 6) determining a B cell differentiation level;
wherein the step 5) is performed concurrently with, during, or after any one of the steps 1) to 4) described above.

In some embodiments, a screening method for an immunomodulator is provided, the method comprising the following steps:
step 1) administering to B cells a TLR agonist and an IFN and culturing for M days, wherein M is selected from an integer of 1-28;
step 2) removing the TLR agonist and the IFN;
step 3) adding one or more interleukins and one or more TNF family members and culturing for N days, wherein N is selected from an integer of 1-28;
step 4) adjusting concentrations of the TNF family members and continuing culturing; step 5) adding a test sample; and
step 6) determining a B cell differentiation level;
wherein the step 5) is performed concurrently with, during, or after any one of the steps 1) to 4) described above.

In some embodiments, the method further comprises:
step 7) selecting a test sample capable of activating or inhibiting B cell differentiation as an immunomodulator, wherein optionally, the step 6) comprises determining a CD27⁺ CD38⁺ B cell percentage to determine the B cell differentiation level.

In some embodiments, the TLR agonist is selected from any one or more of TLR7, TLR8, and TLR9 agonists; illustratively, the TLR agonist is selected from any one or more of R848, CpG, and LPS; illustratively, the TLR agonist is CpG-A or CpG-B; more illustratively, the TLR agonist is CpG-B;
when present, the IFN is IFNα or IFNβ, illustratively IFNα;
the interleukin is selected from the group consisting of one or more of IL-3, IL-6, IL-10, and IL-21; illustratively, the interleukin is a combination of IL-6, IL-10, and IL-21;
the TNF family members are selected from any one or more of BAFF and APRIL; illustratively, the TNF family members are a combination of BAFF and APRIL.

In some embodiments, when the TNF family members are BAFF and APRIL, adjusting the concentrations of the TNF family members comprises reducing a BAFF concentration and/or increasing an APRIL concentration;
illustratively, a BAFF concentration in step 4) is reduced to be 0.01-0.9 times a BAFF concentration added in step 3); more illustratively, a BAFF concentration in step 4) is reduced to be 0.02-0.5 times a BAFF concentration added in step 3); more illustratively, a BAFF concentration in step 4) is reduced to be about 0.02, about 0.04, about 0.05, about 0.06, about 0.07, about 0.08, about 0.09, about 0.1, about 0.11, about 0.12, about 0.13, about 0.14, about 0.15, about 0.2, about 0.3, about 0.4, about 0.5, about 0.6, about 0.7, about 0.8, or about 0.9 times a BAFF concentration added in step 3);
illustratively, an APRIL concentration in step 4) is increased to be 1.1-100 times an APRIL concentration added in step 3); more illustratively, an APRIL concentration in step 4) is increased to be 5-50 times an APRIL concentration added in step 3); more illustratively, an APRIL concentration in step 4) is increased to be about 1.5, about 2, about 3, about 4, about 5, about 6, about 7, about 8, about 9, about 10, about 12, about 15, about 20, about 25, about 30, about 35, about 40, or about 50 times an APRIL concentration added in step 3).

In some embodiments, a screening method for an immunomodulator is provided, the method comprising:
1) administering to B cells a TLR agonist and an interferon;
2) adding a test sample on any one of days 0-21 and continuing culturing; and
3) determining a B cell differentiation level;
illustratively, the method further comprises:
4) selecting a test sample capable of activating or inhibiting B cell differentiation as an immunomodulator.

In some embodiments, the TLR agonist is selected from the group consisting of one or more of TLR7, TLR8, and TLR9 agonists; illustratively, the TLR agonist is selected from the group consisting of one or more of R848 and CpG; illustratively, the TLR agonist is CpG-A or CpG-B; more illustratively, the TLR agonist is CpG-B;
the IFN is IFNα or IFNβ, illustratively IFNα.

In some embodiments, a screening method for an immunomodulator is provided, the method comprising:
1) administering to the B cells 0.1-20 µg/mL CpG-B and 50-1000 U/mL IFNα;
2) adding a test sample on any one of days 0-21 and continuing culturing; and
3) determining a B cell differentiation level;
illustratively, the method further comprises:
4) selecting a test sample capable of activating or inhibiting B cell differentiation as an immunomodulator;
illustratively, the method comprises step 1) administering to the B cells 0.5-5 µg/mL CpG-B and 100-800 U/mL IFNα;
more illustratively, the method comprises step 1) administering to the B cells about 1-2 µg/mL CpG-B and 250-500 U/mL IFNα.

In some embodiments, the immunomodulator is a type I interferon pathway modulator and/or a TNF pathway modulator;
illustratively, the type I interferon pathway modulator is an IFNAR1 signaling pathway modulator;
illustratively, the TNF pathway modulator is a BAFF and/or ARRIL pathway modulator.

In some embodiments, activating or inhibiting B cell differentiation is achieved by determining a number or proportion of plasma cells differentiated from the B cells, and the plasma cells are illustratively CD27⁺ CD38⁺ B cells.

In some embodiments, the B cells are at a concentration of 1 × 10⁵/well in a 96-well plate.

In some embodiments, the B cells are obtained by the following steps:
1) separating B cells from PBMCs, and
2) culturing in a 1640 culture medium containing 50 µM β-mercaptoethanol, 100 µM MEM non-essential amino acids, and sodium pyruvate, the 1640 culture medium containing GlutaMAX.

In some embodiments, the immunomodulator is selected from the group consisting of a protein or polypeptide, a nucleic acid, an aptamer, a small-molecule compound, and a molecule comprising the protein or polypeptide, the nucleic acid, the aptamer, or the small-molecule compound; the protein or polypeptide is illustratively an antibody or an antigen-binding fragment thereof, a cytokine, a receptor, or a ligand;
illustratively, the immunomodulator is used for treating a B cell disorder or an autoimmune disease;
more illustratively, the B cell disorder or the autoimmune disease is a disease or disorder associated with TACI and/or BCMA expression;
more illustratively, the autoimmune disease is selected from the group consisting of systemic lupus erythematosus, myasthenia gravis, multiple sclerosis, insulin-dependent diabetes mellitus, Crohn's disease, rheumatoid arthritis, polyarticular juvenile rheumatoid arthritis, and psoriatic arthritis; the B cell disorder is selected from the group consisting of tumors, chronic leukocytic leukemia, multiple myeloma, non-Hodgkin lymphoma, post-transplant lymphoproliferative disorder, and light chain gammopathy.

In a second aspect, in some embodiments of the present disclosure, a method for promoting B cell differentiation is provided, the method comprising administering to B cells:
e) CpG-B and IFNα;
f) R484 and IFNα;
g) CpG-B and BAFF and APRIL;
h) R484 and BAFF and APRIL;
i) CpG-B and IFNα and RA and BAFF and APRIL; or
j) R484 and IFNα and RA and BAFF and APRIL;
the method can increase the proportion of B cells differentiating into plasma cells, and/or increase B cell viability.

In some embodiments, e), f), i), and j) can improve the proportion of B cells differentiating into plasma cells, and each of e)-j) can increase B cell viability, wherein i) and j) increase B cell viability to a greater extent than g) and h).

In the aforementioned method, CpG-B is at a concentration of 0.1-100 µg/mL, 0.2-20 µg/mL, 0.5-10 ug/mL, or 0.5-5 µg/mL, e.g., about 0.5, about 0.6, about 0.7, about 0.8, about 0.9, about 1, about 1.1, about 1.2, about 1.3, about 1.4, about 1.5, or about 2 µg/mL;
IFNα is at a concentration of 1-10,000 U/mL, 100-2000 U/mL, 200-800 U/mL, or 100-500 U/mL, e.g., about 50, about 100, about 150, about 200, about 250, about 300, about 400, about 500, about 600, about 700, about 800, about 900, or about 1000 U/mL; R484 is at a concentration of 0.1-100 µg/mL, 0.2-20 µg/mL, 0.5-10 µg/mL, or 0.5-5 µg/mL, e.g., about 0.5, about 0.6, about 0.7, about 0.8, about 0.9, about 1, about 1.1, about 1.2, about 1.3, about 1.4, about 1.5, or about 2 µg/mL;
RA is at a concentration of 0.1-50 µg/mL, 0.2-20 µg/mL, 0.2-10 µg/mL, or 1-5 µg/mL, e.g., about 1, about 2, about 3, about 4, about 5, about 6, about 7, about 8, about 9, or about 10 µg/mL;
BAFF and APRIL are at a concentration of 1-10,000 ng/mL, 100-5000 ng/mL, or 200-1000 ng/mL, e.g., about 200, about 300, about 400, about 500, about 600, about 7000, about 800, about 900, or about 1000 ng/mL; or BAFF and APRIL are at a concentration of 0.1-100 nM, 0.5-20 nM, or 1-10 nM, e.g., about 1, about 2, about 3, about 4, about 5, about 6, about 7, about 8, about 9, or about 10 nM;
In some embodiments, the method comprises administering to B cells:
   e-1) 0.5-2 µg/mL CpG-B and 200-800 U/mL IFNα; or about 1-3 µg/mL CpG-B and 100-500 U/mL IFNα;
   f-1) 0.5-2 µg/mL R484 and about 200-800 U/mL IFNα; or 1-3 µg/mL R484 and about 100-500 U/mL IFNα;
   g-1) 0.5-2 µg/mL CpG-B and 200-1000 ng/mL BAFF and 200-1000 ng/mL APRIL;
   h-1) 0.5-2 µg/mL R484 and 200-1000 ng/mL BAFF and 200-1000 ng/mL APRIL;
   i-1) 0.5-2 µg/mL CpG-B and 200-1000 U/mL IFNα and 2-5 µg/mL RA and 200-1000 ng/mL BAFF and 200-1000 ng/mL APRIL; or, about 1-3 µg/mL CpG-B and 100-500 U/mL IFNα and 2-5 µg/mL RA and 200-1000 ng/mL BAFF and 200-1000 ng/mL APRIL; or
   j-1) 0.5-2 µg/mL R484 and 200-1000 U/mL IFNα and 2-5 µg/mL RA and 200-1000 ng/mL BAFF and 200-1000 ng/mL APRIL; or, about 1-3 µg/mL R484 and 100-500 U/mL IFNα and 2-5 µg/mL RA and 200-1000 ng/mL BAFF and 200-1000 ng/mL APRIL.

In some embodiments, the method comprises administering to B cells:
e-2) about 1 µg/mL CpG-B and about 500 U/mL IFNα; or about 2 µg/mL CpG-B and 250 U/mL IFNα;
f-2) about 1 µg/mL R484 and about 500 U/mL IFNα; or about 1 µg/mL R484 and about 250 U/mL IFNα;
g-2) about 1 µg/mL or about 2 µg/mL CpG-B and 500 ng/mL BAFF and 500 ng/mL APRIL;
h-2) about 1 µg/mL or about 2 µg/mL R484 and 500 ng/mL BAFF and 500 ng/mL APRIL;
i-2) about 1 µg/mL or about 2 µg/mL CpG-B and about 500 U/mL IFNα and about 3 µg/mL RA and about 500 ng/mL BAFF and about 500 ng/mL APRIL; or, about 1 µg/mL or about 2 µg/mL CpG-B and 250 U/mL IFNα and about 3 µg/mL RA and about 500 ng/mL BAFF and about 500 ng/mL APRIL; or
j-2) about 1 µg/mL or about 2 µg/mLR484 and about 500 U/mL IFNα and about 3 µg/mL RA and about 500 ng/mL BAFF and about 500 ng/mL APRIL; or, about 1 µg/mL or about 2 µg/mL R484 and 250 U/mL IFNα and about 3 µg/mL RA and about 500 ng/mL BAFF and about 500 ng/mL APRIL;

In the preceding embodiments, after factors are added for stimulation, B cell differentiation is assessed (e.g., determining the proportion of differentiated plasma cells (CD27⁺ CD38⁺)) on any day, e.g., on day 1, day 2, day 3, day 4, day 5, or day 6, e.g., on day 4.

In some embodiments, a method for inducing B cell differentiation is provided, the method comprising a step of administering to B cells a TLR agonist and an IFN,
wherein the TLR agonist is one or more of TLR7, TLR8, and TLR9 agonists;
illustratively, the TLR agonist is one or more of R848 or CpG; illustratively, the TLR agonist is CpG-A or CpG-B; more illustratively, the TLR agonist is CpG-B;
the IFN is IFNα or IFNβ, illustratively IFNα.

In some embodiments, a method for inducing B cell differentiation is provided, the method comprising:
administering to B cells CpG-A and IFNα, or
a step of administering to B cells CpG-B or R484 and IFNα;
optionally, the method comprises:
   administering to B cells CpG-A and IFNα, or a step of administering to B cells CpG-B or R484 and IFNα, and
   a step of administering to the B cells IL-3 and IL-21 simultaneously.

In some embodiments, a method for inducing B cell differentiation is provided, the method comprising:
administering to the B cells 5-20 ng/mL IL-3, 0.2-1 µM CpG-A, 500-2000 U/mL IFNα, and 16.5-66 ng/mL IL-21, or
administering to the B cells 0.5-5 µg/mL CpG-B or R484 and 100-800 U/mL IFNα; illustratively,
administering to the B cells about 10 ng/mL IL-3, about 0.5 µM CpG-A, about 1000 U/mL IFNα, and about 33 ng/mL IL-21, or
administering to the B cells 1-2 µg/mL CpG-B or R484 and 250-500 U/mL IFNα.

In an embodiment of the method for inducing B cell differentiation, the method further comprises adding retinoic acid to the B cells at the same time; illustratively, the retinoic acid is at a concentration of 2-5 µg/mL; more illustratively, the retinoic acid is at a concentration of about 3 µg/mL.

In some embodiments, a cell model for screening for an immunomodulator is provided, the cell model comprising B cells induced to differentiate by a method described herein. In a third aspect, in some embodiments of the present disclosure, screening and preparation methods for an immunomodulator are provided, the methods comprising administering to B cells:
k) CpG-B and IFNα and an immunosuppressant. The method can promote the differentiation of B cells into plasma cells (CD27⁺ CD38⁺).

In the preceding embodiments, CpG-B is at a concentration of 0.1-100 µg/mL, 0.2-20 µg/mL, 0.5-10 µg/mL, or 0.5-5 µg/mL, e.g., about 1, about 1.2, about 1.5, about 1.6, about 1.7, about 1.8, about 1.9, about 2, about 2.1, about 2.2, about 2.3, about 2.4, about 2.5, or about 2 µg/mL;
IFNα is at a concentration of 1-10,000 U/mL, 50-1000 U/mL, or 100-500 U/mL, e.g., about 150, about 200, about 250, about 300, about 350, about 400, or about 500 U/mL. In some embodiments, screening and preparation methods for an immunomodulator are provided, the methods comprising administering to B cells:
k-1) 1-5 µg/mL CpG-B and 100-500 U/mL IFNα and gradient concentrations of the immunomodulator;
k-2) about 2 µg/mL CpG-B and about 250 U/mL IFNα and gradient concentrations of the immunomodulator; or
k-3) about 1 µg/mL CpG-B and about 500 U/mL IFNα and gradient concentrations of the immunomodulator.

In a fourth aspect, in some embodiments of the present disclosure, a screening, preparation, or production method for an immunomodulator is provided, the method comprising:
an induction step: administering to B cells a TLR agonist; removing the TLR agonist on day M; adding an interleukin; adding a TNF family member; and adjusting a concentration of the TNF family member N days after the TNF family member is added; and
an assessment step: during the induction step or after the induction step, adding a test sample and assessing B cell differentiation; and selecting a test sample capable of activating or inhibiting B cell differentiation as an immunomodulator;
wherein M and N are each an integer of 1-30, for example, 2, 3, 4, 5, 6, 7, 8, 9, or 10.

In some embodiments of the present disclosure, a screening, preparation, or production method for an immunomodulator is provided, the method comprising: steps 1), 6), and 7); optionally, the method may further comprise steps 2)-3); optionally, the method may further comprise steps 4)-5):
step 1) administering to B cells a TLR agonist;
step 2) removing the TLR agonist on day M;
step 3) adding an interleukin;
step 4) adding TNF family members;
step 5) adjusting concentrations of the TNF family member;
step 6) adding a test sample and assessing B cell differentiation; and
step 7) selecting a test sample capable of activating or inhibiting B cell differentiation as an immunomodulator;
wherein M and N are each an integer of 1-30, for example, 2, 3, 4, 5, 6, 7, 8, 9, or 10.

In some specific embodiments, the aforementioned TLR agonist includes, but is not limited to, TLR7, TLR8, and TLR9 agonists; for example, the aforementioned TLR agonist is selected from the group consisting of R848, LPS, and CpG; for example, the aforementioned TLR agonist is selected from the group consisting of CpG-A and CpG-B; for example, the aforementioned TLR agonist is CpG-B;
for example, the aforementioned interleukin is selected from the group consisting of IL-4, IL-3, IL-6, IL-10, and/or IL-21; for example, the aforementioned interleukin is selected from the group consisting of IL-6, IL-10, and/or IL-21; for example, the aforementioned interleukin is IL-6 and IL-10 and IL-21;
the aforementioned TNF family members are molecules capable of modulating the TNF signaling pathway; for example, the aforementioned TNF family members are selected from the group consisting of BAFF and APRIL; for example, the aforementioned TNF family members are BAFF and APRIL;
the aforementioned TLR agonist is at a concentration selected from the group consisting of 0.1-100 µg/mL, 0.2-20 µg/mL, 0.5-10 µg/mL, and 0.5-5 µg/mL, e.g., about 1, about 2, about 3, about 4, or about 5 µg/mL;
adjusting the concentrations of the TNF family members in the aforementioned step 5) comprises: for example, reducing a BAFF concentration (e.g., to be 0.01-0.99 times, 0.05-0.9 times, 0.05-0.5 times, or 0.05-0.2 times, e.g., about 0.02, about 0.04, about 0.05, about 0.06, about 0.07, about 0.08, about 0.09, about 0.1, about 0.11, about 0.12, about 0.13, about 0.14, about 0.15, about 0.2, about 0.3, about 0.4, about 0.5, about 0.6, about 0.7, about 0.8, or about 0.9 times, the original concentration), and increasing an APRIL concentration (e.g., to be 1.1-1000 times, 2-100 times, 5-50 times, or 10-20 times, e.g., about 1.5, about 2, about 3, about 4, about 5, about 6, about 7, about 8, about 9, about 10, about 12, about 15, about 20, about 25, about 30, about 35, about 40, or about 50 times, the original concentration));
assessing B cell differentiation comprises, for example, counting plasma cells (CD27⁺ CD38⁺);
the aforementioned test sample is an immunomodulator (e.g., an immune agonist or an immunosuppressant) or a B cell differentiation modulator, such as a TNF pathway modulator (e.g., a BAFF and APRIL pathway agonist or inhibitor).

In some embodiments of the present disclosure, a screening, preparation, or production method for an immunomodulator is provided, the method comprising:
step 1) administering to B cells a CpG (e.g., CpG-A or CpG-B) or R848 or LPS;
step 2) removing the CpG (e.g., CpG-A or CpG-B) or R848 or LPS on day M;
step 3) adding the interleukin(s) IL-6, IL-10, and/or IL-21;
optionally step 4) adding BAFF and APRIL;
optionally step 5) adjusting concentrations of BAFF and APRIL;
optionally step 6) adding a test sample and assessing B cell differentiation; and
optionally step 7) selecting a test sample capable of activating or inhibiting B cell differentiation as an immunomodulator;
wherein M and N are each an integer of 1-30.

In some embodiments of the present disclosure, a screening, preparation, or production method for an immunomodulator is provided, the method comprising:
step 1) administering to B cells CpG-B;
step 2) removing CpG-B on day M;
step 3) adding the interleukins IL-6 and IL-10 and IL-21;
optionally step 4) adding BAFF and APRIL;
optionally step 5) reducing a BAFF concentration and increasing an APRIL concentration;
optionally step 6) adding a test sample and assessing B cell differentiation; and
optionally step 7) selecting a test sample capable of activating or inhibiting B cell differentiation as an immunomodulator;
wherein M and N are each an integer of 1-20, M is 3, 4, 5, or 6, and N is 2, 3, or 4.

In the preceding embodiments, CpG-B is at a concentration selected from the group consisting of 0.1-100 µg/mL, 0.2-20 µg/mL, 0.5-10 µg/mL, and 0.5-5 µg/mL; for example, CpG-B is at a concentration of about 0.5, about 0.8, about 1, about 1.2, about 1.4, about 1.5, about 1.6, about 1.8, about 2, about 2.2, about 2.5, about 2.8, about 3, about 3.5, about 4, about 4.5, or about 5 µg/mL;
CpG-A or LPS is at a concentration of 0.01-10 µM, 0.05-5 µM, 0.1-2 µM, or 0.2-1 µM, e.g., about 0.2, about 0.3, about 0.4, about 0.5, about 0.6, about 0.7, about 0.8, about 0.9, about 1, about 1.5, about 2, about 2.5, or about 3 µM;
R484 is at a concentration of 0.1-100 µg/mL, 0.2-20 µg/mL, 0.5-10 µg/mL, or 0.5-5 µg/mL, e.g., about 0.5, about 0.6, about 0.7, about 0.8, about 0.9, about 1, about 1.1, about 1.2, about 1.3, about 1.4, about 1.5, about 2, about 3, or about 4 µg/mL;
the interleukins (e.g., IL-3, IL-4, IL-6, IL-10, and IL-21) are at concentrations of 0.1-1000 ng/mL, 1-500 ng/mL, and 5-200 ng/mL, respectively, e.g., about 5, about 10, about 20, about 30, about 40, about 50, about 60, about 70, about 80, about 90, about 100, about 150, or about 200 ng/mL;
the TFN pathway modulators (e.g., BAFF and APRIL) are at a concentration of 1-10,000 ng/mL, 10-5000 ng/mL, 50-2000 ng/mL, or 20-200 ng/mL, e.g., about 10, about 20, about 30, about 40, about 50, about 60, about 70, about 80, about 90, about 100, about 200, about 250, about 300, about 400, about 500, about 600, about 7000, about 800, about 900, or about 1000 ng/mL.

In some embodiments of the present disclosure, a screening, preparation, or production method for an immunomodulator is provided, the method comprising:
step 1) administering to B cells 0.5-10 µg/mL CpG-B;
step 2) removing CpG-B on days 2-8;
step 3) adding 2-100 ng/mL IL-6 and 10-1000 ng/mL IL-10 and 10-1000 ng/mL IL-21; optionally step 4) adding 10-2000 ng/mL BAFF and 10-2000 ng/mL APRIL and stimulating for 2-5 days;
optionally step 5) reducing a BAFF concentration and increasing an APRIL concentration;
optionally step 6) adding a test sample on days 0-21 and assessing B cell differentiation; and
optionally step 7) selecting a test sample capable of activating or inhibiting B cell differentiation as an immunomodulator.

In some embodiments of the present disclosure, a screening, preparation, or production method for an immunomodulator is provided, the method comprising:
step 1) administering to B cells 1-5 µg/mL CpG-B;
step 2) removing CpG-B on days 3-5;
step 3) adding 5-50 ng/mL IL-6 and 100-500 ng/mL IL-10 and 100-500 ng/mL IL-21;
step 4) adding 200-1000 ng/mL BAFF and 20-200 ng/mL APRIL and stimulating for 2-4 days;
step 5) reducing the BAFF concentration to 20-200 ng/mL and increasing the APRIL concentration to 200-1000 ng/mL;
step 6) adding a test sample at gradient concentrations on days 0-14 and assessing B cell differentiation; and
step 7) selecting a test sample capable of activating or inhibiting B cell differentiation as an immunomodulator.

In some embodiments of the present disclosure, a screening, preparation, or production method for an immunomodulator is provided, the method comprising:
step 1) administering to B cells about 2 µg/mL CpG-B;
step 2) removing CpG-B on day 4 or 5;
step 3) adding about 10 ng/mL IL-6 and about 50 ng/mL IL-10 and about 50 ng/mL IL-21;
step 4) adding about 500 ng/mL BAFF and about 50 ng/mL APRIL;
step 5) after 3 days, adjusting the BAFF concentration to about 50 ng/mL and adjusting the APRIL concentration to about 500 ng/mL;
step 6) adding a test sample at gradient concentrations on days 0-14 and assessing B cell differentiation; and
step 7) selecting a test sample capable of activating or inhibiting B cell differentiation as an immunomodulator.

In the preceding embodiments, the immunomodulator includes, but is not limited to, type I interferon pathway modulators, such as IFNAR1 signaling pathway modulators; the IFNAR1 modulators include, but are not limited to, antibodies or antigen-binding fragments thereof, small-molecule compounds, nucleic acids, aptamers, receptors, ligands, and any molecule comprising the antibodies or the antigen-binding fragments thereof, the small-molecule compounds, the nucleic acids, the aptamers, the receptors, or the ligands. Exemplary immunomodulators include, but are not limited to, anifrolumab and other anti-IFNAR1 antibodies in the present disclosure, as well as fusion proteins comprising the anti-IFNAR1 antibodies (e.g., fusion proteins comprising a TACI polypeptide and a BCMA polypeptide and an anti-IFNAR1 antibody, fusion proteins comprising a TACI polypeptide and an anti-IFNAR1 antibody, and fusion proteins comprising a BCMA polypeptide and an anti-IFNAR1 antibody), and the fusion proteins comprising a TACI polypeptide and a BCMA polypeptide in the present disclosure.

In a fifth aspect, in some embodiments of the present disclosure, a screening, preparation, or production method for an immunomodulator is provided, the method comprising:
an induction step: administering to B cells a TLR agonist and an IFN; removing the TLR agonist and the IFN on day M; adding an interleukin; adding a TNF family member; and adjusting a concentration of the TNF family member N days after the TNF family member is added; and
an assessment step: during the induction step or after the induction step, adding a test sample and assessing B cell differentiation; and selecting a test sample capable of activating or inhibiting B cell differentiation as an immunomodulator;
wherein M and N are each an integer of 1-30, for example, 2, 3, 4, 5, 6, 7, 8, 9, or 10.

In some embodiments of the present disclosure, a screening, preparation, or production method for an immunomodulator is provided, the method comprising: steps 1), 6), and 7); optionally, the method may further comprise steps 2)-3); optionally, the method may further comprise steps 4)-5):
step 1) administering to B cells a TLR agonist and an IFN;
step 2) removing the TLR agonist and the IFN on day M;
step 3) adding an interleukin;
step 4) adding TNF family members;
step 5) adjusting concentrations of the TNF family member;
step 6) adding a test sample and assessing B cell differentiation; and
step 7) selecting a test sample capable of activating or inhibiting B cell differentiation as an immunomodulator;
wherein M and N are each an integer of 1-30, for example, 2, 3, 4, 5, 6, 7, 8, 9, or 10.

In some specific embodiments, the aforementioned TLR agonist includes, but is not limited to, TLR7, TLR8, and TLR9 agonists; for example, the aforementioned TLR agonist is selected from the group consisting of R848, LPS, and CpG; for example, the aforementioned TLR agonist is selected from the group consisting of CpG-A and CpG-B; for example, the aforementioned TLR agonist is CpG-B;
the aforementioned IFN is selected from the group consisting of IFNβ and IFNα;
for example, the aforementioned interleukin is selected from the group consisting of IL-4, IL-3, IL-6, IL-10, and/or IL-21; for example, the aforementioned interleukin is selected from the group consisting of IL-6, IL-10, and/or IL-21; for example, the aforementioned interleukin is IL-6 and IL-10 and IL-21;
the aforementioned TNF family members are molecules capable of modulating the TNF signaling pathway; for example, the aforementioned TNF family members are selected from the group consisting of BAFF and APRIL; for example, the aforementioned TNF family members are BAFF and APRIL;
the aforementioned TLR agonist is at a concentration selected from the group consisting of 0.1-100 µg/mL, 0.2-20 µg/mL, 0.5-10 µg/mL, or 0.5-5 µg/mL, e.g., about 1, about 2, about 3, about 4, or about 5 µg/mL;
adjusting the concentrations of the TNF family members comprises: for example, reducing a BAFF concentration (e.g., to be 0.01-0.99 times, 0.05-0.9 times, 0.05-0.5 times, or 0.05-0.2 times, e.g., about 0.02, about 0.04, about 0.05, about 0.06, about 0.07, about 0.08, about 0.09, about 0.1, about 0.11, about 0.12, about 0.13, about 0.14, about 0.15, about 0.2, about 0.3, about 0.4, about 0.5, about 0.6, about 0.7, about 0.8, or about 0.9 times, the original concentration), and increasing an APRIL concentration (e.g., to be 1.1-1000 times, 2-100 times, 5-50 times, or 10-20 times, e.g., about 1.5, about 2, about 3, about 4, about 5, about 6, about 7, about 8, about 9, about 10, about 12, about 15, about 20, about 25, about 30, about 35, about 40, or about 50 times, the original concentration));
assessing B cell differentiation comprises, for example, counting plasma cells (CD27⁺ CD38⁺);
the aforementioned test sample is an immunomodulator (e.g., an immune agonist or an immunosuppressant) or a B cell differentiation modulator, such as a TNF pathway modulator (e.g., a BAFF and APRIL pathway agonist or inhibitor).

In some embodiments of the present disclosure, a screening, preparation, or production method for an immunomodulator is provided, the method comprising:
step 1) administering to B cells a CpG (e.g., CpG-A or CpG-B) or R848 or LPS and IFNα;
step 2) removing the CpG (e.g., CpG-A or CpG-B) or R848 or LPS and IFNα on day M;
step 3) adding the interleukin(s) IL-6, IL-10, and/or IL-21;
optionally step 4) adding BAFF and APRIL;
optionally step 5) adjusting concentrations of BAFF and APRIL;
optionally step 6) adding a test sample and assessing B cell differentiation; and
optionally step 7) selecting a test sample capable of activating or inhibiting B cell differentiation as an immunomodulator;
wherein M and N are each an integer of 1-30.

In some embodiments of the present disclosure, a screening, preparation, or production method for an immunomodulator is provided, the method comprising:
step 1) administering to B cells CpG-B and IFNα;
step 2) removing CpG-B and IFNα on day M;
step 3) adding the interleukins IL-6 and IL-10 and IL-21;
optionally step 4) adding BAFF and APRIL;
optionally step 5) reducing a BAFF concentration and increasing an APRIL concentration;
optionally step 6) adding a test sample and assessing B cell differentiation; and
optionally step 7) selecting a test sample capable of activating or inhibiting B cell differentiation as an immunomodulator;
wherein M and N are each an integer of 1-20, M is 3, 4, 5, or 6, and N is 2, 3, or 4.

In the preceding embodiments, CpG-B is at a concentration selected from the group consisting of 0.1-100 µg/mL, 0.2-20 µg/mL, 0.5-10 µg/mL, and 0.5-5 µg/mL; for example, CpG-B is at a concentration of about 0.5, about 0.8, about 1, about 1.2, about 1.4, about 1.5, about 1.6, about 1.8, about 2, about 2.2, about 2.5, about 2.8, about 3, about 3.5, about 4, about 4.5, or about 5 µg/mL;
CpG-A or LPS is at a concentration of 0.01-10 µM, 0.05-5 µM, 0.1-2 µM, or 0.2-1 µM, e.g., about 0.2, about 0.3, about 0.4, about 0.5, about 0.6, about 0.7, about 0.8, about 0.9, about 1, about 1.5, about 2, about 2.5, or about 3 µM;
R484 is at a concentration of 0.1-100 µg/mL, 0.2-20 µg/mL, 0.5-10 µg/mL, or 0.5-5 µg/mL, e.g., about 0.5, about 0.6, about 0.7, about 0.8, about 0.9, about 1, about 1.1, about 1.2, about 1.3, about 1.4, about 1.5, about 2, about 3, or about 4 µg/mL;
the IFN (e.g., IFNα) is at a concentration of 1-10,000 U/mL, 50-1000 U/mL, or 100-500 U/mL, e.g., about 150, about 200, about 250, about 300, about 350, about 400, about 500, about 600, about 700, about 800, about 900, or about 1000 U/mL;
the interleukins (e.g., IL-3, IL-4, IL-6, IL-10, and IL-21) are at concentrations of 0.1-1000 ng/mL, 1-500 ng/mL, and 5-200 ng/mL, respectively, e.g., about 5, about 10, about 20, about 30, about 40, about 50, about 60, about 70, about 80, about 90, about 100, about 150, or about 200 ng/mL;
the TFN pathway modulators (e.g., BAFF and APRIL) are at a concentration of 1-10,000 ng/mL, 10-5000 ng/mL, 50-2000 ng/mL, or 20-200 ng/mL, e.g., about 10, about 20, about 30, about 40, about 50, about 60, about 70, about 80, about 90, about 100, about 200, about 250, about 300, about 400, about 500, about 600, about 7000, about 800, about 900, or about 1000 ng/mL.

In some embodiments of the present disclosure, a screening, preparation, or production method for an immunomodulator is provided, the method comprising:
step 1) administering to the B cells 0.5-10 µg/mL CpG-B and 50-1000 U/mL IFNα on day 0;
step 2) removing CpG-B and IFNα on days 2-8;
step 3) adding 2-100 ng/mL IL-6 and 10-1000 ng/mL IL-10 and 2-30 ng/mL IL-21; optionally step 4) adding 10-2000 ng/mL BAFF and 10-2000 ng/mL APRIL and stimulating for 2-5 days;
optionally step 5) reducing a BAFF concentration and increasing an APRIL concentration;
optionally step 6) adding a test sample on days 0-21 and assessing B cell differentiation; and
optionally step 7) selecting a test sample capable of activating or inhibiting B cell differentiation as an immunomodulator.

In some embodiments of the present disclosure, a screening, preparation, or production method for an immunomodulator is provided, the method comprising:
step 1) administering to the B cells 1-5 µg/mL CpG-B and 100-500 U/mL IFNα on day 0;
step 2) removing CpG-B and IFNα on days 3-5;
step 3) adding 5-50 ng/mL IL-6 and 100-500 ng/mL IL-10 and 5-20 ng/mL (or 5-21, 5-22, 5-23, or 5-24 ng/mL) IL-21;
step 4) adding 200-1000 ng/mL BAFF and 20-200 ng/mL APRIL and stimulating for 2-4 days;
step 5) reducing the BAFF concentration to 20-200 ng/mL and increasing the APRIL concentration to 200-1000 ng/mL;
step 6) adding a test sample at gradient concentrations on days 0-14 and assessing B cell differentiation; and
step 7) selecting a test sample capable of activating or inhibiting B cell differentiation as an immunomodulator.

In some embodiments of the present disclosure, a screening, preparation, or production method for an immunomodulator is provided, the method comprising:
step 1) administering to B cells about 2 µg/mL CpG-B and 250 U/mL IFNα;
step 2) removing CpG-B and IFNα on day 4 or 5;
step 3) adding about 10 ng/mL IL-6 and about 50 ng/mL IL-10 and about 15 ng/mL IL-21;
step 4) adding about 500 ng/mL BAFF and about 50 ng/mL APRIL;
step 5) after 3 days, adjusting the BAFF concentration to about 50 ng/mL and adjusting the APRIL concentration to about 500 ng/mL;
step 6) adding a test sample at gradient concentrations on days 0-14 and assessing B cell differentiation; and
step 7) selecting a test sample capable of activating or inhibiting B cell differentiation as an immunomodulator.

In some embodiments, the present disclosure provides a cell model for screening for an immunomodulator, the cell model comprising: B cells induced to differentiate by the method according to any one of the foregoing.

In the embodiments according to the foregoing fourth and fifth aspects, the immunomodulator includes, but is not limited to, type I interferon pathway modulators (e.g., IFNAR1 signaling pathway modulators) and TNF pathway modulators (e.g., BAFF and ARRIL pathway modulators); the IFNAR1 modulators and the BAFF and/or ARRIL pathway modulators include, but are not limited to, antibodies or antigen-binding fragments thereof, small-molecule compounds, nucleic acids, aptamers, receptors, ligands, and any molecule comprising the antibodies or the antigen-binding fragments thereof, the small-molecule compounds, the nucleic acids, the aptamers, the receptors, or the ligands. Exemplary IFNAR1 modulators include, but are not limited to, anifrolumab and other anti-IFNAR1 antibodies in the present disclosure, as well as fusion proteins comprising the anti-IFNAR1 antibodies (e.g., fusion proteins comprising a TACI polypeptide and a BCMA polypeptide and an anti-IFNAR1 antibody, fusion proteins comprising a TACI polypeptide and an anti-IFNAR1 antibody, and fusion proteins comprising a BCMA polypeptide and an anti-IFNAR1 antibody), and the fusion proteins comprising a TACI polypeptide and a BCMA polypeptide in the present disclosure.

Immunomodulators (e.g., BAFF and/or ARRIL pathway modulators) obtained by screening, prepared, and produced using the aforementioned methods provided by the present disclosure include, but are not limited to, TACI, telitacicept, the TACI polypeptide and BCMA polypeptide provided below in the present disclosure, and fusion proteins comprising the TACI polypeptide and/or the BCMA polypeptide (e.g., fusion proteins comprising the TACI polypeptide and the BCMA polypeptide and an anti-IFNAR1 antibody, fusion proteins comprising the TACI polypeptide and an anti-IFNAR1 antibody, fusion proteins comprising the BCMA polypeptide and an anti-IFNAR1 antibody, and fusion proteins comprising the TACI polypeptide and the BCMA polypeptide).

As described above, B cells are, for example, 1 × 10⁵ cells/well (in a 96-well plate). A preparation method for B cells comprises: isolating B cells from PBMCs, and culturing in a 1640 basal culture medium containing 50 µM β-mercaptoethanol, MEM non-essential amino acids (glycine, L-alanine, L-asparagine, L-aspartic acid, L-glutamic acid, L-proline, and L-serine), sodium pyruvate, and GlutaMAX.

In a sixth aspect, the present disclosure further provides a pharmaceutical composition comprising an immunomodulator obtained by screening using an aforementioned method, and one or more pharmaceutically acceptable carriers, diluents, or excipients. In some embodiments, the amount of the immunomodulator in the pharmaceutical composition is 0.1-2000 mg; in some specific embodiments, the amount of the immunomodulator in the pharmaceutical composition is 1-1000 mg.

In addition, the present disclosure further provides use of an immunomodulator obtained by screening using an aforementioned method in the preparation of a pharmaceutical composition. In addition, the present disclosure further provides a method for producing or preparing a pharmaceutical composition, the method comprising using an immunomodulator obtained by screening using an aforementioned method. The aforementioned method for producing or preparing a pharmaceutical composition comprises a step of mixing the immunomodulator according to any one of the foregoing with one or more pharmaceutically acceptable carriers, diluents, or excipients, or comprises the steps of preparing or producing an immunomodulator according to any one of the foregoing and a step of mixing the resulting immunomodulator with one or more pharmaceutically acceptable carriers, diluents, or excipients.

In a seventh aspect, the present disclosure provides a method for treating or ameliorating a disease or disorder with the aforementioned immunomodulator or pharmaceutical composition, the method comprising administering to a subject in need thereof the aforementioned immunomodulator or pharmaceutical composition.

In some embodiments, the disease or disorder is a B cell disorder or an autoimmune disease.

In some specific embodiments, the B cell disorder or the autoimmune disease is a disease or disorder associated with TACI and/or BCMA expression.

In some specific embodiments, the autoimmune disease is selected from the group consisting of systemic lupus erythematosus, myasthenia gravis, multiple sclerosis, insulin-dependent diabetes mellitus, Crohn's disease, rheumatoid arthritis, polyarticular juvenile rheumatoid arthritis, and psoriatic arthritis; the B cell disorder is selected from the group consisting of tumors, chronic leukocytic leukemia, multiple myeloma, non-Hodgkin lymphoma, post-transplant lymphoproliferative disorder, and light chain gammopathy.

In some embodiments, a preparation method for the aforementioned immunomodulators is provided; without limitations, these immunomodulators can all be prepared by well-known or conventional methods in the art. For example, the antibody or the antigen-binding fragment is expressed in a host cell, and the aforementioned fusion protein is isolated from the host cell. Optionally, the method may further comprise a purification step. Optionally, the method may further comprise a filtration step and a concentration step. Soluble mixtures and polymers can also be removed by conventional methods, such as molecular sieves and ion exchange. The resulting products need to be immediately frozen, such as at -70 °C, or lyophilized.

In addition, the present disclosure provides a fusion protein of a new structure comprising a TACI polypeptide, a nucleic acid encoding same, a vector, a host cell, a pharmaceutical composition, a method for treating or ameliorating a disease (e.g., a B cell disorder or an autoimmune disease) with same, and pharmaceutical use thereof.

### Fusion Protein

The present disclosure provides a fusion protein, the fusion protein:
(1) comprising a TACI polypeptide;
(2) comprising a BCMA polypeptide;
(3) comprising a TACI polypeptide and a BCMA polypeptide;
(4) comprising a TACI polypeptide and an antibody (e.g., an anti-IFNAR1 antibody);
(5) comprising a BCMA polypeptide and an antibody (e.g., an anti-IFNAR1 antibody); or
(6) comprising a TACI polypeptide, a BCMA polypeptide, and an antibody (e.g., an anti-IFNAR1 antibody).

### Regarding the TACI polypeptide in the fusion protein:

In some embodiments, the TACI polypeptide comprises a CRD1 and/or a CRD2 of a TACI extracellular region, e.g., a CRD2 of a TACI extracellular region.

In some embodiments, the TACI polypeptide comprises the following amino acid sequence:
(1) SEQ ID NO: 1 or an amino acid sequence having at least 90% sequence identity thereto;
(2) the amino acid residues at positions 33-67 of SEQ ID NO: 1;
(3) the amino acid residues at positions 70-104 of SEQ ID NO: 1, or a variant thereof;
(4) the amino acid residues at positions 30-110, 69-111, 69-112, 13-118, or 33-104 of SEQ ID NO: 1, or a variant thereof; or
(5) the amino acid residues at positions 68-105, 68-106, 68-107, or 68-108 of SEQ ID NO: 1, or a variant thereof.

In some specific embodiments, the variant has one or more amino acid mutations selected from the group consisting of positions 69, 72, 73, 77, 85, 102, and 103.

In some specific embodiments, the variant has one or more amino acid replacements selected from the group consisting of 69T or 69R, 72S, 73E or 73Q, 77E, 85T or 85A, 102A or 102R, and 103Y.

In some specific embodiments, the variant has an amino acid replacement or a combination of replacements selected from any one of the following: 69T; 72S; 73E; 73Q; 77E; 69R/85T; 69R/85A; 102A; 69R/85T/102R; 73E/77E; 72S/73E/77E; 69T/102A; 69T/103Y; 69T/102A and 103Y; and 69T/73E/77E/102A.

In some specific embodiments, single amino acid substitutions are linked by "/" to indicate a mutation combination at multiple given positions. For example, a mutation combination at positions 69T and 102A can be expressed as 69T/102A.

In some specific embodiments, the variant has a combination of amino acid replacements 69T/73E/77E/102A, for example, a combination of amino acid replacements L69T/K73E/K77E/Y102A.

The sites of the amino acid mutations are amino acid residue sites numbered in natural order relative to the TACI extracellular region (SEQ ID NO: 1). For example, "the variant has an amino acid mutation selected from the group consisting of position 69" means that the amino acid residue corresponding to position 69 of SEQ ID NO: 1 has a mutation.

In some embodiments, the amino acid sequence of the TACI polypeptide comprises an amino acid sequence selected from any one of SEQ ID NOs: 1, 2, and 6-29.

In the present disclosure, "at least 90% (sequence) identity" encompasses at least 90%, at least 91%, at least 92%, at least 93%, at least 94%, at least 95%, at least 96%, at least 97%, at least 98%, at least 99%, or 100% (sequence) identity; "at least 80% (sequence) identity" encompasses at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% (sequence) identity.

### Regarding the BCMA polypeptide in the fusion protein:

In some embodiments, the BCMA polypeptide comprises a CRD1 of a BCMA extracellular region.

In some embodiments, the BCMA polypeptide comprises the amino acid residues at positions 7-41 of SEQ ID NO: 30.

In some embodiments, the BCMA polypeptide comprises an amino acid sequence set forth in any one of SEQ ID NOs: 30, 67, and 68 or having at least 90% sequence identity thereto.

### Regarding the antibody in the fusion protein:

In some embodiments, the antibody in the fusion protein is an anti-IFNAR1 antibody.

In some embodiments, the anti-IFNAR1 antibody comprises a heavy chain variable region (VH) and a light chain variable region (VL), wherein the heavy chain variable region comprises a HCDR1, a HCDR2, and a HCDR3, and the amino acid sequences of the HCDR1, the HCDR2, and the HCDR3 are set forth in SEQ ID NOs: 45-47, respectively; the light chain variable region comprises a LCDR1, a LCDR2, and a LCDR3, and the amino acid sequences of the LCDR1, the LCDR2, and the LCDR3 are set forth in SEQ ID NOs: 48-50, respectively.

In some embodiments, the amino acid sequence of the heavy chain variable region of the anti-IFNAR1 antibody is set forth in SEQ ID NO: 43 or has at least 90% sequence identity thereto, and the amino acid sequence of the light chain variable region is set forth in SEQ ID NO: 44 or has at least 90% sequence identity thereto.

In some embodiments, the amino acid sequence of the heavy chain of the anti-IFNAR1 antibody is set forth in SEQ ID NO: 55 or has at least 80% sequence identity thereto, and the amino acid sequence of the light chain is set forth in SEQ ID NO: 54 or has at least 80% sequence identity thereto.

In some embodiments, the amino acid sequence of the heavy chain of the anti-IFNAR1 antibody is set forth in SEQ ID NO: 61 or has at least 80% sequence identity thereto, and the amino acid sequence of the light chain is set forth in SEQ ID NO: 54 or has at least 80% sequence identity thereto.

In some embodiments, the present disclosure incorporates anifrolumab, sifalimumab, and the entirety of the anti-IFNAR1 antibodies or the antigen-binding fragments thereof in WO20062002177A, WO2009100309A (e.g., 3F11, 4G5, 11E2, and 9D4), WO2020156474A (e.g., 7G4 and 10C5), WO2020057541A (e.g., 8G11H and 485G10H), CN201610634601.7 (e.g., H19B7 + L16C11, H19B7 + L8C3, H15D10 + L16C11, H15D10 + L8C3, or anti-IFNAR1-C1), CN201510685200.X, and WO2012162367A as the anti-IFNAR1 antibody of the present disclosure.

### Regarding the fusion protein:

As previously described, the present disclosure provides a fusion protein comprising a TACI polypeptide and a BCMA polypeptide, a fusion protein comprising a TACI polypeptide and an anti-IFNAR1 antibody, or a fusion protein comprising a TACI polypeptide, a BCMA polypeptide, and an anti-IFNAR1 antibody.

In some embodiments, the fusion protein further comprises a heavy chain constant region (Fc region) of an immunoglobulin. In some specific embodiments, the Fc region comprises two subunits capable of associating. In some embodiments, the two subunits are a first subunit and a second subunit that are identical or different. In some embodiments, the Fc region is an Fc region of IgG, IgA, IgM, or IgD; in some embodiments, the Fc region is an Fc region of human IgG1, IgG2, IgG3, or IgG4, e.g., an Fc region of human IgG1, or, e.g., an Fc region set forth in SEQ ID NO: 3.

In some embodiments, the Fc region in the fusion protein comprises one or more amino acid substitutions compared to a wild-type Fc region, and the amino acid substitutions are capable of reducing the binding of the Fc region to an Fc receptor (FcR); in some embodiments, the amino acid substitutions are capable of reducing the binding of the Fc region to an Fcγ receptor (FcγR); in some embodiments, the Fc region has YTE mutations (M252Y, S254T, and T256E), S228P, L234F, L235E, L234F/L235E, L234A/L235A, or L234F/L235E/P331S mutations, and the mutation sites are numbered according to the EU index.

In some embodiments, the Fc region in the fusion protein comprises a first subunit and a second subunit capable of associating with each other, and the first subunit and second subunit have one or more amino acid substitutions for reducing homodimerization. In some embodiments, the first subunit has a knob structure according to the knob-into-hole technique, and the second subunit has a hole structure according to the knob-into-hole technique; or the first subunit has a hole structure according to the knob-into-hole technique, and the second subunit has a knob structure according to the knob-into-hole technique. In some embodiments, the amino acid residue substitutions in the first subunit include one or more amino acid substitutions selected from the group consisting of positions 354, 356, 358, and 366, and the amino acid residue substitutions in the second subunit include one or more amino acid substitutions selected from the group consisting of positions 349, 356, 358, 366, 368, and 407. In some embodiments, the first subunit comprises one or more amino acid substitutions selected from the group consisting of 354C, 356E, 358M, and 366W, and the second subunit comprises one or more amino acid substitutions selected from the group consisting of 349C, 356E, 358M, 366S, 368A, and 407V. In some embodiments, the first subunit comprises amino acid substitutions 354C, 356E, 358M, and 366W, and the second subunit comprises amino acid substitutions 349C, 356E, 358M, 366S, 368A, and 407V.

### Regarding the fusion protein comprising a TACI polypeptide and a BCMA polypeptide:

In some embodiments, the TACI polypeptide is any one of the TACI polypeptides provided above by the present disclosure, and the BCMA polypeptide is any one of the BCMA polypeptides provided above by the present disclosure.

In some embodiments, the fusion protein comprises one or more (e.g., 2, 3, 4, 5, or 6) said TACI polypeptides and one or more (e.g., 2, 3, 4, 5, or 6) said BCMA polypeptides. In some embodiments, the fusion protein comprises one said TACI polypeptide and one said BCMA polypeptide, or two said TACI polypeptides and two said BCMA polypeptides, or three said TACI polypeptides and three said BCMA polypeptides, or four said TACI polypeptides and four said BCMA polypeptides, or two said TACI polypeptides and one said BCMA polypeptide, or one said TACI polypeptide and two said BCMA polypeptides, or three said TACI polypeptides and one said BCMA polypeptide, or one said TACI polypeptide and three said BCMA polypeptides; when the fusion protein comprises two or more said TACI polypeptides, the TACI polypeptides may be TACI polypeptides identical to or different from the sequence provided by the present disclosure; when the fusion protein comprises two or more said BCMA polypeptides, the BCMA polypeptides may be BCMA polypeptides identical to or different from the sequence provided by the present disclosure.

In some embodiments, in the fusion protein, the TACI polypeptide and a subunit of the Fc region are linked in any order; optionally, the TACI polypeptide is linked to the first subunit or the second subunit of the Fc region by a linker or directly. In some embodiments, in any one of the aforementioned fusion proteins, the BCMA polypeptide and a subunit of the Fc region are linked in any order; optionally, the BCMA polypeptide is linked to the first subunit or the second subunit of the Fc region by a linker or directly. In some embodiments, the C-terminus of the TACI polypeptide is linked to the N-terminus of the first subunit or the second subunit of the Fc region by a linker or directly, or the N-terminus of the TACI polypeptide is linked to the C-terminus of the first subunit or the second subunit of the Fc region by a linker or directly. In some embodiments, the C-terminus of the BCMA polypeptide is linked to the N-terminus of the first subunit or the second subunit of the Fc region by a linker or directly, or the N-terminus of the BCMA polypeptide is linked to the C-terminus of the first subunit or the second subunit of the Fc region by a linker or directly.

In some embodiments, the fusion protein comprising a TACI polypeptide and a BCMA polypeptide comprises a polypeptide chain set forth in any one of (I)-(VIII) below:
(I) [TACI polypeptide]-[linker 1]a-[BCMA polypeptide]-[linker 2]b-[Fc region]e;
(II) [BCMA polypeptide]-[linker 1]a-[TACI polypeptide]-[linker 2]b-[Fc region]e;
(III) [Fc region]e-[linker 3]c-[TACI polypeptide]-[linker 4]d-[BCMA polypeptide];
(IV) [Fc region]e-[linker 3]c-[BCMA polypeptide]-[linker 4]d-[TACI polypeptide];
(V) [TACI polypeptide 1]-[linker 1]a-[BCMA polypeptide 1]-[linker 2]b-[Fc region]e-[linker 3]c-[TACI polypeptide 2]-[linker 4]d-[BCMA polypeptide 2];
(VI) [BCMA polypeptide 1]-[linker 1]a-[TACI polypeptide 1]-[linker 2]b-[Fc region]e-[linker 3]c-[BCMA polypeptide 2]-[linker 4]d-[TACI polypeptide 2];
(VII) [TACI polypeptide 1]-[linker 1]a-[BCMA polypeptide 1]-[linker 2]b-[Fc region]e-[linker 3]c-[BCMA polypeptide 2]-[linker 4]d-[TACI polypeptide 2]; and
(VIII) [BCMA polypeptide 1]-[linker 1]a-[TACI polypeptide 1]-[linker 2]b-[Fc region]e-[linker 3]c-[TACI polypeptide 2]-[linker 4]d-[BCMA polypeptide 2];
wherein - represents a peptide bond; the linkers are polypeptides capable of fulfilling the function of linking, and linker 1, linker 2, linker 3 and, linker 4 may be identical or different; TACI polypeptide 1 and TACI polypeptide 2 are selected from the group consisting of the TACI polypeptides provided by the present disclosure and may be identical or different; BCMA polypeptide 1 and BCMA polypeptide 2 are selected from the group consisting of the BCMA polypeptides provided by the present disclosure and may be identical or different; a, b, c, d, and e are each independently 0 or 1; for example, in solution (I) or (II), both a and b are 1, or a is 1 and b is 0, or a is 0 and b is 1, and e is 0 or 1; for example, in solution (III) or (IV), both c and d are 1, or c is 1 and d is 0, or c is 0 and d is 1, and e is 0 or 1; for example, in solution (V), (VI), (VII), or (VIII), a, b, c, and d are all 1, or a, b, and c are all 1 and d is 0, or a and b are 1 and c and d are 0, or a is 1 and b, c, and d are all 0, or a is 0 and b, c, and d are all 1, or a and b are 0 and c and d are 1, or a, b, and c are 0 and d is 1, or a and c are 1 and b and d are 0, or a and c are 0 and b and d are 1, and e is 0 or 1.

In some embodiments, the linkers are amino acid sequences represented by (GₘSₙ)ₕ or (GGNGT)ₕ (SEQ ID NO: 75) or (YGNGT)ₕ (SEQ ID NO: 76) or (EPKSS)ₕ (SEQ ID NO: 77), wherein m and n are each independently selected from an integer of 1-8 (e.g., 1, 2, 3, 4, 5, 6, 7, or 8), and h is independently selected from an integer of 1-20 (e.g., 1, 2, 3, 4, 5, 6, 7, 8, 9, 10, 11, 12, 13, 14, 15, 16, 17, 18, 19, or 20). In some specific embodiments, the linkers have an amino acid sequence represented by (G₄S)_{y} (SEQ ID NO: 78), wherein y is independently selected from an integer of 1-6 (for example, y is 1, 2, 3, 4, 5, or 6). For example, the linkers are (GₓS)_{y} linkers, wherein x is selected from an integer of 1-5, and y is selected from an integer of 1-6; for example, the linkers are linkers set forth in any one of SEQ ID NOs: 39-41. For example, the linkers have an amino acid sequence represented by (G₄S)_{y} (SEQ ID NO: 78), wherein y is independently selected from an integer of 1-6 (for example, y is 1, 2, 3, 4, 5, or 6).

In some embodiments, a fusion protein comprising a TACI polypeptide and a BCMA polypeptide is provided, the fusion protein comprising an amino acid sequence set forth in any one of SEQ ID NOs: 31-38, 69, and 70 or having at least 90% sequence identity thereto.

In some embodiments, the fusion protein is a multimer, e.g., a dimer (e.g., a homodimer or a heterodimer).

In some embodiments, a fusion protein comprising a TACI polypeptide and a BCMA polypeptide is provided, wherein the TACI polypeptide may be a variant, and the variant has 1 to 10 (1, 2, 3, 4, 5, 6, 7, 8, 9, or 10) amino acid mutations; in some embodiments, a fusion protein comprising a TACI polypeptide and a BCMA polypeptide is provided, wherein the BCMA polypeptide may be a variant, and the variant has 1 to 10 (1, 2, 3, 4, 5, 6, 7, 8, 9, or 10) amino acid mutations; the amino acid mutations may be conservative replacements, substitutions, or modifications, and/or deletions and additions that do not affect functionality.

In some embodiments, a protein or molecule is provided, and the protein or molecule competes with the fusion protein comprising a TACI polypeptide and a BCMA polypeptide, the TACI polypeptide, or the BCMA polypeptide for binding to BAFF and/or APRIL or blocks the binding of the fusion protein comprising a TACI polypeptide and a BCMA polypeptide, the TACI polypeptide, or the BCMA polypeptide to BAFF and/or APRIL.

Regarding the fusion protein comprising a TACI polypeptide and an anti-IFNAR1 antibody:

In some embodiments, the TACI polypeptide is any one of the TACI polypeptides provided above by the present disclosure, and the anti-IFNAR1 antibody is any one of the anti-IFNAR1 antibodies provided above by the present disclosure.

In some embodiments, the fusion protein comprising a TACI polypeptide and an anti-IFNAR1 antibody comprises one or more (e.g., 2, 3, 4, 5, or 6) TACI polypeptides. In some embodiments, the fusion protein comprising a TACI polypeptide and an anti-IFNAR1 antibody comprises one or more (e.g., 2, 3, or 4) anti-IFNAR1 antibodies. In some embodiments, the fusion protein comprising a TACI polypeptide and an anti-IFNAR1 antibody comprises polypeptide chains set forth in any one of (I)-(VIII) below:
(I) a first polypeptide chain that is, from the N-terminus to the C-terminus, [TACI polypeptide]-[linker 1]a-[antibody heavy chain], and a second polypeptide chain that is an antibody light chain;
(II) a first polypeptide chain that is, from the N-terminus to the C-terminus, [antibody heavy chain]-[linker 2]b-[TACI polypeptide], and a second polypeptide chain that is an antibody light chain;
(III) a first polypeptide chain that is an antibody heavy chain, and a second polypeptide chain that is, from the N-terminus to the C-terminus, [TACI polypeptide]-[linker 3]c-[antibody light chain];
(IV) a first polypeptide chain that is an antibody heavy chain, and a second polypeptide chain that is, from the N-terminus to the C-terminus, [antibody light chain]-[linker 4]d-[TACI polypeptide];
(V) a first polypeptide chain that is, from the N-terminus to the C-terminus, [TACI polypeptide 1]-[linker 1]a-[antibody heavy chain], and a second polypeptide chain that is, from the N-terminus to the C-terminus, [antibody light chain]-[linker 4]d-[TACI polypeptide 2];
(VI) a first polypeptide chain that is, from the N-terminus to the C-terminus, [antibody heavy chain]-[linker 2]b-[TACI polypeptide 1], and a second polypeptide chain that is, from the N-terminus to the C-terminus, [TACI polypeptide 2]-[linker 3]c-[antibody light chain];
(VII) a first polypeptide chain that is, from the N-terminus to the C-terminus, [TACI polypeptide 1]-[linker 1]a-[antibody heavy chain]-[linker 2]b-[TACI polypeptide 2], and a second polypeptide chain that is an antibody light chain; and
(VIII) a first polypeptide chain that is an antibody heavy chain, and a second polypeptide chain that is, from the N-terminus to the C-terminus, [TACI polypeptide 1]-[linker 3]c-[antibody light chain]-[linker 4]d-[TACI polypeptide 2];
wherein - represents a peptide bond; the linkers are polypeptides capable of fulfilling the function of linking; linker 1, linker 2, linker 3, and linker 4 may be identical or different; TACI polypeptide 1 and TACI polypeptide 2 are selected from any one of the aforementioned TACI polypeptides of the present disclosure, and TACI polypeptide 1 and TACI polypeptide 2 may be identical or different; a, b, c, and d are each independently 0 or 1. In solution (I), a is 1 or 0. In solution (II), b is 1 or 0. In solution (III), c is 1 or 0. In solution (IV), d is 1 or 0. In solution (V), a and d may be 0 or 1 simultaneously, or a is 0 and d is 1, or a is 1 and d is 0; in solution (VI), b and c may be 0 or 1 simultaneously, or b is 0 and c is 1, or b is 1 and c is 0; in solution (VII), a and b may be 0 or 1 simultaneously, or a is 0 and b is 1, or a is 1 and b is 0; in solution (VIII), c and d may be 0 or 1 simultaneously, or c is 0 and d is 1, or c is 1 and d is 0; in the above solutions, e may be independently 0 or 1.

In some embodiments, the linkers are amino acid sequences represented by (GₘSₙ)ₕ or (GGNGT)ₕ or (YGNGT)ₙ or (EPKSS)ₕ, wherein m and n are each independently selected from an integer of 1-8 (e.g., 1, 2, 3, 4, 5, 6, 7, or 8), and h is independently selected from an integer of 1-20 (e.g., 1, 2, 3, 4, 5, 6, 7, 8, 9, 10, 11, 12, 13, 14, 15, 16, 17, 18, 19, or 20). In some specific embodiments, the linkers have an amino acid sequence represented by (G₄S)_{y}, wherein y is independently selected from an integer of 1-6 (for example, y is 1, 2, 3, 4, 5, or 6). For example, the linkers are (GₓS)_{y} linkers, wherein x is selected from an integer of 1-5, and y is selected from an integer of 1-6; for example, the linkers are linkers set forth in any one of SEQ ID NOs: 39-41.

In some embodiments, a fusion protein comprising a TACI polypeptide and an anti-IFNAR1 antibody is provided, the fusion protein comprising: a first polypeptide chain set forth in any one of SEQ ID NOs: 51-53 and 62 or having at least 80% or at least 90% sequence identity thereto, and a second polypeptide chain set forth in SEQ ID NO: 54 or having at least 80% or at least 90% sequence identity thereto;
a first polypeptide chain set forth in SEQ ID NO: 55 or having at least 80% or at least 90% sequence identity thereto, and a second polypeptide chain set forth in any one of SEQ ID NOs: 56-60 or having at least 80% or at least 90% sequence identity thereto; or
a first polypeptide chain set forth in SEQ ID NO: 61 or having at least 80% or at least 90% sequence identity thereto, and a second polypeptide chain set forth in any one of SEQ ID NOs: 56-60 or having at least 80% or at least 90% sequence identity thereto.

In some embodiments, the aforementioned fusion protein comprises two identical first polypeptide chains and two identical second polypeptide chains.

In some embodiments, the present disclosure provides a fusion protein comprising a TACI polypeptide and an anti-IFNAR1 antibody, wherein the TACI polypeptide may be a variant, and the variant has 1 to 10 (1, 2, 3, 4, 5, 6, 7, 8, 9, or 10) amino acid mutations; the amino acid mutations may be conservative replacements, substitutions, or modifications, and/or deletions and additions that do not affect functionality.

In some embodiments, a protein or molecule is provided, and the protein or molecule competes with the aforementioned fusion protein comprising a TACI polypeptide and an anti-IFNAR1 antibody for binding to BAFF and/or APRIL or blocks the binding of the fusion protein to BAFF and/or APRIL.

### Regarding the fusion protein comprising a TACI polypeptide, a BCMA polypeptide, and an anti-IFNAR1 antibody:

In some embodiments, the TACI polypeptide is any one of the TACI polypeptides provided above by the present disclosure, the BCMA polypeptide is any one of the BCMA polypeptides provided above by the present disclosure, and the anti-IFNAR1 antibody is any one of the anti-IFNAR1 antibodies provided above by the present disclosure.

In some embodiments, the antibody fusion protein comprising a TACI polypeptide, a BCMA polypeptide, and an anti-IFNAR1 antibody comprises one or more (e.g., 2, 3, 4, 5, or 6) TACI polypeptides. In some embodiments, the antibody fusion protein comprising a TACI polypeptide, a BCMA polypeptide, and an anti-IFNAR1 antibody comprises one or more (e.g., 2, 3, 4, 5, or 6) BCMA polypeptides. In some embodiments, the antibody fusion protein comprising a TACI polypeptide, a BCMA polypeptide, and an anti-IFNAR1 antibody comprises one or more (e.g., 2, 3, or 4) anti-IFNAR1 antibodies. In some embodiments, the aforementioned antibody fusion protein comprising a TACI polypeptide, a BCMA polypeptide, and an anti-IFNAR1 antibody comprises one aforementioned TACI polypeptide and one aforementioned BCMA polypeptide, or two aforementioned TACI polypeptides and two aforementioned BCMA polypeptides, or three aforementioned TACI polypeptides and three aforementioned BCMA polypeptides, or four aforementioned TACI polypeptides and four aforementioned BCMA polypeptides, or two aforementioned TACI polypeptides and one aforementioned BCMA polypeptide, or one aforementioned TACI polypeptide and two aforementioned BCMA polypeptides, or three aforementioned TACI polypeptides and one aforementioned BCMA polypeptide, or one aforementioned TACI polypeptide and three aforementioned BCMA polypeptides, and one anti-IFNAR1 antibody; when the fusion protein comprises two or more aforementioned TACI polypeptides, the TACI polypeptides may be TACI polypeptides with identical or different sequences provided by the present disclosure; when the fusion protein comprises two or more aforementioned BCMA polypeptides, the BCMA polypeptides may be BCMA polypeptides with identical or different sequences provided by the present disclosure.

In some embodiments, the fusion protein comprising a TACI polypeptide, a BCMA polypeptide, and an anti-IFNAR1 antibody according to any one of the foregoing comprises polypeptide chains set forth in any one of (I)-(VIII) below:
(I) a first polypeptide chain that is, from the N-terminus to the C-terminus, [TACI polypeptide and BCMA polypeptide domain]-[linker 1]a-[antibody heavy chain], and a second polypeptide chain that is an antibody light chain;
(II) a first polypeptide chain that is, from the N-terminus to the C-terminus, [antibody heavy chain]-[linker 2]b-[TACI polypeptide and BCMA polypeptide domain], and a second polypeptide chain that is an antibody light chain;
(III) a first polypeptide chain that is an antibody heavy chain, and a second polypeptide chain that is, from the N-terminus to the C-terminus, [TACI polypeptide and BCMA polypeptide domain]-[linker 3]c-[antibody light chain];
(IV) a first polypeptide chain that is an antibody heavy chain, and a second polypeptide chain that is, from the N-terminus to the C-terminus, [antibody light chain]-[linker 4]d-[TACI polypeptide and BCMA polypeptide domain];
(V) a first polypeptide chain that is, from the N-terminus to the C-terminus, [TACI polypeptide and BCMA polypeptide domain 1]-[linker 1]a-[antibody heavy chain], and a second polypeptide chain that is, from the N-terminus to the C-terminus, [antibody light chain]-[linker 4]d-[TACI polypeptide and BCMA polypeptide domain 2];
(VI) a first polypeptide chain that is, from the N-terminus to the C-terminus, [antibody heavy chain]-[linker 2]b-[TACI polypeptide and BCMA polypeptide domain 1], and a second polypeptide chain that is, from the N-terminus to the C-terminus, [TACI polypeptide and BCMA polypeptide domain 2]-[linker 3]c-[antibody light chain];
(VII) a first polypeptide chain that is, from the N-terminus to the C-terminus, [TACI polypeptide and BCMA polypeptide domain 1]-[linker 1]a-[antibody heavy chain]-[linker 2]b-[TACI polypeptide and BCMA polypeptide domain 2], and a second polypeptide chain that is an antibody light chain; and
(VIII) a first polypeptide chain that is an antibody heavy chain, and a second polypeptide chain that is, from the N-terminus to the C-terminus, [TACI polypeptide and BCMA polypeptide domain 1]-[linker 3]c-[antibody light chain]-[linker 4]d-[TACI polypeptide and BCMA polypeptide domain 2];

wherein "[TACI polypeptide and BCMA polypeptide domain]", "[TACI polypeptide and BCMA polypeptide domain 1]", and "[TACI polypeptide and BCMA polypeptide domain 2]" are, from the N-terminus to the C-terminus: [TACI polypeptide]-[linker 5]e-[BCMA polypeptide] or [BCMA polypeptide]-[linker 5]e-[TACI polypeptide]; in "TACI polypeptide and BCMA polypeptide domain 1" and "TACI polypeptide and BCMA polypeptide domain 2", the TACIs may be identical or different, and the BCMAs may be identical or different;
- represents a peptide bond; the linkers are polypeptides capable of fulfilling the function of linking; linker 1, linker 2, linker 3, linker 4, and linker 5 may be identical or different; a, b, c, d, and e are each independently 0 or 1; a, b, c, and d are each independently 0 or 1; in solution (I), a is 1 or 0. In solution (II), b is 1 or 0. In solution (III), c is 1 or 0. In solution (IV), d is 1 or 0. In solution (V), a and d may be 0 or 1 simultaneously, or a is 0 and d is 1, or a is 1 and d is 0; in solution (VI), b and c may be 0 or 1 simultaneously, or b is 0 and c is 1, or b is 1 and c is 0; in solution (VII), a and b may be 0 or 1 simultaneously, or a is 0 and b is 1, or a is 1 and b is 0; in solution (VIII), c and d may be 0 or 1 simultaneously, or c is 0 and d is 1, or c is 1 and d is 0; in the above solutions, e may be independently 0 or 1.

In some embodiments, the linkers are amino acid sequences represented by (GₘSₙ)ₕ or (GGNGT)ₙ or (YGNGT)ₙ or (EPKSS)ₕ, wherein m and n are each independently selected from an integer of 1-8 (e.g., 1, 2, 3, 4, 5, 6, 7, or 8), and h is independently selected from an integer of 1-20 (e.g., 1, 2, 3, 4, 5, 6, 7, 8, 9, 10, 11, 12, 13, 14, 15, 16, 17, 18, 19, or 20). In some specific embodiments, the linkers have an amino acid sequence represented by (G₄S)_{y}, wherein y is independently selected from an integer of 1-6 (for example, y is 1, 2, 3, 4, 5, or 6). For example, the linkers are (GₓS)_{y} linkers, wherein x is selected from an integer of 1-5, and y is selected from an integer of 1-6; for example, the linkers are linkers set forth in any one of SEQ ID NOs: 39-41. For example, the linkers have an amino acid sequence represented by (G₄S)_{y}, wherein y is independently selected from an integer of 1-6 (for example, y is 1, 2, 3, 4, 5, or 6).

In some embodiments, a fusion protein comprising a TACI polypeptide, a BCMA polypeptide, and an anti-IFNAR1 antibody is provided, the fusion protein comprising: a first polypeptide chain set forth in SEQ ID NO: 55 or having at least 80% or at least 90% sequence identity thereto, and a second polypeptide chain set forth in any one of SEQ ID NOs: 63-66, 71, and 72 or having at least 80% or at least 90% sequence identity thereto;
a first polypeptide chain set forth in SEQ ID NO: 61 or having at least 80% or at least 90% sequence identity thereto, and a second polypeptide chain set forth in any one of SEQ ID NOs: 63-66, 71, and 72 or having at least 80% or at least 90% sequence identity thereto; or
a first polypeptide chain set forth in SEQ ID NO: 73 or 74 or having at least 80% or at least 90% sequence identity thereto, and a second polypeptide chain set forth in SEQ ID NO: 54 or having at least 80% or at least 90% sequence identity thereto.

In some embodiments, the aforementioned fusion protein comprises two identical first polypeptide chains and two identical second polypeptide chains.

In some embodiments, the present disclosure provides a fusion protein comprising a TACI polypeptide, a BCMA polypeptide, and an anti-IFNAR1 antibody, wherein the TACI polypeptide may be a variant, and the variant has 1 to 10 (1, 2, 3, 4, 5, 6, 7, 8, 9, or 10) amino acid mutations; the amino acid mutations may be conservative replacements, substitutions, or modifications, and/or deletions and additions that do not affect functionality.

In some embodiments, a protein or molecule is provided, and the protein or molecule competes with the aforementioned fusion protein comprising a TACI polypeptide, a BCMA polypeptide, and an anti-IFNAR1 antibody for binding to BAFF and/or APRIL or blocks the binding of the fusion protein to BAFF and/or APRIL.

### Regarding the aforementioned fusion protein:

In some embodiments, the fusion protein comprising a TACI polypeptide and a BCMA polypeptide, the fusion protein comprising a TACI polypeptide and an IFNAR1 antibody, and the fusion protein comprising a TACI polypeptide, a BCMA polypeptide, and an IFNAR1 antibody have one or more of the following properties or functions:
(a) the activity of binding to BAFF;
(b) the activity of binding to TALL-2/APRIL;
(c) reducing the concentration of a serum immunoglobulin (e.g., IgE or IgM);
(d) reducing the weight of the spleen;
(e) inhibiting or blocking the BAFF/APRIL pathway; and
(f) reducing the number of B cells.

In some embodiments, the protein comprising a TACI polypeptide provided by the present disclosure (e.g., TACI-Fc) has one or more of the following functional activities:
a. being not prone to producing fragments resulting from the cleavage of the TACI polypeptide; in some embodiments, the cleavage may be detected by mass spectrometry, e.g., the TACI cleavage analysis method in Example 2;
b. blocking the binding of BAFF to BAFF-R; in some embodiments, TACI-Fc blocks the binding of BAFF to BAFF-R with an IC₅₀ value of less than 23 nM, less than 10 nM, less than 6 nM, less than 5 nM, less than 4 nM, less than 3 nM, less than 2 nM, less than 1 nM, or less, as determined by an ELISA method; in some embodiments, the method for determining the IC₅₀ value is described in Example 1;
c. the ability to bind to BAFF; in some embodiments, the TACI-Fc binds to BAFF with an EC₅₀ value of less than 5 nM, 1 nM, 0.5 nM, 0.4 nM, 0.3 nM, 0.2 nM, 0.1 nM, or less, as determined by an ELISA method, e.g., the ELISA binding assay of Example 2 (coating with the antigen protein);
d. inhibiting BAFF-induced B cell proliferation; in some embodiments, TACI-Fc inhibits BAFF-induced B cell proliferation with an IC₅₀ value of less than 0.3 nM, less than 0.2 nM, less than 0.1 nM, 0.06 nM, less than 0.05 nM, less than 0.01 nM, or less, as determined by an ELISA method, e.g., the ELISA assay in Example 3;
e. inhibiting APRIL-induced B cell proliferation; in some embodiments, TACI-Fc inhibits APRIL-induced B cell proliferation with an IC₅₀ value of less than 3 nM, less than 2 nM, less than 1 nM, less than 0.5 nM, less than 0.4 nM, less than 0.3 nM, 0.2 nM, less than 0.1 nM, less than 0.05, or less, as determined by an ELISA method;
f. binding to human BAFF and/or human APRIL with a high affinity; in some embodiments, the affinity was measured by a Biacore method; in some embodiments, the TACI-Fc fusion protein binds to human BAFF with a KD value of less than 1E-10 M, less than 9E-11 M, 8E-11 M, or less; in some embodiments, the TACI-Fc fusion protein binds to human APRIL with a KD value of less than 2.3E-11 M, less than 2.0E-11 M, 1.9E-11 M, 1.3E-11 M, or less;
g. a good *in vivo* pharmacokinetic profile; in some embodiments, TACI-Fc has a half-life of greater than 4 days in rats; and/or
h. good stability; in some embodiments, after the TACI-Fc has been stored at a constant temperature of 40 °C for four weeks, the TACI-Fc fusion protein is still able to maintain a purity of 94% or higher (SEC%); in some embodiments, the TACI-Fc has a pI value of less than 9, less than 8, less than 7, less than 6, or less, as determined by "18cProt/TrEMBL" system analysis.

In some embodiments, the fusion protein comprising a TACI polypeptide and a BCMA polypeptide provided above by the present disclosure has one or more of the following functional activities:
a. being not prone to producing fragments resulting from the cleavage of the TACI polypeptide; in some embodiments, the cleavage may be detected by mass spectrometry, e.g., the TACI cleavage analysis method in Example 2;
b. the ability to bind to BAFF; in some embodiments, the fusion protein binds to BAFF with an EC₅₀ value of less than 1 nM, 0.5 nM, 0.4 nM, 0.3 nM, 0.2 nM, 0.1 nM, or less, as determined by an ELISA method, e.g., the ELISA assay of Example 5;
c. the ability to bind to APRIL; in some embodiments, the fusion protein binds to APRIL with an EC₅₀ value of less than 1 nM, 0.5 nM, 0.4 nM, 0.3 nM, 0.2 nM, 0.1 nM, or less, as determined by an ELISA method, e.g., the ELISA assay of Example 5;
d. a good *in vivo* pharmacokinetic profile; and
e. good stability.

In some embodiments, the antibody fusion protein comprising a TACI polypeptide, a BCMA polypeptide, and an anti-IFNAR1 antibody provided above by the present disclosure has one or more of the following functional activities:
a. being not prone to producing fragments resulting from the cleavage of the TACI polypeptide; in some embodiments, the cleavage may be detected by mass spectrometry, e.g., the TACI cleavage analysis method in Example 2;
b. inhibiting the activity of type I interferons; in some embodiments, the fusion protein has an inhibition rate against the activity of IFNs consistent with or similar to that of an anti-IFNAR1 antibody (e.g., anifrolumab), wherein the inhibition rate is determined by an IFNα/β reporter gene method *in vitro,* e.g., the experiment in Example 10; the inhibition rate is determined by assessing the expression of the mRNA of the ISG gene downstream of IFNα in PBMCs *in vivo,* e.g., the experiment in Example 21;
c. the ability to bind to BAFF; in some embodiments, the fusion protein binds to BAFF with an EC₅₀ value of less than 1.5 nM, 1 nM, 0.5 nM, 0.4 nM, 0.3 nM, 0.2 nM, 0.1 nM, or less, as determined by an ELISA method, e.g., the ELISA assay of Example 11;
d. the ability to bind to APRIL; in some embodiments, the fusion protein binds to APRIL with an EC₅₀ value of less than 0.5 nM, 0.4 nM, 0.3 nM, 0.2 nM, 0.1 nM, or less, as determined by an ELISA method, e.g., the ELISA assay of Example 11;
e. inhibiting IFNα secretion; in some embodiments, the fusion protein has a capability to inhibit the secretion of cytokine IFN-α from plasmacytoid dendritic cells (pDCs) consistent with or similar to that of an anti-IFNAR1 antibody (e.g., anifrolumab), wherein the inhibition capability is measured, e.g., by the method in Example 15; in some embodiments, the fusion protein has a capability to inhibit IFNα-induced *in vitro* differentiation of B cells into plasma cells consistent with or similar to that of an anti-IFNAR1 antibody (e.g., anifrolumab), wherein the inhibition capability is measured, e.g., by the method in Example 16;
f. inhibiting plasma cell differentiation and inhibiting BAFF-induced B cell proliferation; in some embodiments, the fusion protein inhibits BAFF-induced B cell proliferation, with an inhibition rate of at least 50%, at least 40%, at least 30%, at least 20%, or at least 10%, wherein the inhibition rate is the ratio of the B cell count after the antibody fusion protein is added to the B cell count after IgG1, at an equal concentration, is added and is determined by FACS, e.g., the method in Example 17;
g. inhibiting APRIL-induced B cell proliferation; in some embodiments, the fusion protein inhibits APRIL-induced B cell proliferation, with an inhibition rate of at least 50%, at least 40%, at least 30%, at least 20%, or at least 10%, wherein the inhibition rate is the ratio of the B cell count after the antibody fusion protein is added to the B cell count after IgG1, at an equal concentration, is added and is determined by FACS, e.g., the method in Example 18;
h. inhibiting BAFF and APRIL-induced plasma cell production; in some embodiments, the fusion protein inhibits BAFF and APRIL-induced plasma cell production, with an inhibition rate of at least 90%, at least 80%, at least 70%, at least 60%, at least 50%, at least 40%, at least 30%, or at least 20%, wherein the inhibition rate is the ratio of the B cell count after the antibody fusion protein is added to the B cell count after IgG1, at an equal concentration, is added and is determined by FACS, e.g., the method in Example 19;
i. inhibiting IgA production *in vivo;* in some embodiments, the fusion protein inhibits IgA production *in vivo,* with an inhibition rate of at least 80%, at least 70%, at least 60%, at least 50%, at least 40%, or at least 30%, wherein the inhibition rate is relative to an equal concentration of PBS and is determined by ELISA, e.g., the method in Example 22;
j. a good *in vivo* pharmacokinetic profile; and
k. good stability.

In addition, in some embodiments, a product or combination is provided, the product or combination comprising any one of the fusion proteins comprising a TACI polypeptide and a BCMA polypeptide described above in the present disclosure and further an antibody. In some embodiments, a product or combination is provided, the product or combination comprising any one of the TACI polypeptides described above in the present disclosure and further an antibody. In some embodiments, the antibody is an anti-IFNAR1 antibody, e.g., anifrolumab.

In some embodiments, a complex is provided, the complex comprising any one of the TACI polypeptides described above in the present disclosure and any one of the BCMA polypeptides described above in the present disclosure. Optionally, the TACI polypeptide and the BCMA polypeptide may be operably linked directly or by any one of the linkers described above in the present disclosure, or may not be linked.

In some embodiments, a complex is provided, the complex comprising: any one of the TACI polypeptides described above in the present disclosure and any one of the BCMA polypeptides described above in the present disclosure; or any one of the TACI polypeptides described above in the present disclosure and any one of the anti-IFNAR1 antibodies described above in the present disclosure; or any one of the BCMA polypeptides described above in the present disclosure and any one of the anti-IFNAR1 antibodies described above in the present disclosure; or any one of the TACI polypeptides described above in the present disclosure and any one of the anti-IFNAR1 antibodies described above in the present disclosure and any one of the BCMA polypeptides described above in the present disclosure. Optionally, the TACI polypeptide, the BCMA polypeptide, and the anti-IFNAR1 antibody may be operably linked directly or by any one of the linkers described above in the present disclosure, or may not be linked.

### Polynucleotide and Vector

The present disclosure provides an (isolated) polynucleotide encoding any one of the TACI polypeptides, the BCMA polypeptides, the fusion proteins comprising a TACI polypeptide and a BCMA polypeptide, the fusion proteins comprising a TACI polypeptide and an antibody (e.g., an anti-IFNAR1 antibody), the fusion proteins comprising a TACI polypeptide and a BCMA polypeptide and an antibody (e.g., an anti-IFNAR1 antibody), the products, the combinations, or the complexes described above in the present disclosure. The polynucleotide may be DNA or RNA (e.g., mRNA).

The nucleic acid of the present disclosure may also be in the form of a vector, and it may be present in the vector and/or may be part of the vector. The vector is, e.g., a plasmid, cosmid, YAC, or viral vector. The vector may especially be an expression vector, i.e., a vector that provides for *in vitro* and/or *in vivo* (i.e., in suitable host cells, host organisms, and/or expression systems) expression of the CD40-binding molecule. The expression vector generally comprises at least one of the nucleic acids of the present disclosure, which is operatively linked to one or more suitable expression regulatory elements (e.g., promoters, enhancers, and terminators). The selection of the elements and their sequences for expression in a particular host is within the knowledge of those skilled in the art. Regulatory elements and other elements useful or necessary for the expression of the TACI polypeptides, the BCMA polypeptides, the fusion proteins comprising a TACI polypeptide and a BCMA polypeptide, the fusion proteins comprising a TACI polypeptide and an antibody (e.g., an anti-IFNAR1 antibody), the fusion proteins comprising a TACI polypeptide and a BCMA polypeptide and an antibody (e.g., an anti-IFNAR1 antibody), the products, the combinations, or the complexes of the present disclosure include, e.g., promoters, enhancers, terminators, integration factors, selection labels, leader sequences, and reporter genes.

The nucleic acid of the present disclosure may be prepared or obtained by known means (e.g., by automatic DNA synthesis and/or recombinant DNA techniques) based on information on the amino acid sequence of the polypeptide of the present disclosure, and/or may be isolated from a suitable natural source.

### Host Cell

The present disclosure provides a recombinant host cell for expressing or capable of expressing one or more of the TACI polypeptides, the BCMA polypeptides, the fusion proteins comprising a TACI polypeptide and a BCMA polypeptide, the fusion proteins comprising a TACI polypeptide and an antibody (e.g., an anti-IFNAR1 antibody), the fusion proteins comprising a TACI polypeptide and a BCMA polypeptide and an antibody (e.g., an anti-IFNAR1 antibody), the products, the combinations, or the complexes of the present disclosure, and/or comprising the nucleic acid or vector of the present disclosure.

In some embodiments, the host cell is a bacterial cell, a fungal cell, or a mammalian cell.

Bacterial cells include, e.g., cells of gram-negative bacterial strains (e.g., *Escherichia coli* strains, *Proteus* strains, and *Pseudomonas* strains), and gram-positive bacterial strains (e.g., *Bacillus* strains, *Streptomyces* strains, *Staphylococcus* strains, and *Lactococcus* strains).

Fungal cells include, e.g., cells of the species *Trichoderma, Neurospora,* and *Aspergillus;* or cells of the species *Saccharomyces* (e.g., *Saccharomyces cerevisiae*), *Schizosaccharomyces* (e.g., *Schizosaccharomyces pombe*), *Pichia* (e.g., *Pichia pastoris* and *Pichia methanolica*), and *Hansenula.*

Mammalian cells include, for example, HEK293 cells, CHO cells, BHK cells, HeLa cells, COS cells, and the like.

However, amphibian cells, insect cells, plant cells, and any other cells used in the art for expressing heterologous proteins may also be used in the present disclosure.

The cells of the present disclosure are unable to develop into complete plants or animal individuals.

### Preparation Method

The present disclosure provides a method for preparing a TACI polypeptide, a BCMA polypeptide, a fusion protein comprising a TACI polypeptide and a BCMA polypeptide, a fusion protein comprising a TACI polypeptide and an antibody (e.g., an anti-IFNAR1 antibody), a fusion protein comprising a TACI polypeptide and a BCMA polypeptide and an antibody (e.g., an anti-IFNAR1 antibody), a product, a combination, or a complex, the method comprising expressing the aforementioned polypeptides, fusion proteins, product, combination, or complex in a host cell as described above and isolating the polypeptides, fusion proteins, product, combination, or complex from the host cell. Optionally, the method may further comprise a purification step. Optionally, the method may further comprise a filtration step and a concentration step. Soluble mixtures and polymers can also be removed by conventional methods, such as molecular sieves and ion exchange. The resulting products need to be immediately frozen, such as at -70 °C, or lyophilized.

The polypeptides, fusion proteins, product, combination, or complex may be prepared and purified using conventional methods. For example, a cDNA sequence encoding the fusion protein can be cloned and recombined into an expression vector. The recombinant expression vector can stably transfect CHO cells. Mammalian expression systems can result in protein glycosylation (e.g., at the highly conserved N-terminus of the Fc region). Positive clones are obtained and expanded in a culture medium in a bioreactor to produce protein. The culture medium with secreted protein can be purified, collected, filtered, and concentrated using conventional techniques. Soluble mixtures and multimers can also be removed by conventional methods, such as molecular sieves and ion exchange. However, the polypeptides, fusion proteins, products, combinations, or complexes of the present disclosure may also be obtained by other protein production methods known in the art, such as chemical synthesis, including solid-phase or liquid-phase synthesis.

### Composition

The present disclosure provides a composition, e.g., a pharmaceutical composition, comprising a therapeutically effective amount of a TACI polypeptide, a BCMA polypeptide, a fusion protein comprising a TACI polypeptide and a BCMA polypeptide, a fusion protein comprising a TACI polypeptide and an antibody (e.g., an anti-IFNAR1 antibody), a fusion protein comprising a TACI polypeptide and a BCMA polypeptide and an antibody (e.g., an anti-IFNAR1 antibody), a product, a combination, a complex, or a polynucleotide, and one or more pharmaceutically acceptable excipients, diluents, or carriers.

In some specific embodiments, a unit dose of the pharmaceutical composition may comprise 0.01 to 99 weight% of any one of the polypeptides, fusion proteins, product, combination, complex, or polynucleotide according to the foregoing. In some specific embodiments, the amount of any one of the polypeptides, fusion proteins, product, combination, complex, or polynucleotide according to the foregoing in a unit dose of the pharmaceutical composition is 0.1-2000 mg; in some specific embodiments, the amount is 1-1000 mg.

### Kit

The present disclosure provides a kit comprising a TACI polypeptide, a BCMA polypeptide, a fusion protein comprising a TACI polypeptide and a BCMA polypeptide, a fusion protein comprising a TACI polypeptide and an antibody (e.g., an anti-IFNAR1 antibody), a fusion protein comprising a TACI polypeptide and a BCMA polypeptide and an antibody (e.g., an anti-IFNAR1 antibody), a product, a combination, a complex, or a polynucleotide of the present disclosure. In some embodiments, a diagnostic reagent comprising the polynucleotide described above and related diagnostic use are further provided.

### Method for Treating Disease and Pharmaceutical Use

In some embodiments, the present disclosure provides a method for treating or ameliorating a disease or disorder, the method comprising administering to a subject in need thereof any one of the TACI polypeptides, the BCMA polypeptides, the fusion proteins comprising a TACI polypeptide and a BCMA polypeptide, the fusion proteins comprising a TACI polypeptide and an antibody (e.g., an anti-IFNAR1 antibody), the fusion proteins comprising a TACI polypeptide and a BCMA polypeptide and an antibody (e.g., an anti-IFNAR1 antibody), the products, the combinations, the complexes, the polynucleotides, or the pharmaceutical compositions described above in the present disclosure. In some embodiments, the disease or disorder is a disease or disorder associated with TACI, BCMA, and/or IFN expression.

In some embodiments, provided is a method for treating a disease or disorder with the combination of any one of the TACI polypeptides described above in the present disclosure, any one of the BCMA polypeptides described above in the present disclosure, and any one of the anti-IFNAR1 antibodies described above in the present disclosure, or pharmaceutical use of any one of the TACI polypeptides described above in the present disclosure, any one of the BCMA polypeptides described above in the present disclosure, and any one of the anti-IFNAR1 antibodies described above in the present disclosure. In some embodiments, provided is a method for treating a disease or disorder with the combination of any one of the TACI polypeptides described above in the present disclosure and any one of the anti-IFNAR1 antibodies described above in the present disclosure, or pharmaceutical use of any one of the TACI polypeptides described above in the present disclosure and any one of the anti-IFNAR1 antibodies described above in the present disclosure; in some specific embodiments, use in combination with a BCMA polypeptide is further included. In some embodiments, provided is a method for treating a disease or disorder with any one of the fusion proteins comprising a BCMA polypeptide and an anti-IFNAR1 antibody described above in the present disclosure, or pharmaceutical use of any one of the fusion proteins comprising a BCMA polypeptide and an anti-IFNAR1 antibody described above in the present disclosure; in some specific embodiments, use in combination with a TACI polypeptide is further included. The anti-IFNAR1 antibody is, for example, anifrolumab.

In some embodiments, provided is a method for treating or ameliorating a B cell disorder or an autoimmune disease, the method comprising a step of administering to a subject in need thereof a therapeutically effective amount of a TACI polypeptide, a BCMA polypeptide, a fusion protein comprising a TACI polypeptide and a BCMA polypeptide, a fusion protein comprising a TACI polypeptide and an antibody (e.g., an anti-IFNAR1 antibody), a fusion protein comprising a TACI polypeptide and a BCMA polypeptide and an antibody (e.g., an anti-IFNAR1 antibody), a product, a combination, a complex, a polynucleotide, or a pharmaceutical composition of the present disclosure.

In some embodiments, provided is use of a TACI polypeptide, a BCMA polypeptide, a fusion protein comprising a TACI polypeptide and a BCMA polypeptide, a fusion protein comprising a TACI polypeptide and an antibody (e.g., an anti-IFNAR1 antibody), a fusion protein comprising a TACI polypeptide and a BCMA polypeptide and an antibody (e.g., an anti-IFNAR1 antibody), a product, a combination, a complex, a polynucleotide, or a pharmaceutical composition of the present disclosure in the preparation of a medicament for treating or preventing a disease.

In some embodiments, the disease or disorder is a B cell disorder or an autoimmune disease.

In some embodiments, the disease or disorder is associated with TACI expression, or with BCMA expression, or with IFN expression, or with the expression of any two or three of TACI, BCMA, and IFNs. The expression is, for example, overexpression or aberrant expression.

In some embodiments, the autoimmune disease is selected from the group consisting of systemic lupus erythematosus, myasthenia gravis, multiple sclerosis, insulin-dependent diabetes mellitus, Crohn's disease, rheumatoid arthritis, polyarticular juvenile rheumatoid arthritis, and psoriatic arthritis; in some embodiments, the B cell disorder is selected from the group consisting of tumors, chronic leukocytic leukemia, multiple myeloma, non-Hodgkin lymphoma, post-transplant lymphoproliferative disorder, and light chain gammopathy. In some embodiments, the autoimmune disease is systemic lupus erythematosus.

### Definitions of Terms

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains. The three-letter and single-letter codes for amino acids used in the present disclosure are as described in J. biol. chem., 243, p3558 (1968).

As used in the present disclosure, the singular forms "a", "an", and "the" include plural referents unless the context clearly dictates otherwise.

Unless otherwise clearly stated in the context, throughout the description and the claims, the words "comprise", "have", "include", and the like, should be construed in an inclusive sense as opposed to an exclusive or exhaustive sense.

The term "cytokine" is a general term for proteins that are released by a cell population to act on other cells as intercellular mediators. Examples of such cytokines include lymphokines, monokines, chemokines, and traditional polypeptide hormones. Exemplary cytokines include: IL-2, IFN-γ, IL-6, TNFα, IL-17, and IL-5.

As described in the present disclosure, TACI (transmembrane activator and calcium modulator and cyclophilin ligand-interactor) is a membrane-bound receptor. Human TACI belongs to the TNFR superfamily, is a polypeptide of 293 amino acids, and contains a protein N-terminal region (amino acid residues 1-165, see SEQ ID NO: 1 of the present disclosure), a transmembrane region (amino acid residues 166-186), and an intracellular region (amino acid residues 187-293). The extracellular region contains two CRDs (cysteine-rich pseudo-repeats) (CRD1 and CRD2). The "CRD1" of TACI in the present disclosure encompasses wild-type human TACI's CRD1 (e.g., amino acids at positions 33-67 of SEQ ID NO: 1) and variants thereof. The "CRD2" of TACI in the present disclosure encompasses wild-type human TACI's CRD2 (e.g., amino acids at positions 70-104 or 69-104 of SEQ ID NO: 1) and variants thereof (including, but not limited to, variants of one or more of the amino acids at positions 69, 72, 73, 77, 85, 102, and 103 of SEQ ID NO: 1 of the present disclosure, e.g., the variants set forth in SEQ ID NOs: 6-29). The CRD1 and CRD2 of TACI and variants thereof are all capable of binding to APRIL and/or BAFF, e.g., APRIL and BAFF simultaneously, and methods for detecting the binding are well known in the art. See, e.g., those provided in the examples of the present disclosure.

As described in the present disclosure, BCMA (B cell maturation antigen) is a membrane-bound receptor. Human BCMA protein is a polypeptide of 184 amino acids and contains a protein N-terminal region (amino acid residues 1-50; see amino acids at positions 1-50 of SEQ ID NO: 30 of the present disclosure), a transmembrane region (amino acid residues 51-93), and an intracellular region (amino acid residues 94-184). The extracellular region of BCMA contains a CRD (or referred to as CRD1). The "CRD1" of BCMA in the present disclosure encompasses wild-type human BCMA's CRD1 (e.g., amino acids at positions 7-41 of SEQ ID NO: 30 of the present disclosure) and variants thereof. The CRD1 of BCMA and variants thereof are all capable of binding to APRIL and/or BAFF, e.g., APRIL and BAFF simultaneously, and methods for detecting the binding are well known in the art. See, e.g., those provided in the examples of the present disclosure.

As used in the present disclosure, "TACI extracellular region" and "TACI extracellular domain" are interchangeable with each other, and "BCMA extracellular region" and "BCMA extracellular domain" are interchangeable with each other.

The terms "interferon α", "IFNα", "IFNa", "IFNA", and "IFN alpha" are used interchangeably and intended to refer to IFNα proteins encoded by a functional gene of the interferon a gene locus, which has 75% or greater sequence identity to IFNα1 (the protein encoded by GenBank accession number NP_076918 or GenBank accession number NM_024013). Examples of IFNα subtypes include IFNα1, α2a, α2b, α4, α4b α5, α6, α7, α8, α10, α13, α14, α16, α17, and α21. "Interferon α" and "IFNα" are intended to encompass recombinant forms of the various IFNα subtypes, as well as naturally occurring preparations that comprise IFNα proteins, such as leukocyte IFN and lymphoblast IFN.

The terms "Interferon α receptor-1", "IFNAR1", "IFNAR-1", and "IFNAR-1 antigen" are used interchangeably and include variants, isoforms, species homologs of human IFNAR-1, and analogs having at least one common epitope with IFNAR-1. Therefore, in some embodiments, human antibodies of the present disclosure may cross-react with IFNAR-1 from species other than human or other proteins structurally related to human IFNAR-1 (e.g., human IFNAR-1 homologs). In other embodiments, the antibodies may be completely specific for human IFNAR-1 and not exhibit species or other types of cross-reactivity. The complete cDNA sequence of human IFNAR-1 has the GenBank accession number NM_000629.

The term "and/or" is intended to include two meanings, "and" and "or". For example, the phrase "A, B, and/or C" is intended to encompass each of the following: A, B, and C; A, B, or C; A or C; A or B; B or C; A and C; A and B; B and C; A (alone); B (alone); and C (alone).

The term "antibody" is used in the broadest sense and encompasses a variety of antibody structures, including, but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) so long as they exhibit the desired antigen-binding activity. An antibody may refer to an immunoglobulin that is a four-peptide-chain structure formed by linking two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, or isotypes of immunoglobulins, namely IgM, IgD, IgG, IgA, and IgE, with their corresponding heavy chains being µ chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into κ or λ chains according to differences in the constant regions. Each of the five classes of Ig may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (CDRs) and 4 framework regions (FRs) with relatively conservative sequences. The 3 hypervariable regions determine the specificity of the antibody and thus are also known as complementarity-determining regions (CDRs). Each of the light chain variable regions (VLs) and the heavy chain variable regions (VHs) consists of 3 CDRs and 4 FRs arranged from the amino-terminus to the carboxyl-terminus in the following order: FR1, CDR1, FR2, CDR2, FR3, CDR3, and FR4. The 3 CDRs of the light chain refer to LCDR1, LCDR2, and LCDR3; and the 3 CDRs of the heavy chain refer to HCDR1, HCDR2, and HCDR3. As used in the present disclosure, "antibody" encompasses "antigen-binding fragment", and the "antigen-binding fragment" includes a single-chain antibody (i.e., full-length heavy and light chains); Fab, a modified Fab, Fab', a modified Fab', F(ab')2, Fv, Fab-Fv, Fab-dsFv, a single-domain antibody (e.g., VH or VL or VHH), scFv, a bivalent or trivalent or tetravalent antibody, Bis-scFv, a diabody, a tribody, a triabody, a tetrabody and an epitope-binding fragment of any one of the above (see, e.g., Holliger and Hudson, 2005, Nature Biotech. 23 (9):1126-1136; Adair and Lawson, 2005, Drug Design Reviews-Online 2 (3), 209-217).

For the determination or definition of "CDRs", the deterministic depiction of CDRs and identification of residues comprising antigen-binding sites of the antibody can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition. The Kabat numbering scheme is a standard for numbering residues in antibodies and is generally used to identify CDRs (see, e.g., Johnson & Wu, 2000, Nucleic Acids Res., 28:214-8). The Chothia numbering scheme is similar to the Kabat numbering scheme, except that it takes into account the position of certain structural loop regions. (see, e.g., Chothia et al., 1986, J. Mol. Biol., 196:901-17; Chothia et al., 1989, Nature, 342:877-83). The AbM numbering scheme adopts a computer program integration suite for modeling antibody structures manufactured by Oxford Molecular Group (see, e.g., Martin et al., 1989, Proc Natl Acad Sci (USA), 86:9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3:194-198). The contact definition is based on the analysis of the available complex crystal structures (see, e.g., MacCallum et al., 1996, J. Mol. Biol., 5:732-45). In the conformational definition, the positions of the CDRs can be identified as residues that contribute to antigen binding (see, e.g., Makabe et al., 2008, Journal of Biological Chemistry, 283:1156-1166). In addition, other CDR boundary definitions may not strictly follow one of the methods described above, but still overlap with at least a portion of the Kabat CDRs, although they may be shortened or lengthened based on predictions or experimental results that a particular residue or a particular group of residues do not significantly affect the antigen binding. As used in the present disclosure, a CDR may refer to a CDR defined by any method known in the art, including combinations of methods. The correspondence between the various numbering schemes is well known to those skilled in the art, and examples are shown in Table 1 below.

**Table 1. Relationships between CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the Kabat numbering scheme is applied to the sequences of the variable regions and CDRs in the present disclosure.

The term "Fc region" or "fragment crystallizable region" is used to define the C-terminal region of the heavy chain of an antibody, including native Fc regions and engineered Fc regions. In some embodiments, the Fc region comprises two identical or different subunits. In some embodiments, the Fc region of the human IgG heavy chain is defined as extending from the amino acid residue at position Cys226 or from Pro230 to its carboxyl terminus. Suitable Fc regions used for the antibodies described herein include Fc regions of human IgG1, IgG2 (IgG2A and IgG2B), IgG3, and IgG4. In some embodiments, the boundaries of the Fc region may also be varied, for example, by deleting the C-terminal lysine of the Fc region (residue 447 according to the EU numbering scheme) or deleting the C-terminal glycine and lysine of the Fc region (residues 446 and 447 according to the EU numbering scheme). Unless otherwise stated, the numbering scheme for the Fc region is the EU numbering scheme, also known as the EU index.

The term "homology" or "identity" refers to sequence similarity between two polynucleotide sequences or between two polypeptides. When positions in two compared sequences are occupied by identical nucleotides or amino acid monomer subunits, e.g., if the position of each of two DNA molecules is occupied by an identical nucleotide, the molecules are homologous at that position. The homology percentage between two sequences is a function of the number of matching or homologous positions shared by the two sequences divided by the number of positions compared × 100%. For example, if 6 out of 10 positions are matched or homologous when two sequences are optimally aligned, the two sequences are 60% homologous. In general, when two sequences are aligned, comparison is performed to obtain the maximum homology percentage.

The term "amino acid mutation" includes amino acid substitutions (also known as amino acid replacements), deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., the replacement of one amino acid with another amino acid having different structural and/or chemical properties. Amino acid substitutions include replacement with non-naturally occurring amino acids or with derivatives of the 20 native amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 366W indicates that an amino acid residue at position 366 is W. T366W indicates that an amino acid residue at position 366 is mutated from the original T to W.

The term "conservative replacement" refers to replacement by another amino acid residue having similar properties to the original amino acid residue. For example, lysine, arginine, and histidine have similar properties in that they have basic side chains, and aspartic acid and glutamic acid have similar properties in that they have acidic side chains. Additionally, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is replaced, it will not exhibit a particular change in properties.

The term "polypeptide" or "protein" is used interchangeably herein and refers to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized in a manner similar to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA). "Isolated" nucleic acid refers to a polynucleotide that has been separated from components of its natural environment. The isolated nucleic acid includes a polynucleotide comprised in a cell that generally comprises the polynucleotide, but the polynucleotide is present extrachromosomally or at a chromosomal position different from its natural chromosomal position. An isolated nucleic acid encoding a polypeptide or a fusion protein refers to one or more polynucleotides encoding the polypeptide or fusion protein, including such one or more polynucleotides in a single vector or separate vectors, and such one or more polynucleotides present at one or more positions in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with mixed bases and/or deoxyinosine residues.

The term "effector function" refers to biological activities that can be attributed to the Fc region of an antibody (either the native sequence Fc region or the amino acid sequence variant Fc region) and vary with the antibody isotype. Examples of effector functions of an antibody include, but are not limited to: C1q binding and complement-dependent cytotoxicity, Fc receptor binding, antibody-dependent cell-mediated cytotoxicity (ADCC), phagocytosis, down-regulation of cell surface receptors (e.g., B cell receptors), and B cell activation.

The term "binding affinity" or "affinity" is used in the present disclosure as a measure of the strength of a non-covalent interaction between two molecules (e.g., between an antibody or a portion thereof and an antigen or between a ligand and a receptor). The binding affinity between two molecules can be quantified by determining the dissociation constant (KD). K_{D} can be determined by measuring the kinetics of complex formation and dissociation by using, e.g., the surface plasmon resonance (SPR) method (Biacore). The rate constants corresponding to the association and dissociation of a monovalent complex are referred to as the association rate constant ka (or kon) and the dissociation rate constant kd (or koff), respectively. K_{D} is related to ka and kd by the equation K_{D} = kd/ka. The value of the dissociation constant can be determined directly by well-known methods and can be calculated by methods such as those described by Caceci et al (1984, Byte 9:340-362) even for complex mixtures. For example, K_{D} can be determined by using a dual filtration nitrocellulose filter binding assay such as that disclosed by Wong & Lohman (1993, Proc. Natl. Acad. Sci. USA 90:5428-5432). Other standard assays for evaluating the binding ability of an antibody to a target antigen are known in the art and include, for example, ELISA, western blot, RIA, and flow cytometry, as well as other assays exemplified elsewhere in the present disclosure. The binding kinetics and binding affinity of the antibody can also be evaluated by standard assays known in the art, such as surface plasmon resonance (SPR), e.g., by using the Biacore^{™} system or KinExA. The binding affinities associated with different molecular interactions, e.g., the binding affinities of different antibodies for a given antigen, can be compared by comparing the K_{D} values of antibody/antigen complexes. Similarly, the specificity of an interaction can be evaluated by determining and comparing the K_{D} value for the interaction of interest (e.g., a specific interaction between an antibody and an antigen) with the K_{D} value for an interaction not of interest (e.g., a control antibody known not to bind to the target antigen).

The term "linker" refers to a linker unit that links two polypeptide fragments, and generally has some flexibility, such that the use of the linker does not result in the loss of the original function of the protein domain. Herein, the linkers present in the same structure may be identical or different. The linker may be a peptide linker comprising one or more amino acids, typically about 1-30, 2-24, or 3-15 amino acids. The linkers used in the present disclosure may be identical or different.

The term "fusion" or "linkage" means that components (e.g., a TACI polypeptide and a BCMA polypeptide) are linked by a covalent bond, either directly or via one or more linkers. When the linker is a peptide linker, the covalent bond is a peptide bond.

"Giving", "administering", and "treating", when applied to animals, humans, experimental subjects, cells, tissues, organs, or biological fluid, refer to contact of an exogenous drug, a therapeutic agent, a diagnostic agent, or a composition with the animals, humans, subjects, cells, tissues, organs, or biological fluid. "Giving", "administering", and "treating" can refer to, for example, therapeutic, pharmacokinetic, diagnostic, research, and experimental methods. The treatment of cells comprises contacting a reagent with cells and contacting the reagent with a fluid, where the fluid is in contact with the cells. "Giving", "administering", and "treating" also refer to treating, e.g., a cell, by a reagent, diagnosis, a binding composition, or by another cell *in vitro* and *ex vivo.* "Treating", when applied to humans, veterinary or research subjects, refers to therapeutic treatment, preventive or prophylactic measures, and research and diagnostic applications.

"Treatment" refers to administering a therapeutic agent, such as a composition comprising any one of the antibodies or the antigen-binding fragments thereof or fusion proteins thereof of the present disclosure, either internally or externally to a subject who has had, is suspected of having, or is predisposed to having one or more diseases or symptoms thereof on which the therapeutic agent is known to have a therapeutic effect. Generally, the therapeutic agent is administered in an amount effective to alleviate one or more disease symptoms in the subject or population being treated, whether by inducing regression of such symptoms or inhibiting the development of such symptoms into any clinically measurable degree. The amount of therapeutic agent effective to alleviate any particular disease symptom (also referred to as the "therapeutically effective amount") may vary depending on factors such as the disease state, the age and weight of the subject, and the capability of the drug to produce a desired therapeutic effect in the subject. Whether a disease symptom has been alleviated can be evaluated by any clinical testing methods commonly used by doctors or other health care professionals to evaluate the severity or progression of the symptom. Although embodiments of the present disclosure (e.g., treatment methods or products) may be ineffective in alleviating the disease symptom of interest in a certain subject, they shall alleviate the disease symptom of interest in a statistically significant number of subjects as determined by any statistical test method known in the art, such as the Student's t-test, Chi-square test, U-test by Mann and Whitney, Kruskal-Wallis test (H-test), Jonckheere-Terpstra test, and Wilcoxon test.

"Effective amount" comprises an amount sufficient to ameliorate or prevent a symptom or sign of a medical disorder. An effective amount also means an amount sufficient to allow or facilitate diagnosis. The effective amount for a particular subject or veterinary subject may vary depending on factors such as the disorder to be treated, the general health of the subject, the method and route and dosage of administration, and the severity of side effects. An effective amount may be the maximum dose or administration regimen to avoid significant side effects or toxic effects.

The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progeny derived therefrom, regardless of the number of passages. Progeny may not be completely identical to parent cells in terms of nucleic acid content and may contain mutations. Mutant progenies that have the same function or biological activity as the cells screened or selected from the group consisting of the initially transformed cells are included herein. Host cells include prokaryotic and eukaryotic host cells, wherein eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells.

"Optional" or "optionally" means that the event or circumstance subsequently described may, but does not necessarily occur; this description includes instances where the event or circumstance occurs or does not occur. For example, "optionally comprising 1-3 antibody heavy chain variable regions" means that the antibody heavy chain variable region of a particular sequence may, but does not necessarily, exist.

As used in the present disclosure, "a fusion protein comprising a TACI polypeptide and a BCMA polypeptide" encompasses all molecules of fusion proteins comprising a TACI polypeptide and a BCMA polypeptide of the present disclosure; "a fusion protein comprising a TACI polypeptide and an anti-IFNAR1 antibody" encompasses all molecules of fusion proteins comprising a TACI polypeptide and an anti-IFNAR1 antibody (or an antigen-binding fragment thereof) of the present disclosure; "a fusion protein comprising a TACI polypeptide and a BCMA polypeptide and an anti-IFNAR1 antibody (or an antigen-binding fragment thereof)" encompasses all molecules of fusion proteins comprising a TACI polypeptide and a BCMA polypeptide and an anti-IFNAR1 antibody (or an antigen-binding fragment thereof) of the present disclosure. For example, the fusion protein may comprise one or more effector molecules, e.g., in the form of a conjugate. The "effector molecule" alone may be therapeutically active (e.g., have anti-tumor activity or immune-activating or inhibiting activity) or have a detecting function, and may be in any form, such as biologically active proteins (e.g. enzymes), other antibody or antibody fragments, synthetic or naturally occurring polymers, nucleic acids and fragments thereof (DNA and RNA and fragments thereof), radionuclides (particularly radioiodides), radioisotopes, chelated metals, nanoparticles and reporter groups (e.g., fluorescent compounds) or compounds that can be detected by NMR or ESR spectroscopy. Conjugation of effector molecules to the fusion proteins of the present disclosure can be achieved by conventional methods.

### BRIEF DESCRIPTION OF THE DRAWINGS

FIG. 1 shows a schematic diagram of the structure of fusion proteins of TACI and Fc.
FIG. 2 shows a schematic diagram of ligands BAFF and APRIL interacting with receptors BAFF-R, TACI, and BCMA. BAFF is a membrane expressed protein and functions in the form of a soluble trimer after undergoing protease digestion. BAFF can bind to BAFF-R, TACI, and BCMA. APRIL is a soluble trimeric protein and can bind to TACI and BCMA. The thicker the black arrows, the stronger the interactions. The thinner the black arrows, the weaker the interactions.
FIG. 3 shows schematic diagrams of different TACI and BCMA protein domains, including TACI-ECD, TACI-d2, TACI-T2, TACI-T4, and BCMA-ECD.
FIG. 4 shows schematic diagrams of the structures of TACI-BCMA fusion proteins, including B575701, B575702, B575703, and B575704.
FIG. 5 shows the capabilities of TACI-BCMA fusion proteins (B575701, B575702, B575703, and B575704) to bind to BAFF as measured by ELISA, with telitacicept and IgG1 isotype as controls.
FIG. 6 shows the capabilities of TACI-BCMA fusion proteins (B575701, B575702, B575703, and B575704) to bind to APRIL as measured by ELISA, with telitacicept and IgG1 isotype as controls.
FIG. 7 shows schematic diagrams of domains TACI-CRD2 and TACI-19-16, including TACI-CRD2 and TACI-19-16.
FIG. 8 shows schematic diagrams of the structures of fusion proteins of anifrolumab and TACI-T4 (B385801, B385802, B385803, B385804, B498301, and B498302) and fusion proteins of anifrolumab and TACI-19-16 (B606401, B606401-LALA, B805201, and B805201-LALA; the LALA mutations are mutations L234A/L235A in Fc, and the same applies hereinafter).
FIG. 9 shows the inhibitory effects of fusion proteins of anifrolumab and TACI-T4 on the activity of IFN-β, wherein A of FIG. 9 shows the inhibitory effects of B385801, B385802, B385803, and B385804 on the activity of IFN-β; B of FIG. 9 shows the inhibitory effects of B385804, B498301, and B498302 on the activity of IFN-β; FIG. 9C shows the inhibitory effect of B606401 on the activity of IFN-β, and A-C of FIG. 9 all use anifrolumab and IgG1 isotype as controls.
FIG. 10 shows the capabilities of fusion proteins of anifrolumab and TACI-T4 to bind to BAFF as measured by ELISA, wherein A of FIG. 10 shows the capabilities of B385801, B385802, B385803, and B385804 to bind to BAFF, with atacicept, telitacicept, and IgG1 isotype as controls; B of FIG. 10 shows the capabilities of B385804, B498301, and B498302 to bind to BAFF; C of FIG. 10 shows the capability of B606401 to bind to BAFF; B-C of FIG. 10 both use telitacicept and IgG1 isotype as controls.
FIG. 11 shows schematic diagrams of the structures of fusion proteins of anifrolumab and TACI-BCMA, including B613301, B613302, B613303, and B613304.
FIG. 12 shows the inhibitory effects of fusion proteins of anifrolumab and TACI-BCMA (B613301, B613302, and B613303) on the activity of IFN-β, with anifrolumab and IgG1 isotype as controls.
FIG. 13 shows the capabilities of fusion proteins of anifrolumab and TACI-BCMA (B613301, B613302, and B613303) to bind to BAFF as measured by ELISA, with telitacicept and IgG1 isotype as controls.
FIG. 14 shows the capabilities of fusion proteins of anifrolumab and TACI-BCMA (B613301, B613302, and B613303) to bind to APRIL as measured by ELISA, with telitacicept and IgG1 isotype as controls.
FIG. 15 shows schematic diagrams of the structures of a fusion protein of anifrolumab, TACI-19-16, and BCMA-ECD-1 (B637301) and fusion proteins of anifrolumab, TACI-19-16, and BCMA-ECD-2 (B637302, B637302-LALA, B746201, and B746201-LALA).
FIG. 16 shows the inhibitory effects of fusion proteins of anifrolumab, TACI-19-16, and BCMA (B637302 and B746201) and fusion proteins of anifrolumab and TACI-19-16 (B606401 and B805201) on the activity of IFN-β, with anifrolumab and IgG1 isotype as controls.
FIG. 17 shows the capabilities of B637302, B746201, B606401, and B805201 to bind to BAFF as measured by ELISA, with telitacicept and IgG1 isotype as controls.
FIG. 18 shows the capabilities of B637302, B746201, B606401, and B805201 to bind to APRIL as measured by ELISA, with telitacicept and IgG1 isotype as controls.
FIG. 19 shows the results of a functional experiment of pDCs among PBMCs. Human PBMCs cultured *in vitro* were stimulated with CpG-A and treated with gradient concentrations of B637302 and B606401, with anifrolumab and IgG1 isotype as controls. After 24 h, the level of secreted IFNα in the supernatant was measured.
FIG. 20 shows the results of an *in vitro* plasma cell differentiation experiment. B cells sorted from human PBMCs were cultured *in vitro* and treated with CpG-B and IFN-α and gradient concentrations of B637302, B746201, B606401, and B805201, with anifrolumab and IgG1 isotype as controls. After 4 days, the plasma cell (CD27+ CD38+) differentiation proportion was determined by flow cytometry.
FIG. 21 shows the results of a BAFF-induced *in vitro* B cell proliferation experiment. B cells sorted from human PBMCs were labeled with CTV and then cultured *in vitro.* IL-4, anti-IgM, CD40L, and IL-17 were added as basal stimulation signals. On this basis, BAFF was added to induce B cell proliferation, and the cells were treated with gradient concentrations of B637302, B606401, and B805201, with telitacicept and IgG1 isotype as controls. On day 5, B cell proliferation signals were detected by flow cytometry, with drug concentrations of 100 nM, 300 nM, and 1000 nM.
FIG. 22 shows an APRIL-induced *in vitro* B cell proliferation experiment. B cells sorted from human PBMCs were labeled with CTV and then cultured *in vitro.* IL-4, anti-IgM, CD40L, and IL-17 were added as basal stimulation signals. On this basis, APRIL was added to induce B cell proliferation, and the cells were treated with gradient concentrations of B637302 and B606401, with telitacicept and IgG1 isotype as controls. On day 5, B cell proliferation signals were detected by flow cytometry, with drug concentrations of 100 nM, 300 nM, and 1000 nM.

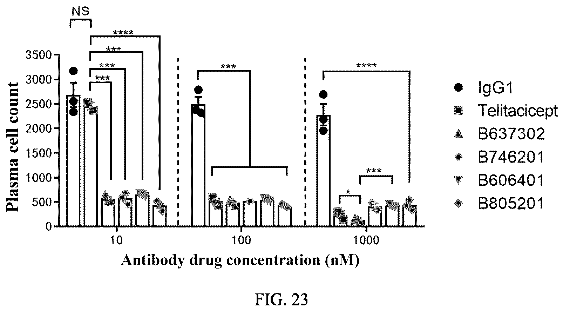

FIG. 23 shows a BAFF + APRIL-induced *in vitro* plasma cell production experiment. B cells sorted from human PBMCs were cultured *in vitro.* On days 1 to 4, CpG-B was added for stimulation. On days 4 to 10, CpG-B was removed, IL-6 + IL-10 + IL-21 was added, and the culture was continued. On this basis, 500 ng/mL BAFF and 50 ng/mL APRIL were added on days 4 to 7, and 50 ng/mL BAFF and 500 ng/mL APRIL were added on days 7 to 10 to induce *in vitro* production of plasma cells. The cells were treated with different concentrations (10, 100, and 1000 nM) of drugs B637302, B606401, B606401, and B805201 on days 4 to 10, with telitacicept and IgG1 isotype as controls. On day 10, plasma cells were counted by flow cytometry.

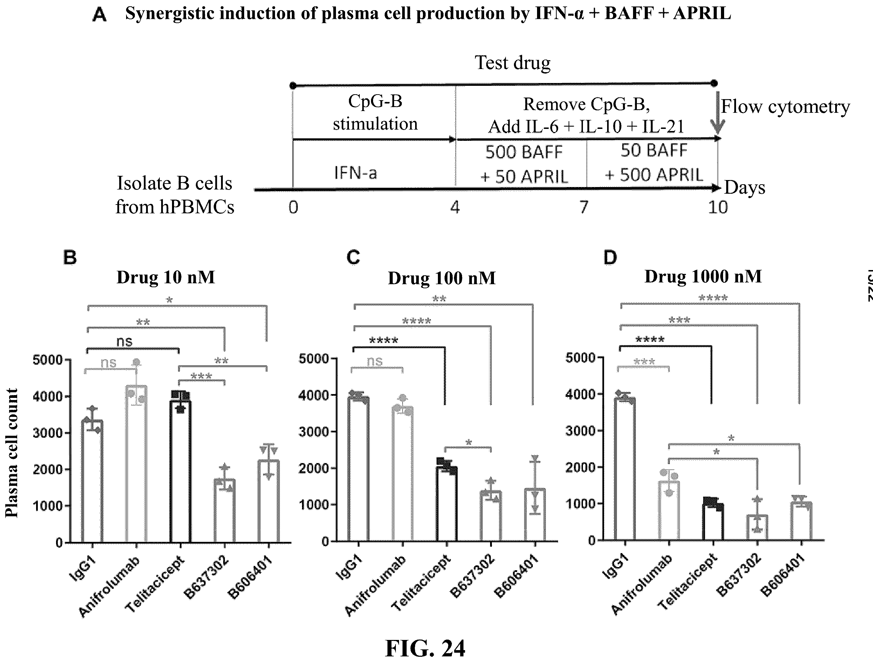

FIG. 24 shows an IFN-α, BAFF, and APRIL three-factor-induced *in vitro* plasma cell production experiment. A of FIG. 24 shows a schematic diagram of the process of the experiment: B cells sorted from human PBMCs were cultured *in vitro.* On days 1 to 4, CpG-B and IFN-α were added for stimulation. On days 4 to 10, CpG-B was removed, IL-6 + IL-10 + IL-21 was added, and the culture was continued. On this basis, 500 ng/mL BAFF and 50 ng/mL APRIL were added on days 4 to 7, and 50 ng/mL BAFF and 500 ng/mL APRIL were added on days 7 to 10 to induce *in vitro* production of plasma cells. The cells were treated with different concentrations (10, 100, and 1000 nM) of drugs B637302 and B606401 on days 0 to 10, with telitacicept and IgG1 isotype as controls. On day 10, plasma cells were counted by flow cytometry. B, C, and D of FIG. 24 shows assay results for 10 nM, 100 nM, and 1000 nM drug treatments, respectively.

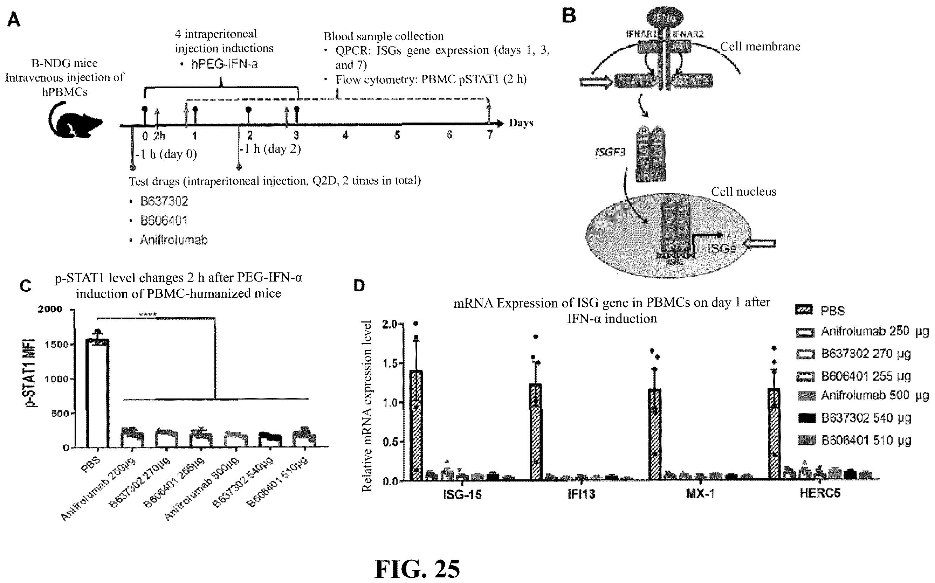

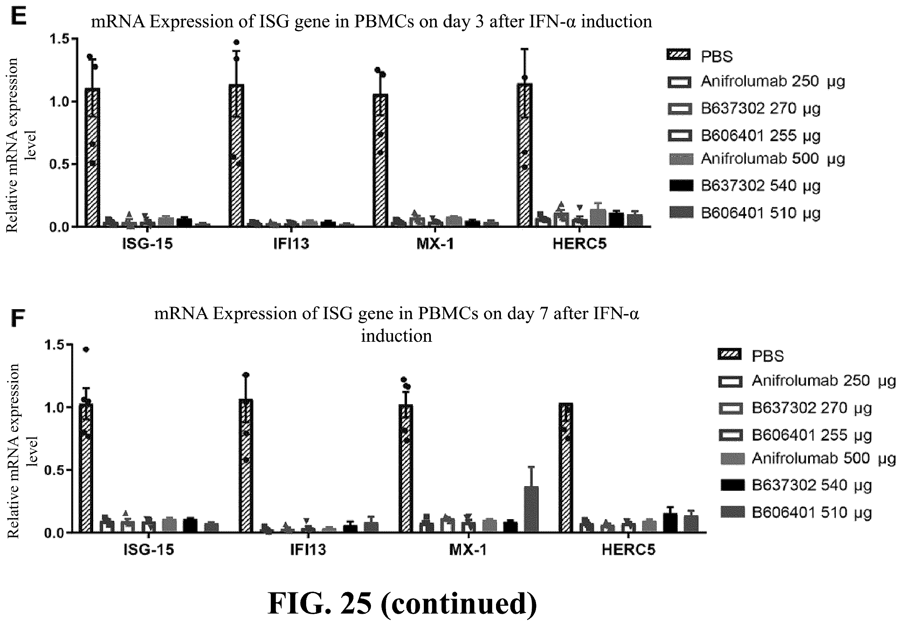

FIG. 25 shows the results of a PEG-IFN-α-induced PBMC-humanized mouse PD experiment. A of FIG. 25 shows a schematic diagram of the process of the experiment: B-NDG immunodeficient mice were humanized by injection of PBMCs into the tail vein and induced by intraperitoneal injection of PEG-IFN-α. In this model, drugs B637302 and B606401, at different concentrations, were intraperitoneally injected to treat the mice, with anifrolumab and PBS as controls. B of FIG. 25 shows a schematic diagram of IFN-α inducing a downstream signaling pathway. C of FIG. 25 shows pSTAT1 levels in PBMCs collected at 2 h. D, E, and F of FIG. 25 show the mRNA expression levels of a gene downstream of IFN-α in PBMCs collected at time points days 1, 3, and 7, respectively, as measured by QPCR.
FIG. 26 shows the results of a BAFF + APRIL-induced mouse PD experiment. A of FIG. 26 shows a schematic diagram of the process of the experiment: C57/B6 mice were induced by intraperitoneal injection of BAFF + APRIL. In this model, drugs B637302 and B606401, at different concentrations, were intraperitoneally injected to treat the mice, with telitacicept and PBS as controls. Peripheral blood was collected on days 4 and 7, and the IgA levels in plasma were measured by ELISA. B and C of FIG. 26 show the IgA levels in the plasma of the blood collected on days 4 and 7, respectively.

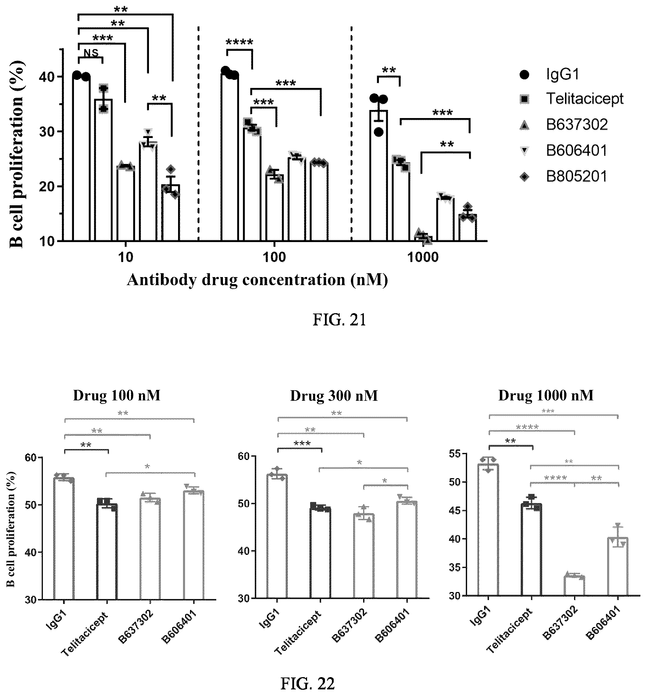

In FIGs. 21-26, the statistical method Student's t-test was used, * p < 0.05, ** p < 0.01, *** p < 0.001, and **** p < 0.0001.

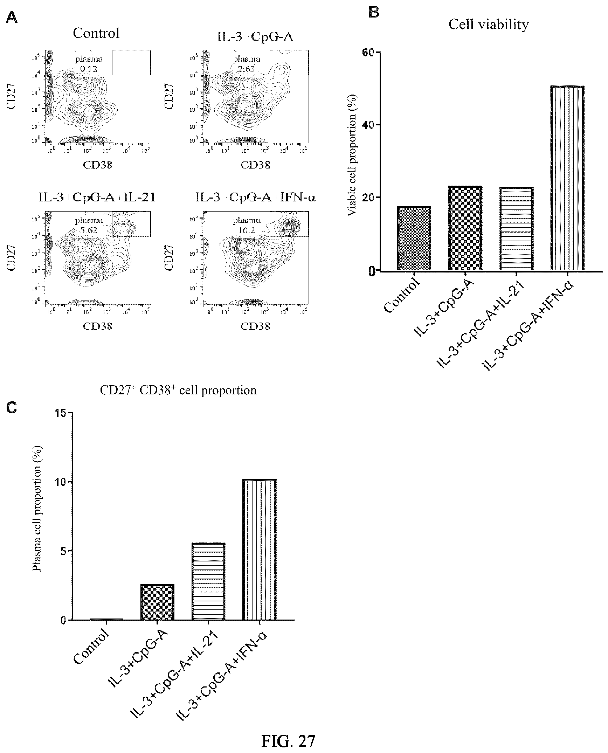

FIG. 27 shows the results of an *in vitro* B cell differentiation experiment. B cells sorted from human PBMCs were cultured *in vitro,* and IL-3, CpG-A, IFNα, and IL-21 were added to stimulate B cell differentiation. After 6 days, the cell viability and the proportion of differentiated plasma cells (CD27+ CD38+) were determined. A of FIG. 27 shows FACS assay results for IL-3 + CpG-A, IL-3 + CpG-A + IL-21, and IL-3 + CpG-A + IFNα, with the 1640 culture medium as a control. B of FIG. 27 shows corresponding viable cell proportions. C of FIG. 27 shows corresponding plasma cell proportions.

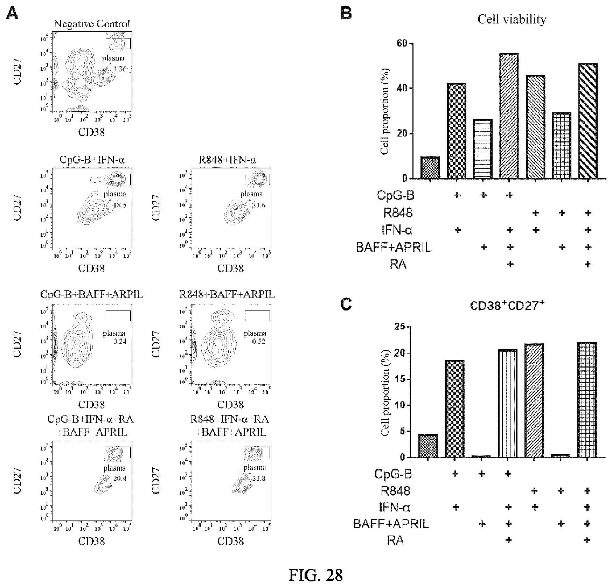

FIG. 28 shows the results of an *in vitro* B cell differentiation experiment. B cells sorted from human PBMCs were cultured *in vitro,* and R848, CpG-B, IFNα, BAFF, APRIL, and RA were added to stimulate B cell differentiation. After 6 days, the cell viability and the proportion of differentiated plasma cells (CD27+ CD38+) were determined. A of FIG. 28 shows FACS assay results for CpG-B + IFNα, R484 + IFNα, CpG-B + BAFF + APRIL, R484 + BAFF + APRIL, CpG-B + IFNα + RA + BAFF + APRIL, and R484 + IFNα + RA + BAFF + APRIL, with the 1640 culture medium as a control. B of FIG. 28 shows corresponding viable cell proportions. C of FIG. 28 shows corresponding plasma cell (CD27⁺ CD38⁺) proportions.

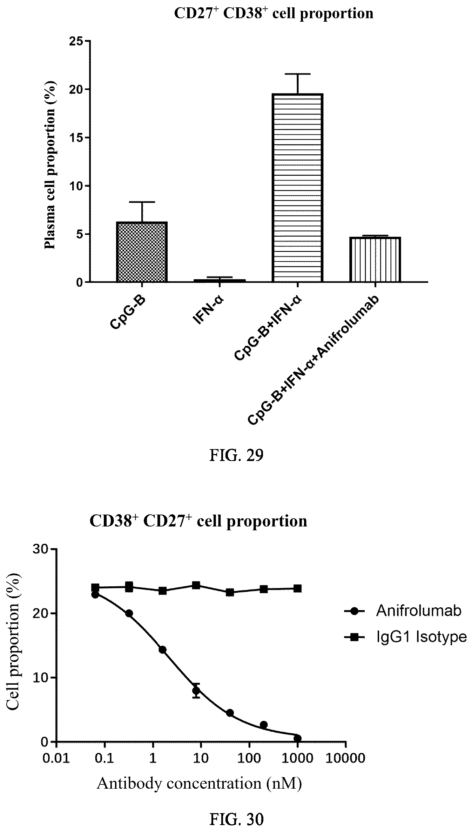

FIG. 29 shows the results of an *in vitro* B cell differentiation experiment. B cells sorted from human PBMCs were cultured *in vitro.* B cell differentiation was stimulated with CpG-B and IFNα, and the cells were treated with anifrolumab as a control. After 4 days, the plasma cell (CD27⁺ CD38⁺) differentiation proportion was determined.
FIG. 30 shows the results of an *in vitro* B cell differentiation experiment. B cells sorted from human PBMCs were cultured *in vitro.* B cell differentiation was stimulated with CpG-B and IFNα, and the cells were treated with gradient concentrations of anifrolumab as a control. After 4 days, the plasma cell (CD27+ CD38+) differentiation proportion was determined. The results show that anifrolumab stimulated plasma cell differentiation in a concentration-dependent manner under this condition. IgG1 isotype was used as a control.

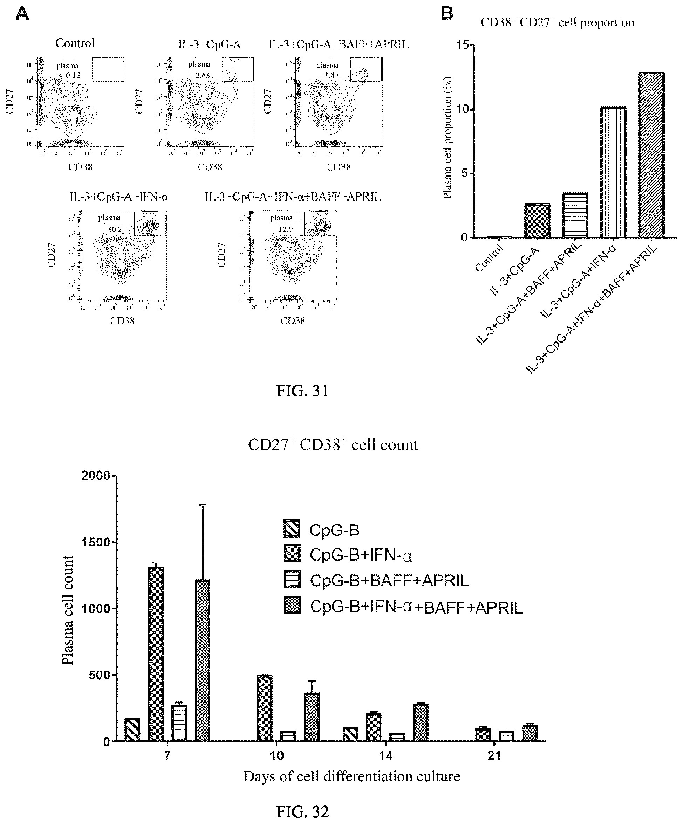

FIG. 31 shows the results of a plasma cell production experiment. B cells sorted from human PBMCs were cultured *in vitro,* and plasma cell production was induced with IL-3, CpG-A, IFNα, BAFF, and APRIL. After 6 days, the plasma cell (CD27⁺ CD38⁺) differentiation proportion was determined. A of FIG. 31 shows FACS assay results for IL-3 + CpG-A, IL-3 + CpG-A + BAFF + APRIL, IL-3 + CpG-A + IFNα, and IL-3 + CpG-A + IFNα + BAFF + APRIL, with the 1640 culture medium as a control. B of FIG. 31 shows corresponding plasma cell (CD27⁺ CD38⁺) proportions.
FIG. 32 shows the results of a plasma cell production experiment. B cells sorted from human PBMCs were cultured *in vitro,* and plasma cell production was induced with CpG-B, IFNα, BAFF, and APRIL. After 7, 10, 14, and 21 days, differentiated plasma cells (CD27⁺ CD38⁺) were counted.

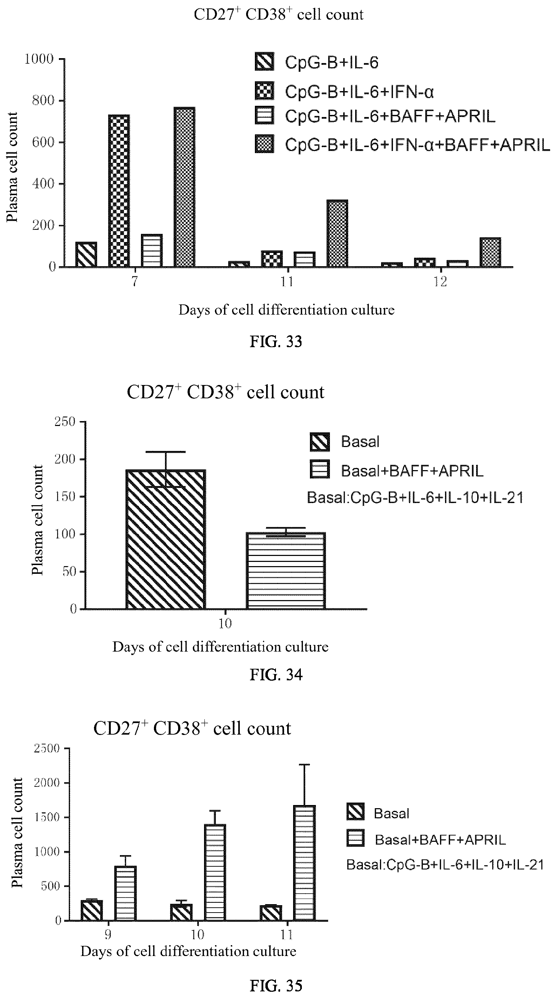

FIG. 33 shows the results of a plasma cell production experiment. B cells sorted from human PBMCs were cultured *in vitro* and stimulated with CpG-B and IFNα on days 0 to 4. After day 4, CpG-B and IFNα were removed, and IL-6, BAFF, and APRIL were added to induce plasma cell production. After 7, 11, and 12 days, differentiated plasma cells (CD27⁺ CD38⁺) were counted.
FIG. 34 shows the results of a plasma cell production experiment. B cells sorted from human PBMCs were cultured *in vitro* and stimulated with CpG-B on days 0 to 4. After day 4, CpG-B was removed, and IL-6, IL-10, IL-21 (5 ng/mL), BAFF, and APRIL were added to induce plasma cell production. On days 4 to 7, 500 ng/mL BAFF and 50 ng/mL APRIL were added. On days 7 to 10, 50 ng/mL BAFF and 500 ng/mL APRIL were added. After 10 days, differentiated plasma cells (CD27⁺ CD38⁺) were counted.
FIG. 35 shows the results of a plasma cell production experiment. B cells sorted from human PBMCs were cultured *in vitro* and stimulated with CpG-B on days 0 to 4. After day 4, CpG-B was removed, and IL-6, IL-10, IL-21 (50 ng/mL), BAFF, and APRIL were added to induce plasma cell production. On days 4 to 7, 500 ng/mL BAFF and 50 ng/mL APRIL were added. On days 7 to 10, 50 ng/mL BAFF and 500 ng/mL APRIL were added. After 9, 10, and 11 days, differentiated plasma cells (CD27⁺ CD38⁺) were counted.

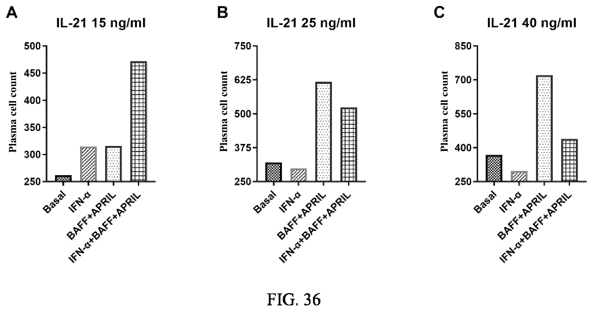

FIG. 36 shows the results of a plasma cell production experiment. B cells sorted from human PBMCs were cultured *in vitro* and stimulated with CpG-B on days 0 to 4. After day 4, CpG-B was removed, and IL-6, IL-10, IL-21 (15, 25, or 40 ng/mL), BAFF, and APRIL were added to induce plasma cell production. On days 5 to 7, 500 ng/mL BAFF and 50 ng/mL APRIL were added. On days 7 to 10, 50 ng/mL BAFF and 500 ng/mL APRIL were added. On day 4, the cells were treated with gradient concentrations of telitacicep as a control. After 10 days, differentiated plasma cells (CD27⁺ CD38⁺) were counted. A, B, and C of FIG. 36 shows plasma cell (CD27⁺ CD38⁺) counts obtained at IL-21 concentrations of 15 ng/mL, 25 ng/mL, and 40 ng/mL, respectively.

### DETAILED DESCRIPTION

The present disclosure is further described with reference to the following examples, but these examples are not intended to limit the scope of the present disclosure.

Experimental procedures without specific conditions indicated in the examples or test examples of the present disclosure are generally conducted under conventional conditions, or under conditions recommended by the manufacturers of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY. Reagents without specific origins indicated are commercially available conventional reagents.

### Example 1. TACI Sequence Analysis and Engineering

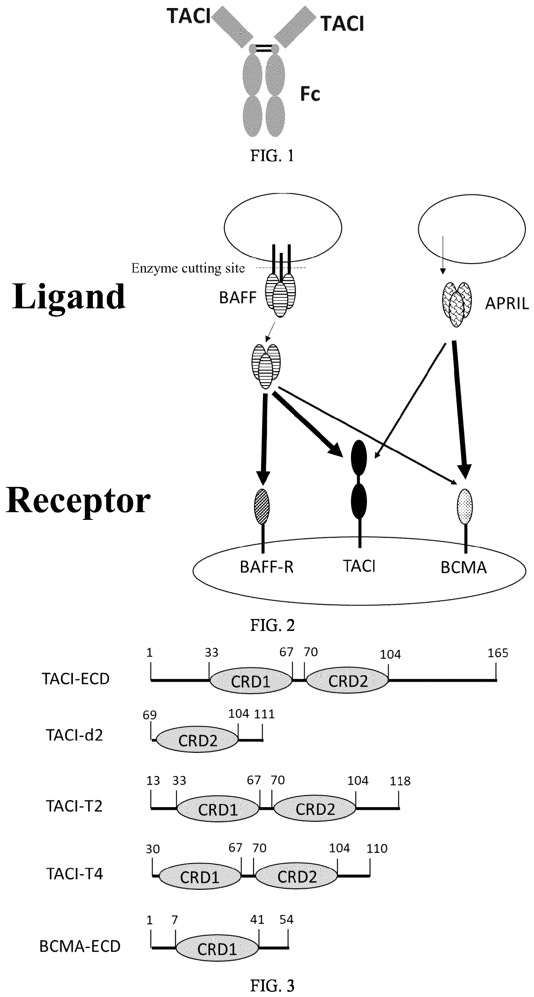

Due to the presence of multiple protease cutting sites in the sequence of TACI, full-length TACI is susceptible to cleavage after expression. In this experiment, TACI sequence fragments with different lengths were designed, and the C-terminus of TACI-ECD or its fragments was directly fused with the N-terminus of human IgG1's Fc (SEQ ID NO: 3) to construct TACI-Fc fusion proteins (including full-length TACI-ECD-Fc and TACI-9-Fc); 293E (EBNA1 gene-modified human embryonic kidney cells) was transfected for expression, and the products were purified to give TACI-Fc fusion proteins containing two identical polypeptide chains (their structure is shown in FIG. 1). RCT-18 (telitacicept) was used as a positive control.

Their sequences are shown below:
> TACI-ECD (the amino acid residues at positions 1 to 165 of wild-type human TACI) 
MSGLGRSRRGGRSRVDQEERFPQGLWTGVAMRSCPEEQYWDPLLGTCMSCKTI
CNHQSQRTCAAFCRSLSCRKEQGKFYDHLLRDCISCASICGQHPKQCAYFCENK
LRSPVNLPPELRRQRSGEVENNSDNSGRYQGLEHRGSEASPALPGLKLSADQVA
LVYS
SEQ ID NO: 1
> TACI-9 (positions 68 to 108 of human TACI-ECD, numbered in natural order)
   SLSCRKEQGKFYDHLLRDCISCASICGQHPKQCAYFCENKL
   SEQ ID NO: 2
> The Fc sequence of human IgG1 
DKTHTCPPCPAPEAEGAPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPSSIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNH
YTQKSLSLSPGK
SEQ ID NO: 3
> Telitacicept (RCT-18) 
SRVDQEERFPQGLWTGVAMRSCPEEQYWDPLLGTCMSCKTICNHQSQRTCAAF
CRSLSCRKEQGKFYDHLLRDCISCASICGQHPKQCAYFCENKLRSPVNLPPELDK
THTCPPCPAPEAEGAPSVFLFPPKPKDTLMISRTPEVTCVVVDVSHEDPEVKFNW
YVDGVEVHNAKTKPREEQYNSTYRVVSVLTVLHQDWLNGKEYKCKVSNKALP
SSIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQ
KSLSLSPGK
SEQ ID NO: 4
> Atacicept

AMRSCPEEQYWDPLLGTCMSCKTICNHQSQRTCAAFCRSLSCRKEQGKFYDHL
LRDCISCASICGQHPKQCAYFCENKLRSEPKSSDKTHTCPPCPAPEAEGAPSVFLF

PPKPKDTLMISRTPEVTCVVVDVSHEDPEVKFNWYVDGVEVHNAKTKPREEQY
NSTYRVVSVLTVLHQDWLNGKEYKCKVSNKALPSSIEKTISKAKGQPREPQVYT
LPPSRDELTKNQVSLTCLVKGFYPSDIAVEWESNGQPENNYKTTPPVLDSDGSFF
LYSKLTVDKSRWQQGNVFSCSVMHEALHNHYTQKSLSLSPGK
SEQ ID NO: 5

Note: In the sequence of telitacicept (RCT-18), the non-underlined part is a TACI sequence (positions 13 to 118 of the human TACI extracellular region, numbered in natural order), and the underlined part is an Fc sequence. In the sequence of atacicept, the non-underlined part is a TACI sequence (positions 30 to 110 of the human TACI extracellular region, numbered in natural order) and the linker EPKSS (indicated in italics), and the underlined part is an Fc sequence.

The obtained TACI-Fc fusion proteins were analyzed by mass spectrometry, and the results show that TACI-9-Fc had no cleavage fragment, but TACI cleavage fragments were detected for both the positive control RCT-18 and full-length TACI-ECD-Fc (results not shown).

The above TACI-Fc fusion proteins' function of blocking the binding of BAFF to BAFF-R was further assessed as follows: The receptor protein was diluted with a pH 7.4 PBS (BasalMedia, B320) buffer to 2 µg/mL, and the resulting dilution was added to a 96-well microplate (Corning, 3590) at 100 µL/well. The plate was incubated overnight at 4 °C. After the liquid was discarded, 1% Casein blocking solution (Thermo, 37528) was added at 200 µL/well for blocking, and the plate was incubated at 37 °C for 2 h. After the blocking, the blocking solution was discarded, and the plate was washed 3 times with a PBST buffer (pH 7.4 PBS containing 0.1% tween-20) and set aside for later use. A biotinylated ligand protein at a fixed concentration was mixed with a serially diluted antibody or fusion protein, and the mixture was pre-incubated at 37 °C for 30 min and then added to the blocked microplate. The plate was incubated at 37 °C for 1.5 h. After the incubation, the plate was washed 3 times with PBST, and streptavidin-HRP (Invitrogen, 434323, diluted in a 1:4000 ratio) was added at 100 µL/well. The plate was incubated at 37 °C for 1 h. The supernatant was removed. After the plate was washed 3 times with PBST, the chromogenic substrate TMB (KPL, 5120-0077) was added at 100 µL/well. The plate was incubated at room temperature for 10-15 min, and 1 M H₂SO₄ was added at 50 µL/well to stop the reaction. The absorbance at 450 nm was measured using a microplate reader. Curves for the inhibition of the binding of the ligand to the receptor were fitted using software, and IC₅₀ values were calculated. The sources of the ligand and the receptor protein used are as follows: BAFF (Sino biological, 10056-HNCH) and BAFF-R (Sino biological, 16079-H02H).

The results show that TACI-9-Fc and RCT-18 blocked the binding of BAFF to BAFF-R with IC₅₀ values of 5.02 nM and 27.48 nM, respectively. The functional activity of TACI-9-Fc was significantly stronger than that of the control RCT-18. Therefore, TACI-9 is considered a potential candidate molecule.

### Example 2. Analysis of Truncated Fragments of TACI

The sequence of TACI was further truncated on the basis of TACI-9, and the functional activity of the truncated TACI fragments was assessed. The sequences of the further truncated TACI fragments are shown below:
> TACI-10 (obtained by C-terminally truncating TACI-9 by 1 amino acid, "L") (positions 68 to 107 of human TACI-ECD, numbered in natural order)
   SLSCRKEQGKFYDHLLRDCISCASICGQHPKQCAYFCENK (SEQ ID NO: 6)
> TACI-11 (obtained by C-terminally truncating TACI-9 by 2 amino acids, "KL") (positions 68 to 106 of human TACI-ECD, numbered in natural order)
   SLSCRKEQGKFYDHLLRDCISCASICGQHPKQCAYFCEN (SEQ ID NO: 7)
> TACI-12 (obtained by C-terminally truncating TACI-9 by 3 amino acids, "NKL") (positions 68 to 105 of human TACI-ECD, numbered in natural order)
   SLSCRKEQGKFYDHLLRDCISCASICGQHPKQCAYFCE (SEQ ID NO: 8)

The C-terminus of the above truncated TACI fragments was directly fused with the N-terminus of human IgG's Fc (SEQ ID NO: 3) to construct TACI-Fc fusion proteins. 293E was transfected for expression, and the products were purified to give TACI-Fc fusion proteins containing two identical polypeptides (their structure is shown in FIG. 1).

The above constructed TACI-Fc fusion proteins were analyzed by mass spectrometry, and the experimental results are shown in Table 2. The experimental results show that the TACI-Fc fusion proteins constructed using the TACI-9 truncated fragments did not undergo TACI polypeptide cleavage.

In addition, the BAFF binding activity of the constructed TACI-Fc fusion proteins was assessed, and the TACI-Fc fusion proteins' functional activity of blocking the binding of BAFF to BAFF-R was assessed (see Example 1 for the experimental method).

The BAFF binding activity of the TACI-Fc fusion proteins was assessed as follows: BAFF (Sino biological, 10056-HNCH) protein was diluted with a pH 7.4 PBS (BasalMedia, B320) buffer to 1 µg/mL, and the resulting dilution was added to a 96-well microplate (Corning, 3590) at 100 µL/well. The plate was incubated overnight at 4 °C. After the liquid was discarded, 5% skim milk (BD, 232100), diluted with PBS, was added at 300 µL/well for blocking, and the plate was incubated at 37 °C for 2 h. After the blocking, the blocking solution was discarded, and the plate was washed 3 times with a PBST buffer (pH 7.4 PBS containing 0.1% tween-20). Serially diluted test antibody or fusion protein solutions were added at 100 µL/well, and the plate was incubated at 37 °C for 1 h. After the incubation, the plate was washed 3 times with PBST, and mouse anti-human IgG (H+L) (Jackson ImmunoResearch, 209-035-088, diluted in a 1:8000 ratio) was added at 100 µL/well. The plate was incubated at 37 °C for 1 h. After the plate was washed 3 times with PBST, the chromogenic substrate TMB (KPL, 5120-0077) was added at 100 µL/well. The plate was incubated at room temperature for 10-15 min, and 1 M H₂SO₄ was added at 50 µL/well to stop the reaction. The absorbance at 450 nm was measured using a microplate reader. Curves for the binding of the antibodies to the antigen were fitted using software, and EC₅₀ values were calculated.

The results in Table 2 show that the BAFF binding activity of fusion proteins TACI-10-Fc, TACI-11-Fc, and TACI-12-Fc, constructed using TACI-9's truncated fragments TACI-10, TACI-11, and TACI-12, and their functional activity of blocking the binding of BAFF to BAFF-R are at the same level as those of fusion protein TACI-9-Fc.

**Table 2. The functional activity of TACI-Fc fusion proteins and the results of the mass spectrometry detection of cleavage**

| Fusion Protein | TACI cleavage analysis | EC₅₀ (nM) for binding of TACI-Fc to BAFF | IC₅₀ (nM) for TACI-Fc blocking binding of BAFF to BAFF-R |
|---|---|---|---|
| TACI-9-Fc | No cleavage | 0.4903 | 2.09 |
| TACI-10-Fc | No cleavage | 0.5606 | 2.7 |
| TACI-11-Fc | No cleavage | 0.5841 | 2.621 |
| TACI-12-Fc | No cleavage | 0.8944 | 3.862 |

### Example 3. Sequence Engineering of Truncated Fragments of TACI

TACI protein is prone to aggregation in neutral solutions. To further improve the stability of TACI, Molecular Operating Environment (MOE) software was used to analyze the hydrophobic and ionic groups in TACI fragments based on the crystal structure of TACI (PDB ID: 1XU1), and several amino acid residues in TACI-9 were subjected to amino acid replacement to reduce the area of exposed hydrophobic and ionic groups of TACI protein, thereby reducing TACI aggregation and improving the stability of TACI while substantially maintaining the functional activity of TACI. The amino acid sequences of the engineered TACI fragments are shown below:
> TACI-9-1 (TACI-9 with replacement L69T) S**T**SCRKEQGKFYDHLLRDCISCASICGQHPKQCAYFCENKL (SEQ ID NO: 9)
> TACI-9-2 (TACI-9 with mutation R72S) SLSC**S**KEQGKFYDHLLRDCISCASICGQHPKQCAYFCENKL (SEQ ID NO: 10)
> TACI-9-3 (TACI-9 with mutation K73E) SLSCR**E**EQGKFYDHLLRDCISCASICGQHPKQCAYFCENKL (SEQ ID NO: 11)
> TACI-9-4 (TACI-9 with mutation K73Q) SLSCR**Q**EQGKFYDHLLRDCISCASICGQHPKQCAYFCENKL (SEQ ID NO: 12)
> TACI-9-5 (TACI-9 with mutation K77E) SLSCRKEQG**E**FYDHLLRDCISCASICGQHPKQCAYFCENKL (SEQ ID NO: 13)
> TACI-9-6 (TACI-9 with mutations L69R and D85T) S**R**SCRKEQGKFYDHLLR**T**CISCASICGQHPKQCAYFCENKL (SEQ ID NO: 14)
> TACI-9-7 (TACI-9 with mutations L69R and D85A) S**R**SCRKEQGKFYDHLLRACISC**A**SICGQHPKQCAYFCENKL (SEQ ID NO: 15)
> TACI-9-8 (TACI-9 with mutation Y102A) SLSCRKEQGKFYDHLLRDCISCASICGQHPKQCA**A**FCENKL (SEQ ID NO: 16)
> TACI-9-9 (TACI-9 with mutations L69R, D85T, and Y102R) S**R**SCRKEQGKFYDHLLR**T**CISCASICGQHPKQCA**R**FCENKL (SEQ ID NO: 17)
> TACI-9-10 (TACI-9 with mutations K73E and K77E) SLSCR**E**EQG**E**FYDHLLRDCISCASICGQHPKQCAYFCENKL (SEQ ID NO: 18)
> TACI-9-11 (TACI-9 with mutations R72S, K73E, and K77E) SLSC**SE**EQG**E**FYDHLLRDCISCASICGQHPKQCAYFCENKL (SEQ ID NO: 19)
> TACI-9-12 (TACI-9 with mutations L69T and Y102A) S**T**SCRKEQGKFYDHLLRDCISCASICGQHPKQCA**A**FCENKL (SEQ ID NO: 20)
> TACI-9-13 (TACI-9 with mutations L69T and F103Y) S**T**SCRKEQGKFYDHLLRDCISCASICGQHPKQCAY**Y**CENKL (SEQ ID NO: 21)
> TACI-9-14 (TACI-9 with mutations L69T, Y102A, and F103Y) S**T**SCRKEQGKFYDHLLRDCISCASICGQHPKQCA**AY**CENKL (SEQ ID NO: 22)
> TACI-9-15 (TACI-9 with mutations L69T, K73E, K77E, and Y102A) S**T**SCR**E**EQG**E**FYDHLLRDCISCASICGQHPKQCA**A**FCENKL (SEQ ID NO: 23)

Note: In the above sequences, the underlined parts are amino acid residues after mutation.

The C-terminus of the above TACI fragments obtained by engineering TACI-9 was fused to the N-terminus of human IgG's Fc (SEQ ID NO: 3) to construct TACI-Fc fusion proteins (their structure is shown in FIG. 1). 293E was transfected for expression, and the products were then purified by Protein A affinity chromatography to TACI-Fc fusion proteins containing two identical polypeptide chains.

The BAFF binding activity of the constructed TACI-Fc fusion proteins was assessed (see Example 2 for the experimental method), and the results are shown in Table 3:

**Table 3. The BAFF binding activity of TACI-Fc fusion proteins**

| TACI-Fc fusion protein | TACI sequence mutation site(s) | EC₅₀ (nM) for binding of TACI-Fc fusion proteins to BAFF |
|---|---|---|
| TACI-9-Fc | None | 0.4181 |
| TACI-9-1-Fc | L69T | 0.3820 |
| TACI-9-2-Fc | R72S | 0.2785 |
| TACI-9-3-Fc | K73E | 0.2654 |
| TACI-9-4-Fc | K73Q | 0.4695 |
| TACI-9-5-Fc | K77E | 0.1456 |
| TACI-9-6-Fc | L69R, D85T | 0.3672 |
| TACI-9-7-Fc | L69R, D85A | 0.7273 |
| TACI-9-8-Fc | Y102A | 0.1999 |
| TACI-9-9-Fc | L69R, D85T, Y102R | 0.9368 |
| TACI-9-10-Fc | K73E, K77E | 0.1199 |
| TACI-9-11-Fc | R72S, K73E, K77E | 0.1950 |
| TACI-9-12-Fc | L69T, Y102A | 0.1223 |
| TACI-9-13-Fc | L69T, F103Y | 0.4327 |
| TACI-9-14-Fc | L69T, Y102A, F103Y | 0.2233 |
| TACI-9-15-Fc | L69T, K73E, K77E, Y102A | 0.0869 |

Note: The mutation sites in the table are amino acid residue sites (numbered in natural order) relative to TACI-ECD (SEQ ID NO: 1). For example, "L69T" indicates that the amino acid residue at position 69 (numbered in natural order) of the sequence SEQ ID NO: 1 is mutated from L to T.

In addition, some of the TACI-Fc fusion proteins obtained by purification were exchanged into a PBS solution using an ultrafiltration tube, and the presence of precipitates in the TACI-Fc fusion protein solutions was determined by the observation of the appearances of the solutions. The experimental results show that there was no precipitate in the solutions of the TACI-Fc fusion proteins constructed by the engineered TACI fragments, while there was a precipitate generated in the solution of RCT-18 in PBS (results not shown).

Truncated forms of TACI-9-15 (TACI-9-15a, TACI-9-15b, and TACI-9-15c) are further provided, and their amino acid sequences are shown below:
> TACI-9-15a
   STSCREEQGEFYDHLLRDCISCASICGQHPKQCAAFCENK (SEQ ID NO: 24)
> TACI-9-15b
   STSCREEQGEFYDHLLRDCISCASICGQHPKQCAAFCEN (SEQ ID NO: 25)
> TACI-9-15c (i.e., TACI-19-16)
   STSCREEQGEFYDHLLRDCISCASICGQHPKQCAAFCE (SEQ ID NO: 26).

### Example 4. Construction, Expression, and Purification of TACI-BCMA Fusion Proteins

As previously mentioned, both atacicept and telitacicept are BAFF/APRIL antagonists in the molecular format of TACI-Fc. As shown in FIG. 2, both TACI and BCMA can bind to BAFF and APRIL. TACI has a relatively strong capability to bind to BAFF and a relatively weak capability to bind to APRIL. BCMA has a relatively strong capability to bind to APRIL. BCMA and TACI were designed as fusion proteins, in hope of improving the neutralizing activity against APRIL.

As shown in FIG. 3, TACI-ECD is defined as a TACI extracellular region containing amino acid residues 1-165; TACI-d2 is a CRD2 ligand-binding domain containing amino acid residues 69-111; TACI-T2 is a CRD1 and CRD2 ligand-binding domain containing amino acid residues 13-118; TACI-T4 is a CRD1 and CRD2 ligand-binding domain containing amino acid residues 30-110; BCMA-ECD is a BCMA extracellular region containing amino acid residues 1-54.

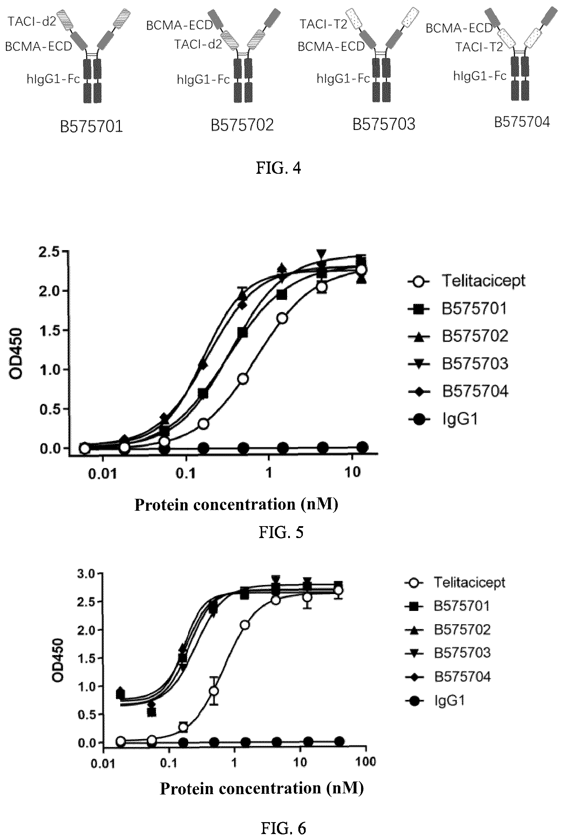

As shown in FIG. 4, 4 formats of fusion proteins containing a TACI polypeptide and a BCMA polypeptide were designed. Fusion proteins B575701 and B575702 were obtained by linking TACI-d2 and the BCMA-ECD sequence and fusing with human wild-type IgG1-Fc. Fusion proteins B575703 and B575704 were obtained by linking TACI-T2 and the BCMA-ECD sequence and fusing with human wild-type IgG1-Fc. The sequence of TACI-ECD is set forth in SEQ ID NO: 1. The remaining sequences are shown below, with human IgG1 Fc underlined. The G at the last position of SEQ ID NO: 27 may also be absent.
> TACI-d2 (SEQ ID NO: 27)
   LSCRKEQGKFYDHLLRDCISCASICGQHPKQCAYFCENKLRSPG
> TACI-T2 (SEQ ID NO: 28) 
SRVDQEERFPQGLWTGVAMRSCPEEQYWDPLLGTCMSCKTICNHQSQRTCAAF
CRSLSCRKEQGKFYDHLLRDCISCASICGQHPKQCAYFCENKLRSPVNLPPEL
> TACI-T4 (SEQ ID NO: 29) 
AMRSCPEEQYWDPLLGTCMSCKTICNHQSQRTCAAFCRSLSCRKEQGKFYDHL
LRDCISCASICGQHPKQCAYFCENKLRS
> BCMA-ECD (SEQ ID NO: 30) 
MLQMAGQCSQNEYFDSLLHACIPCQLRCSSNTPPLTCQRYCNASVTNSVKGTN
A
> TACI-d2-BCMA-ECD (SEQ ID NO: 31) 
LSCRKEQGKFYDHLLRDCISCASICGQHPKQCAYFCENKLRSPGMLQMAGQCS
QNEYFDSLLHACIPCQLRCSSNTPPLTCQRYCNASVTNSVKGTNA
> BCMA-ECD-TACI-d2 (SEQ ID NO: 32) 
MLQMAGQCSQNEYFDSLLHACIPCQLRCSSNTPPLTCQRYCNASVTNSVKGTN
ALSCRKEQGKFYDHLLRDCISCASICGQHPKQCAYFCENKLRSPG
> TACI-T2-BCMA-ECD (SEQ ID NO: 33) 
SRVDQEERFPQGLWTGVAMRSCPEEQYWDPLLGTCMSCKTICNHQSQRTCAAF
CRSLSCRKEQGKFYDHLLRDCISCASICGQHPKQCAYFCENKLRSPVNLPPELM
LQMAGQCSQNEYFDSLLHACIPCQLRCSSNTPPLTCQRYCNASVTNSVKGTNA
> BCMA-ECD-TACI-T2 (SEQ ID NO: 34) 
MLQMAGQCSQNEYFDSLLHACIPCQLRCSSNTPPLTCQRYCNASVTNSVKGTN
ASRVDQEERFPQGLWTGVAMRSCPEEQYWDPLLGTCMSCKTICNHQSQRTCAA
FCRSLSCRKEQGKFYDHLLRDCISCASICGQHPKQCAYFCENKLRSPVNLPPEL.
 B575701, B575702, B575703, and B575704 were obtained by linking human IgG1's Fc (SEQ ID NO: 3) to the C-termini of TACI-d2-BCMA-ECD, BCMA-ECD-TACI-d2, TACI-T2-BCMA-ECD, and BCMA-ECD-TACI-T2, respectively. In the following sequences, the Fc is underlined.
> B575701 (SEQ ID NO: 35) 
LSCRKEQGKFYDHLLRDCISCASICGQHPKQCAYFCENKLRSPGMLQMAGQCS
QNEYFDSLLHACIPCQLRCSSNTPPLTCQRYCNASVTNSVKGTNADKTHTCPPCP
APEAEGAPSVFLFPPKPKDTLMISRTPEVTCVVVDVSHEDPEVKFNWYVDGVE
 
VHNAKTKPREEQYNSTYRVVSVLTVLHQDWLNGKEYKCKVSNKALPSSIEKTI
SKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSL
SPGK
> B575702 (SEQ ID NO: 36) 
MLQMAGQCSQNEYFDSLLHACIPCQLRCSSNTPPLTCQRYCNASVTNSVKGTN
ALSCRKEQGKFYDHLLRDCISCASICGQHPKQCAYFCENKLRSPGDKTHTCPPC
PAPEAEGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPSSIEKTI
SKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSL
SPGK
> B575703 (SEQ ID NO: 37) 
SRVDQEERFPQGLWTGVAMRSCPEEQYWDPLLGTCMSCKTICNHQSQRTCAAF
CRSLSCRKEQGKFYDHLLRDCISCASICGQHPKQCAYFCENKLRSPVNLPPELM
LQMAGQCSQNEYFDSLLHACIPCQLRCSSNTPPLTCQRYCNASVTNSVKGTNAD
KTHTCPPCPAPEAEGAPSVFLFPPKPKDTLMISRTPEVTCVVVDVSHEDPEVKFN
WYVDGVEVHNAKTKPREEQYNSTYRVVSVLTVLHQDWLNGKEYKCKVSNKA
LPSSIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHY
TQKSLSLSPGK
> B575704 (SEQ ID NO: 38)

MLQMAGQCSQNEYFDSLLHACIPCQLRCSSNTPPLTCQRYCNASVTNSVKGTN
ASRVDQEERFPQGLWTGVAMRSCPEEQYWDPLLGTCMSCKTICNHQSQRTCAA
FCRSLSCRKEQGKFYDHLLRDCISCASICGQHPKQCAYFCENKLRSPVNLPPELD
KTHTCPPCPAPEAEGAPSVFLFPPKPKDTLMISRTPEVTCVVVDVSHEDPEVKFN
WYVDGVEVHNAKTKPREEQYNSTYRVVSVLTVLHQDWLNGKEYKCKVSNKA
LPSSIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHY
TQKSLSLSPGK.

The coding gene sequences for the above proteins were synthesized and subcloned into pcDNA3.4. 50 µg of expression plasmids were diluted with a culture medium and well mixed, and 200 µL of a transfection reagent was diluted with a culture medium and well mixed. These two were combined and well mixed and then incubated at 37 °C for 15 min. The mixture was added dropwise to a HEK293 cell suspension. The cell suspension was incubated on a 37 °C shaker for one week and centrifuged at 8000 rpm for 5 min, and the supernatant was collected. A Protein A affinity chromatography column was equilibrated with 20 mL of 1× PBS at a flow rate of 1 mL/min. The protein supernatant was loaded at a flow rate of 1 mL/min. The non-specifically bound protein was rinsed with 20 mL of 1× PBS at a flow rate of 1 mL/min. Finally, elution was performed using a pH 3.4 citric acid buffer at a flow rate of 1 mL/min, and the eluted protein was transferred to a dialysis bag and dialyzed against 1× PBS to be exchanged into the PBS storage buffer. Analysis showed that the protein of interest was obtained.

### Example 5. In Vitro Binding of TACI-BCMA Fusion Proteins to BAFF and APRIL

The capabilities of TACI-BCMA fusion proteins (B575701, B575702, B575703, and B575704) to bind to BAFF and APRIL were assessed by ELISA as follows:
BAFF (PeproTech, Cat: 310-13) or ARPIL (Acro Biosystems, Cat: APL-H52D1) was diluted to 1 µg/mL with PBS, and a 96-well plate (Costar, Cat: 3590) was coated with the dilution overnight at 4 °C. The plate was washed with PBST and blocked with Blocking Buffer (PBST containing 1% BSA) at 37 °C for 1 h. The plate was tapped. The test antibodies were diluted with Blocking Buffer. Incubation was performed at 37 °C for 1.5 h. The plate was washed with PBST. An HRP-mouse anti-human Fc antibody (GenScript, Cat: A01854-200) was diluted with Blocking Buffer. Incubation was performed at 37 °C for 40 min. The plate was washed with PBST. 100 µL of TMB substrate solution (Biopanda, Cat: TMB-S-003) was added, and color development was performed at 37 °C for 3 min. 100 µL of ELISA stop solution (Solarbio, Cat: C1058) was added. OD450 was measured.

The results are shown in Table 4 and FIG. 5. All 4 fusion proteins bound significantly to BAFF and exhibited stronger binding capabilities than telitacicept. The capability of B575702 to bind to BAFF was consistent with that of B575704, and their EC₅₀ values decreased to 1/4 of that of telitacicept. The capability of B575701 to bind to BAFF was consistent with that of B575703, and their EC₅₀ values decreased to 1/2 of that of telitacicept.

**Table 4. The binding of TACI-BCMA fusion proteins to BAFF**

| BAFF binding | Telitacicept | B575701 | B575702 | B575703 | B575704 |
|---|---|---|---|---|---|
| Emax (nM) | 2.279 | 2.313 | 2.236 | 2.431 | 2.290 |
| EC₅₀ (nM) | 0.7132 | 0.3240 | 0.1664 | 0.3523 | 0.1867 |

As shown in Table 5 and FIG. 6. The 4 fusion proteins bound significantly to APRIL, and their binding capabilities were substantially consistent with each other. The capabilities of the 4 fusion proteins to bind to APRIL were all stronger than that of telitacicept, and their EC₅₀ values decreased to 1/4 to 1/3 of that of telitacicept.

**Table 5. The binding of TACI-BCMA fusion proteins to APRIL**

| APRIL binding | Telitacicept | B575701 | B575702 | B575703 | B575704 |
|---|---|---|---|---|---|
| Emax (nM) | 2.625 | 2.695 | 2.640 | 2.777 | 2.684 |
| EC₅₀ (nM) | 0.7106 | 0.2046 | 0.1712 | 0.2643 | 0.1887 |

### Example 6. Construction, Expression, and Purification of Fusion Proteins of Anifrolumab and TACI

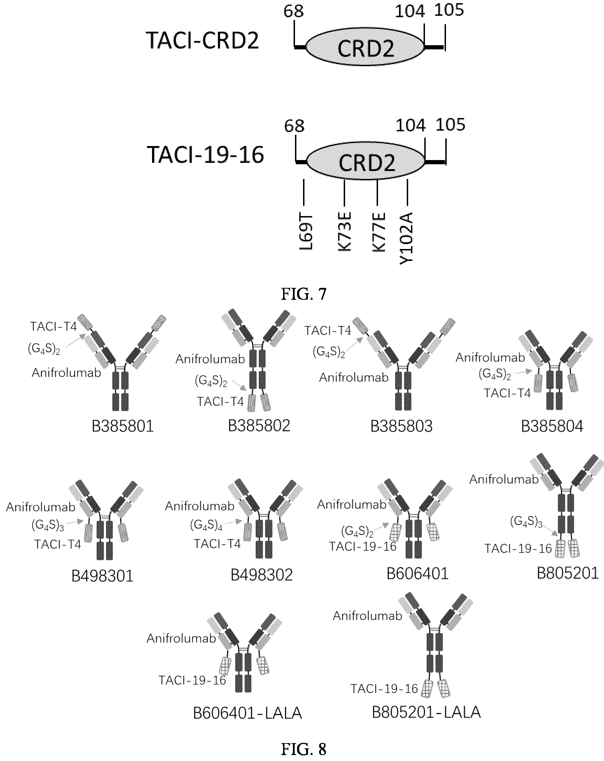

Anifrolumab is an antagonist targeting IFNAR1. It blocks the activation of cells by type I interferons through IFNAR1 and is used as a treatment for systemic lupus erythematosus. As shown in FIG. 7, TACI-CRD2 is a CRD2 ligand-binding domain containing amino acid residues 68-105, and TACI-19-16 is a polypeptide of TACI-CRD2 with several amino acid mutations (L69T, K73E, K77E, and Y102A).

As shown in FIG. 8, TACI-T4 or TACI-19-16 was linked to the N- or C-terminus of the heavy or light chain of anifrolumab by linkers with different lengths. B385801 was obtained by linking TACI-T4 to the N-terminus of the heavy chain of anifrolumab by linker 1, which was (G₄S)₂. B385802 was obtained by linking TACI-T4 to the C-terminus of the heavy chain of anifrolumab by linker 1. B385803 was obtained by linking TACI-T4 to the N-terminus of the light chain of anifrolumab by linker 1. B385804 was obtained by linking TACI-T4 to the C-terminus of the light chain of anifrolumab by linker 1. B498301 was obtained by linking TACI-T4 to the C-terminus of the light chain of anifrolumab by linker 2, which was (G₄S)₃. B498302 was obtained by linking TACI-T4 to the C-terminus of the light chain of anifrolumab by linker 3, which was (G₄S)₄. B606401 was obtained by linking TACI-19-16 to the C-terminus of the light chain of anifrolumab by linker 1. B805201 was obtained by linking TACI-19-16 to the C-terminus of the heavy chain of anifrolumab by linker 2. B606401-LALA was obtained by linking TACI-19-16 to the C-terminus of the light chain of anifrolumab by linker 1, and the Fc moiety is a human IgG1 Fc with mutations L234A and L235A. B805201-LALA was obtained by linking TACI-19-16 to the C-terminus of the heavy chain of anifrolumab by linker 2, and the Fc moiety is a human IgG1 Fc with mutations L234A and L235A.

TACI-19-16 is set forth in SEQ ID NO: 26, and the other sequences are shown below:
> Linker 1 (SEQ ID NO: 39)
   GGGGSGGGGS
> Linker 2 (SEQ ID NO: 40)
   GGGGSGGGGSGGGGS
> Linker 3 (SEQ ID NO: 41)
   GGGGSGGGGSGGGGSGGGGS
> TACI-CRD2 (SEQ ID NO: 42, the same as SEQ ID NO: 8)
   SLSCRKEQGKFYDHLLRDCISCASICGQHPKQCAYFCE
> The VH of anifrolumab (SEQ ID NO: 43)
> The VL of anifrolumab (SEQ ID NO: 44)
> The HCDR1 of anifrolumab (SEQ ID NO: 45)
   NYWIA
> The HCDR2 of anifrolumab (SEQ ID NO: 46)
   IIYPGDSDIRYSPSFQG
> The HCDR3 of anifrolumab (SEQ ID NO: 47)
   HDIEGFDY
> The LCDR1 of anifrolumab (SEQ ID NO: 48)
   RASQSVSSSFFA
> The LCDR2 of anifrolumab (SEQ ID NO: 49)
   GASSRAT
> The LCDR3 of anifrolumab (SEQ ID NO: 50)
   QQYDSSAIT
> The heavy chain of B385801 (SEQ ID NO: 51) 
AMRSCPEEQYWDPLLGTCMSCKTICNHQSQRTCAAFCRSLSCRKEQGKFYDHL
LRDCISCASICGQHPKQCAYFCENKLRSGGGGSGGGGSEVQLVQSGAEVKKPGE
SLKISCKGSGYIFTNYWIAWVRQMPGKGLESMGIIYPGDSDIRYSPSFQGQVTIS
ADKSITTAYLQWSSLKASDTAMYYCARHDIEGFDYWGRGTLVTVSSASTKGPS
VFPLAPSSKSTSGGTAALGCLVKDYFPEPVTVSWNSGALTSGVHTFPAVLQSSGL
YSLSSVVTVPSSSLGTQTYICNVNHKPSNTKVDKRVEPKSCDKTHTCPPCPAPEF
EGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPASIEKTISKAK
GQPREPQVYTLPPSRDELTKNQVSLTCLVKGFYPSDIAVEWESNGQPENNYKTTP
PVLDSDGSFFLYSKLTVDKSRWQQGNVFSCSVMHEALHNHYTQKSLSLSPGK
> The heavy chain of B385802 (SEQ ID NO: 52) 
EVQLVQSGAEVKKPGESLKISCKGSGYIFTNYWIAWVRQMPGKGLESMGIIYPG
DSDIRYSPSFQGQVTISADKSITTAYLQWSSLKASDTAMYYCARHDIEGFDYWG
RGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPK
SCDKTHTCPPCPAPEFEGGPSVFLFPPKPKDTLMISRTPEVTCVVVDVSHEDPEV
KFNWYVDGVEVHNAKTKPREEQYNSTYRVVSVLTVLHQDWLNGKEYKCKVS
NKALPASIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALH
NHYTQKSLSLSPGKGGGGSGGGGSAMRSCPEEQYWDPLLGTCMSCKTICNHQS
QRTCAAFCRSLSCRKEQGKFYDHLLRDCISCASICGQHPKQCAYFCENKLRS
> The heavy chain of B805201 (SEQ ID NO: 53) 
EVQLVQSGAEVKKPGESLKISCKGSGYIFTNYWIAWVRQMPGKGLESMGIIYPG
DSDIRYSPSFQGQVTISADKSITTAYLQWSSLKASDTAMYYCARHDIEGFDYWG
RGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPK
SCDKTHTCPPCPAPEFEGGPSVFLFPPKPKDTLMISRTPEVTCVVVDVSHEDPEV
KFNWYVDGVEVHNAKTKPREEQYNSTYRVVSVLTVLHQDWLNGKEYKCKVS
NKALPASIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALH
 
NHYTQKSLSLSPGKGGGGSGGGGSGGGGSSTSCREEQGEFYDHLLRDCISCASI
CGQHPKQCAAFCE
> The light chains of B385801, B385802, B805201, and B805201-LALA (SEQ ID NO: 54) 
EIVLTQSPGTLSLSPGERATLSCRASQSVSSSFFAWYQQKPGQAPRLLIYGASSRA
TGIPDRLSGSGSGTDFTLTITRLEPEDFAVYYCQQYDSSAITFGQGTRLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
> The heavy chains of B385803, B385804, B498301, B498302, and B606401 (SEQ ID NO: 55) 
EVQLVQSGAEVKKPGESLKISCKGSGYIFTNYWIAWVRQMPGKGLESMGIIYPG
DSDIRYSPSFQGQVTISADKSITTAYLQWSSLKASDTAMYYCARHDIEGFDYWG
RGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPK
SCDKTHTCPPCPAPEFEGGPSVFLFPPKPKDTLMISRTPEVTCVVVDVSHEDPEV
KFNWYVDGVEVHNAKTKPREEQYNSTYRVVSVLTVLHQDWLNGKEYKCKVS
NKALPASIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALH
NHYTQKSLSLSPGK
> The light chain of B385803 (SEQ ID NO: 56) 
AMRSCPEEQYWDPLLGTCMSCKTICNHQSQRTCAAFCRSLSCRKEQGKFYDHL
LRDCISCASICGQHPKQCAYFCENKLRSGGGGSGGGGSEIVLTQSPGTLSLSPGER
ATLSCRASQSVSSSFFAWYQQKPGQAPRLLIYGASSRATGIPDRLSGSGSGTDFTL
TITRLEPEDFAVYYCQQYDSSAITFGQGTRLEIKRTVAAPSVFIFPPSDEQLKSGTA
SVVCLLNNFYPREAKVQWKVDNALQSGNSQESVTEQDSKDSTYSLSSTLTLSK
ADYEKHKVYACEVTHQGLSSPVTKSFNRGEC
> The light chain of B385804 (SEQ ID NO: 57) 
EIVLTQSPGTLSLSPGERATLS5CRASQSVSSSFFAWYQQKPGQAPRLLIYGASSR
ATGIPDRLSGSGSGTDFTLTITRLEPEDFAVYYCQQYDSSAITFGQGTRLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGECGGGGS
GGGGSAMRSCPEEQYWDPLLGTCMSCKTICNHQSQRTCAAFCRSLSCRKEQGK
FYDHLLRDCISCASICGQHPKQCAYFCENKLRS
> The light chain of B498301 (SEQ ID NO: 58) 
EIVLTQSPGTLSLSPGERATLSCRASQSVSSSFFAWYQQKPGQAPRLLIYGASSRA
TGIPDRLSGSGSGTDFTLTITRLEPEDFAVYYCQQYDSSAITFGQGTRLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGECGGGGS
GGGGSGGGGSAMRSCPEEQYWDPLLGTCMSCKTICNHQSQRTCAAFCRSLSCR
KEQGKFYDHLLRDCISCASICGQHPKQCAYFCENKLRS
> The light chain of B498302 (SEQ ID NO: 59) 
EIVLTQSPGTLSLSPGERATLSCRASQSVSSSFFAWYQQKPGQAPRLLIYGASSRA
TGIPDRLSGSGSGTDFTLTITRLEPEDFAVYYCQQYDSSAITFGQGTRLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGECGGGGS
GGGGSGGGGSGGGGSAMRSCPEEQYWDPLLGTCMSCKTICNHQSQRTCAAFC
RSLSCRKEQGKFYDHLLRDCISCASICGQHPKQCAYFCENKLRS
> The light chains of B606401 and B606401-LALA (SEQ ID NO: 60) 
EIVLTQSPGTLSLSPGERATLSCRASQSVSSSFFAWYQQKPGQAPRLLIYGASSRA
TGIPDRLSGSGSGTDFTLTITRLEPEDFAVYYCQQYDSSAITFGQGTRLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGECGGGGS
GGGGSSTSCREEQGEFYDHLLRDCISCASICGQHPKQCAAFCE
> The heavy chain of B606401-LALA (SEQ ID NO: 61) 
EVQLVQSGAEVKKPGESLKISCKGSGYIFTNYWIAWVRQMPGKGLESMGIIYPG
DSDIRYSPSFQGQVTISADKSITTAYLQWSSLKASDTAMYYCARHDIEGFDYWG
RGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPK
SCDKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKVS
NKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALH
NHYTQKSLSLSPGK
> The heavy chain of B805201-LALA (SEQ ID NO: 62)

EVQLVQSGAEVKKPGESLKISCKGSGYIFTNYWIAWVRQMPGKGLESMGIIYPG
DSDIRYSPSFQGQVTISADKSITTAYLQWSSLKASDTAMYYCARHDIEGFDYWG
RGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPK
SCDKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKVS
NKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALH
NHYTQKSLSLSPGKGGGGSGGGGSGGGGSSTSCREEQGEFYDHLLRDCISCASI
CGQHPKQCAAFCE

The underlined part in the heavy chain is the Fc region of an IgG, the underlined part in the light chain is CH1, and the italicized part is a linker.

The protein preparation method provided in Example 1 was used. Transient transfection and protein expression were performed, and the proteins were loaded onto a Protein A affinity chromatography column and eluted. Analysis showed that the protein of interest was obtained.

### Example 7. Inhibition of Activity of Type I Interferons by Fusion Proteins of Anifrolumab and TACI

The inhibitory effects of B385801, B385802, B385803, B385804, B498301, B498302, and B606401, which are fusion proteins of anifrolumab and TACI, on the activity of IFN-α/β were assessed using a HEK-Blue IFN-α/β cell (InvivoGen, Cat: hkb-ifnab) kit as follows:
HEK-Blue^{™} IFN-α/β cells were digested, resuspended to 2.8E5/mL in a DMEM culture medium containing 10% FBS and 1% P/S (Gibco, Cat: 11995065), and seeded in a 96-well plate. The test proteins were added, and the plate was incubated at 37 °C for 30 min. IFN-β (Sino biological, Cat: 10704-HNAS) with a final concentration of 0.01 ng/mL was added, and the plate was incubated at 37 °C for 24 h. 20 µL of the culture supernatant was taken and mixed with 180 µL of Quanti-Blue (InvivoGen, Cat: rep-qbs) in a new 96-well plate. The plate was incubated at 37 °C for 1 h, and OD655 was measured.

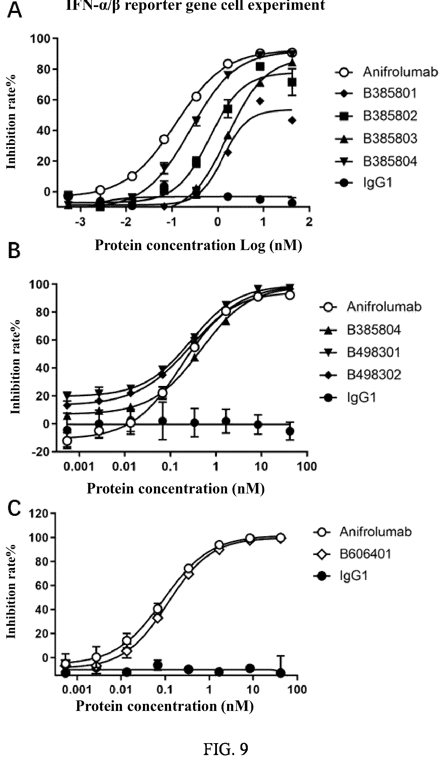

The results show that all the above 7 fusion proteins exhibited inhibitory effects on the activity of IFN-α/β; the activity of B385801 and B385803 is relatively weak and is followed by the activity of B385802; the activity of B385804, B498301, B498302, and B606401 is substantially comparable to that of anifrolumab (A of FIG. 9, B of FIG. 9, and C of FIG. 9).

### Example 8. In Vitro Binding of Fusion Proteins of Anifrolumab and TACI to BAFF

The capabilities of B385801, B385802, B385803, B385804, B498301, B498302, and B606401, which are fusion proteins of anifrolumab and TACI, to bind to BAFF were assessed by ELISA as follows:
BAFF (PeproTech, Cat: 310-13) was diluted to 1 µg/mL with PBS, and a 96-well plate was coated with the dilution overnight at 4 °C. The plate was washed with PBST and blocked with Blocking Buffer (PBST containing 1% BSA) at 37 °C for 1 h. The plate was tapped. The test antibodies were diluted with Blocking Buffer. Incubation was performed at 37 °C for 1.5 h. The plate was washed with PBST. An HRP-mouse anti-human Fc antibody (GenScript, Cat: A01854-200) was diluted with Blocking Buffer. Incubation was performed at 37 °C for 40 min. The plate was washed with PBST. 100 µL of TMB substrate solution (Biopanda, Cat: TMB-S-003) was added, and color development was performed at 37 °C for 3 min. 100 µL of ELISA stop solution (Solarbio, Cat: C1058) was added. OD450 was measured.

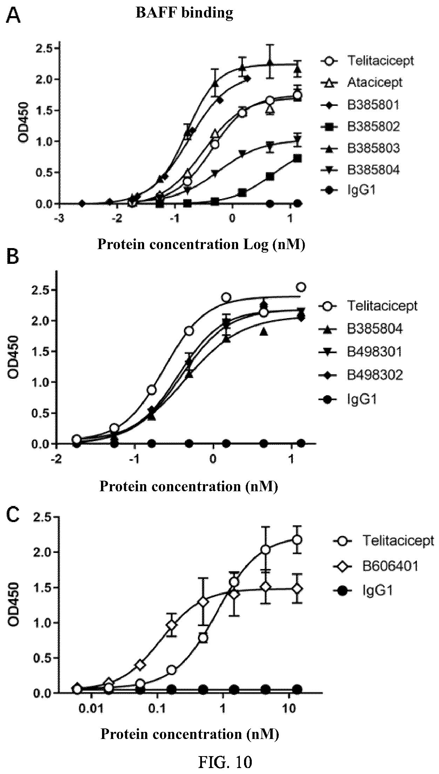

The results are shown in A of FIG. 10 to C of FIG. 10 and Table 6 and indicate that the 7 fusion proteins bound significantly to BAFF. B385801, B385803, and B606401 exhibited the strongest capabilities to bind to BAFF, and their capabilities are stronger than those of telitacicept and atacicept. The capability of B385802 to bind to BAFF is relatively weak. The capabilities of B385804, B498301, and B498302 to bind to BAFF are consistent with each other and slightly weaker than that of telitacicept. The length of the linker did not affect the capability of TACI to bind to BAFF.

**Table 6. The binding of fusion proteins of anifrolumab and TACI to BAFF**

| BAFF binding | Telitacicept | B606401 |
|---|---|---|
| Emax (nM) | 2.236 | 1.484 |
| EC₅₀ (nM) | 0.8006 | 0.1138 |

### Example 9. Construction, Expression, and Purification of Fusion Proteins of Anifrolumab and TACI-BCMA

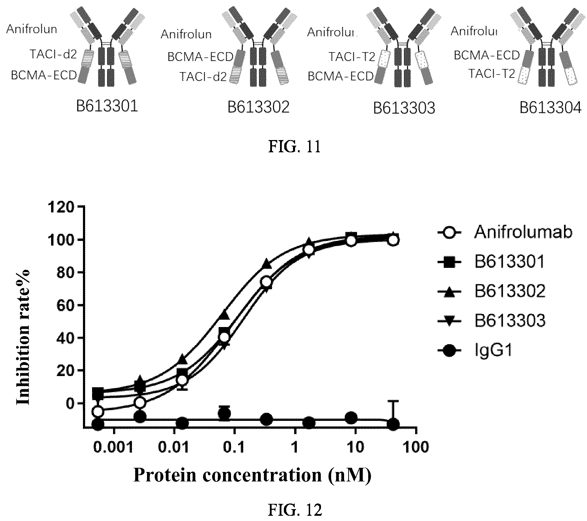

Referring to FIG. 11, the TACI-BCMA fusion proteins described in Example 4 were each linked to the C-terminus of the light chain of the antibody anifrolumab by linker 1. B613301 and B613302 were obtained by linking the fusion protein of TACI-d2 and BCMA-ECD to the C-terminus of the light chain of the antibody anifrolumab by linker 1. B613303 and B613304 were obtained by linking the fusion protein of TACI-T2 and BCMA-ECD to the C-terminus of the light chain of the antibody anifrolumab by linker 1.

The heavy chains of B613301, B613302, B613303, and B613304 are all anifrolumab's full-length heavy chain (SEQ ID NO: 55), and the specific sequences of their light chains are shown below, with light chain constant regions underlined:
> The light chain of B613301 (SEQ ID NO: 63) 
EIVLTQSPGTLSLSPGERATLSCRASQSVSSSFFAWYQQKPGQAPRLLIYGASSRA
TGIPDRLSGSGSGTDFTLTITRLEPEDFAVYYCQQYDSSAITFGQGTRLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGECGGGGS
GGGGSLSCRKEQGKFYDHLLRDCISCASICGQHPKQCAYFCENKLRSPGMLQM
AGQCSQNEYFDSLLHACIPCQLRCSSNTPPLTCQRYCNASVTNSVKGTNA
> The light chain of B613302 (SEQ ID NO: 64) 
EIVLTQSPGTLSLSPGERATLSCRASQSVSSSFFAWYQQKPGQAPRLLIYGASSRA
TGIPDRLSGSGSGTDFTLTITRLEPEDFAVYYCQQYDSSAITFGQGTRLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGECGGGGS
GGGGSMLQMAGQCSQNEYFDSLLHACIPCQLRCSSNTPPLTCQRYCNASVTNS
VKGTNALSCRKEQGKFYDHLLRDCISCASICGQHPKQCAYFCENKLRSPG
> The light chain of B613303 (SEQ ID NO: 65) 
EIVLTQSPGTLSLSPGERATLSCRASQSVSSSFFAWYQQKPGQAPRLLIYGASSRA
TGIPDRLSGSGSGTDFTLTITRLEPEDFAVYYCQQYDSSAITFGQGTRLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGECGGGGS
GGGGSSRVDQEERFPQGLWTGVAMRSCPEEQYWDPLLGTCMSCKTICNHQSQR
TCAAFCRSLSCRKEQGKFYDHLLRDCISCASICGQHPKQCAYFCENKLRSPVNL
PPELMLQMAGQCSQNEYFDSLLHACIPCQLRCSSNTPPLTCQRYCNASVTNSVK
GTNA
> The light chain of B613304 (SEQ ID NO: 66)

EIVLTQSPGTLSLSPGERATLSCRASQSVSSSFFAWYQQKPGQAPRLLIYGASSRA
TGIPDRLSGSGSGTDFTLTITRLEPEDFAVYYCQQYDSSAITFGQGTRLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGECGGGGS
GGGGSMLQMAGQCSQNEYFDSLLHACIPCQLRCSSNTPPLTCQRYCNASVTNS
VKGTNASRVDQEERFPQGLWTGVAMRSCPEEQYWDPLLGTCMSCKTICNHQS
QRTCAAFCRSLSCRKEQGKFYDHLLRDCISCASICGQHPKQCAYFCENKLRSPV
NLPPEL

The protein preparation method provided in Example 4 was used. Transient transfection and protein expression were performed, and the proteins were loaded onto a Protein A affinity chromatography column and eluted. Analysis showed that the protein of interest was obtained.

### Example 10. Inhibition of Activity of Type I Interferons by Fusion Proteins of Anifrolumab and TACI-BCMA

The inhibitory effects of B613301, B613302, and B613303, which are fusion proteins of anifrolumab and TACI-BCMA in Example 9, on the activity of IFN-α/β were assessed using a HEK-Blue IFN-α/β cell (InvivoGen, Cat: hkb-ifnab) kit as follows:
HEK-Blue^{™} IFN-α/β cells were digested, resuspended to 2.8E5/mL in a DMEM culture medium containing 10% FBS and 1% P/S (Gibco, Cat: 11995065), and seeded in a 96-well plate (Costar, Cat: 3599). The test antibodies were added, and the plate was incubated at 37 °C for 30 min. IFN-β (Sino biological, Cat: 10704-HNAS) with a final concentration of 0.01 ng/mL was added, and the plate was incubated at 37 °C for 24 h. 20 µL of the culture supernatant was taken and mixed with 180 µL of Quanti-Blue (InvivoGen, Cat: rep-qbs) in a new 96-well plate. The plate was incubated at 37 °C for 1 h, and OD655 was measured.

The results are shown in FIG. 12 and indicate that all 3 fusion proteins inhibited the activity of IFN-α/β; the 3 fusion proteins were comparable in activity, and their activity was consistent with that of anifrolumab. This indicates that fusing TACI-BCMA to anifrolumab, particularly to the C-terminus of the light chain, hardly affected the activity of anifrolumab.

### Example 11. In Vitro Binding of Fusion Proteins of Anifrolumab and TACI-BCMA to BAFF and APRIL

The capabilities of B613301, B613302, and B613303, which are fusion proteins of anifrolumab and TACI-BCMA, to bind to BAFF and APRIL were assessed by ELISA as follows:
BAFF (PeproTech, Cat: 310-13) or ARPIL (Acro Biosystems, Cat: APL-H52D1) was diluted to 1 µg/mL with PBS, and a 96-well plate (Costar, Cat: 3590) was coated with the dilution overnight at 4 °C. The plate was washed with PBST and blocked with Blocking Buffer (PBST containing 1% BSA) at 37 °C for 1 h. The plate was tapped. The test antibodies were diluted with Blocking Buffer. Incubation was performed at 37 °C for 1.5 h. The plate was washed with PBST. An HRP-mouse anti-human Fc antibody (GenScript, Cat: A01854-200) was diluted with Blocking Buffer. Incubation was performed at 37 °C for 40 min. The plate was washed with PBST. 100 µL of TMB substrate solution (Biopanda, Cat: TMB-S-003) was added, and color development was performed at 37 °C for 3 min. 100 µL of ELISA stop solution (Solarbio, Cat: C1058) was added. OD450 was measured.

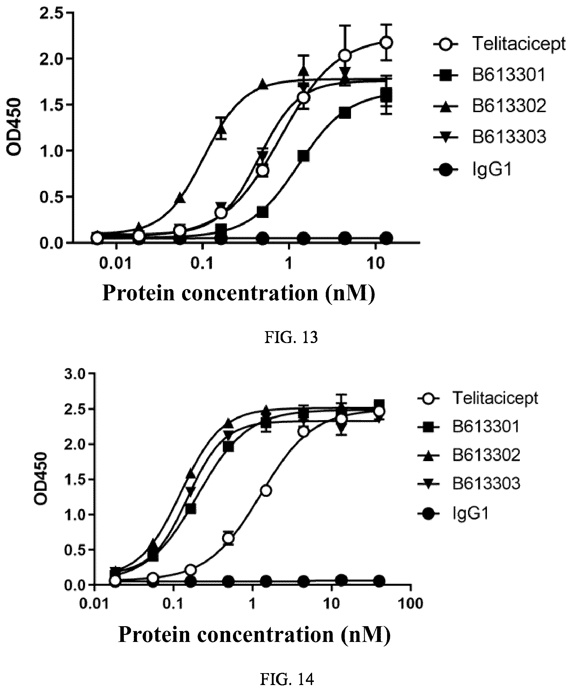

Referring to FIG. 13 and Table 7, the results show that all 3 fusion proteins bound significantly to BAFF. The capability of B613302 to bind to BAFF is significantly stronger than that of telitacicept, and its EC₅₀ value decreased to 1/8 to 1/7 of that of telitacicept. This indicates that placing TACI at the C-terminus of BCMA (as in the case of B613302) may be more conducive to maintaining the capability to bind to BAFF.

**Table 7. The binding of fusion proteins of anifrolumab and TACI-BCMA to BAFF**

| BAFF binding | Telitacicept | B613301 | B613302 | B613303 |
|---|---|---|---|---|
| Emax (nM) | 2.236 | 1.647 | 1.780 | 1.763 |
| EC₅₀ (nM) | 0.8006 | 1.294 | 0.1042 | 0.4483 |

Referring to FIG. 14 and Table 8, the results show that all 3 fusion proteins bound significantly to APRIL. The bindings of B613301, B613302, and B613303 to APRIL were substantially consistent with each other, and their EC₅₀ values decreased to 1/10 to 1/6 of that of telitacicept. The results indicate that the fusion proteins with fused BCMA did exhibit enhanced binding to APRIL.

**Table 8. The binding of fusion proteins of anifrolumab and TACI-BCMA to APRIL**

| APRIL binding | Telitacicept | B613301 | B613302 | B613303 |
|---|---|---|---|---|
| Emax (nM) | 2.498 | 2.489 | 2.515 | 2.328 |
| EC_{50 (}nM) | 1.255 | 0.2009 | 0.1236 | 0.1506 |

### Example 12. Construction, Expression, and Purification of Fusion Proteins of Anifrolumab and TACI-19-16-BCMA

BCMA-ECD-1 (amino acid residues 1-43) and BCMA-ECD-2 (amino acid residues 1-41) exclude risky sites that are likely to exist in the non-CRD domains of BCMA, such as the glycosylation sites (amino acid residues 42-44, NAS) and the deamidation sites (amino acid residues 47-48, NS). Excluding risky sites enables fusion proteins to be more homogeneous.

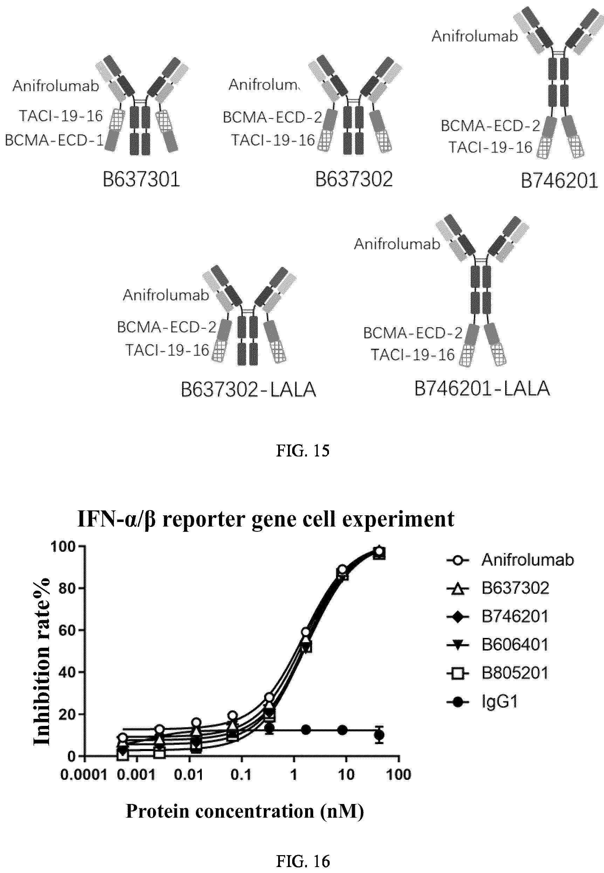

As shown in FIG. 15, B637301 was obtained by fusing TACI-19-16 to the N-terminus of BCMA-ECD-1 and then linking TACI-19-16-BCMA-ECD-1 to the C-terminus of the light chain of the antibody anifrolumab by linker 1; B637302 was obtained by fusing TACI-19-16 to the C-terminus of BCMA-ECD-2 and then linking BCMA-ECD-2-TACI-19-16 to the C-terminus of the light chain of the antibody anifrolumab by linker 1; B746201 was obtained by fusing TACI-19-16 to the C-terminus of BCMA-ECD-2 and then linking BCMA-ECD-2-TACI-19-16 to the C-terminus of the heavy chain of the antibody anifrolumab by linker 2. B637302-LALA was obtained by fusing TACI-19-16 to the C-terminus of BCMA-ECD-2 and then linking BCMA-ECD-2-TACI-19-16 to the C-terminus of the light chain of the antibody anifrolumab by linker 1, and the Fc moiety of the antibody anifrolumab is a human IgG1 Fc with mutations L234A and L235A. B746201-LALA was obtained by fusing TACI-19-16 to the C-terminus of BCMA-ECD-2 and then linking BCMA-ECD-2-TACI-19-16 to the C-terminus of the heavy chain of the antibody anifrolumab by linker 2, and the Fc moiety of the antibody anifrolumab is a human IgG1 Fc with mutations L234A and L235A. See FIG. 15.
> BCMA-ECD-1 (SEQ ID NO: 67)
   MLQMAGQCSQNEYFDSLLHACIPCQLRCSSNTPPLTCQRYCNA
> BCMA-ECD-2 (SEQ ID NO: 68)
   MLQMAGQCSQNEYFDSLLHACIPCQLRCSSNTPPLTCQRYC
> TACI-19-16-BCMA-ECD-1 (SEQ ID NO: 69) 
STSCREEQGEFYDHLLRDCISCASICGQHPKQCAAFCEMLQMAGQCSQNEYFDS
LLHACIPCQLRCSSNTPPLTCQRYCNA
> BCMA-ECD-2-TACI-19-16 (SEQ ID NO: 70)

MLQMAGQCSQNEYFDSLLHACIPCQLRCSSNTPPLTCQRYCSTSCREEQGEFYD
HLLRDCISCASICGQHPKQCAAFCE

The heavy chains of B637301 and B637302 are both anifrolumab's heavy chain SEQ ID NO: 55, and the sequences of their light chains are shown below:
> The light chain of B637301 (SEQ ID NO: 71) 
EIVLTQSPGTLSLSPGERATLSCRASQSVSSSFFAWYQQKPGQAPRLLIYGASSRA
TGIPDRLSGSGSGTDFTLTITRLEPEDFAVYYCQQYDSSAITFGQGTRLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGECGGGGS
GGGGSSTSCREEQGEFYDHLLRDCISCASICGQHPKQCAAFCEMLQMAGQCSQ
NEYFDSLLHACIPCQLRCSSNTPPLTCQRYCNA
> The light chains of B637302 and B637302-LALA (SEQ ID NO: 72)

EIVLTQSPGTLSLSPGERATLSCRASQSVSSSFFAWYQQKPGQAPRLLIYGASSRA
TGIPDRLSGSGSGTDFTLTITRLEPEDFAVYYCQQYDSSAITFGQGTRLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGECGGGGS
GGGGSMLQMAGQCSQNEYFDSLLHACIPCQLRCSSNTPPLTCQRYCSTSCREEQ
GEFYDHLLRDCISCASICGQHPKQCAAFCE

The heavy chain of B637302-LALA is SEQ ID NO: 61.
> The heavy chain of B746201 (SEQ ID NO: 73)

EVQLVQSGAEVKKPGESLKISCKGSGYIFTNYWIAWVRQMPGKGLESMGIIYPG
DSDIRYSPSFQGQVTISADKSITTAYLQWSSLKASDTAMYYCARHDIEGFDYWG
RGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPK
SCDKTHTCPPCPAPEFEGGPSVFLFPPKPKDTLMISRTPEVTCVVVDVSHEDPEV
KFNWYVDGVEVHNAKTKPREEQYNSTYRVVSVLTVLHQDWLNGKEYKCKVS
NKALPASIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALH
NHYTQKSLSLSPGKGGGGSGGGGSGGGGSMLQMAGQCSQNEYFDSLLHACIPC
QLRCSSNTPPLTCQRYCSTSCREEQGEFYDHLLRDCISCASICGQHPKQCAAFCE

The light chains of B746201 and B746201-LALA are anifrolumab's light chain SEQ ID NO: 54.
> The heavy chain of B746201-LALA (SEQ ID NO: 74)

EVQLVQSGAEVKKPGESLKISCKGSGYIFTNYWIAWVRQMPGKGLESMGIIYPG
DSDIRYSPSFQGQVTISADKSITTAYLQWSSLKASDTAMYYCARHDIEGFDYWG
RGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPK
SCDKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKVS
NKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALH
NHYTQKSLSLSPGKGGGGSGGGGSGGGGSMLQMAGQCSQNEYFDSLLHACIPC
QLRCSSNTPPLTCQRYCSTSCREEQGEFYDHLLRDCISCASICGQHPKQCAAFCE

Fusion proteins B637301, B637302, and B746201 were expressed and purified. SDS-PAGE analysis showed that the antibody heavy and light chains linked with TACI-BCMA appeared as single bands (results not shown). The results indicate that excluding sites at risk of glycosylation in the sequence of BCMA protein enables fusion proteins to be more homogeneous.

### Example 13. Inhibition of Activity of Type I Interferons by Fusion Proteins of Anifrolumab and TACI-19-16-BCMA

The inhibitory effects of fusion proteins of anifrolumab and TACI-19-16-BCMA (B606401, B637302, B746201, and B805201) on the activity of IFN-α/β were assessed using a HEK-Blue IFN-α/β cell (InvivoGen, Cat: hkb-ifnab) kit as follows:
HEK-Blue^{™} IFN-α/β cells were digested, resuspended to 2.8E5/mL in a DMEM culture medium containing 10% FBS and 1% P/S (Gibco, Cat: 11995065), and seeded in a 96-well plate (Costar, Cat: 3599). The test antibodies were added, and the plate was incubated at 37 °C for 30 min. IFN-β (Sino biological, Cat: 10704-HNAS) with a final concentration of 0.01 ng/mL was added, and the plate was incubated at 37 °C for 24 h. 20 µL of the culture supernatant was taken and mixed with 180 µL of Quanti-Blue (InvivoGen, Cat: rep-qbs) in a new 96-well plate. The plate was incubated at 37 °C for 1 h, and OD655 was measured.

Referring to FIG. 16 and Table 9, the results show that all 4 fusion proteins inhibited the activity of IFN-α/β; the 4 fusion proteins were comparable in activity, and their activity was consistent with that of anifrolumab.

**Table 9. Results for the inhibition of the activity of type I interferons by fusion proteins of anifrolumab and TACI-19-16-BCMA**

| Antibody drug | Anifrolumab | B637302 | B746201 | B606401 | B805201 |
|---|---|---|---|---|---|
| IC₅₀ (nM) | 1.53 | 1.59 | 1.64 | 1.94 | 1.63 |

### Example 14. In Vitro Binding of Fusion Proteins of Anifrolumab and TACI-19-16-BCMA to BAFF and APRIL

The capabilities of fusion proteins B606401, B637302, B746201, and B805201 to bind to BAFF and APRIL were assessed by ELISA as follows:
BAFF (PeproTech, Cat: 310-13) or ARPIL (Acro Biosystems, Cat: APL-H52D1) was diluted to 1 µg/mL with PBS, and a 96-well plate was coated with the dilution overnight at 4 °C. The plate was washed with PBST and blocked with Blocking Buffer (PBST containing 1% BSA) at 37 °C for 1 h. The plate was tapped. The test antibodies were diluted with Blocking Buffer. Incubation was performed at 37 °C for 1.5 h. The plate was washed with PBST. An HRP-mouse anti-human Fc antibody (GenScript, Cat: A01854-200) was diluted with Blocking Buffer. Incubation was performed at 37 °C for 40 min. The plate was washed with PBST. 100 µL of TMB substrate solution (Biopanda, Cat: TMB-S-003) was added, and color development was performed at 37 °C for 3 min. 100 µL of ELISA stop solution (Solarbio, Cat: C1058) was added. OD450 was measured.

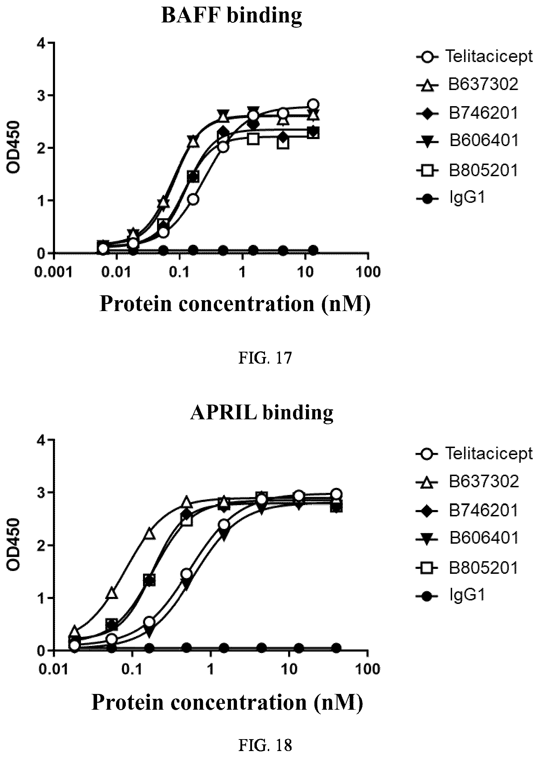

Referring to FIG. 17 and Table 10, all 4 fusion proteins bound significantly to BAFF, and the capabilities of all 4 fusion proteins to bind to BAFF are stronger than that of telitacicept. The capabilities of B606401 and B637302 to bind to BAFF are comparable, and their EC₅₀ values decreased to 1/4 to 1/3 of that of telitacicept. The capabilities of B746201 and B805201 to bind to BAFF are comparable, and their EC₅₀ values decreased to 1/2 of that of telitacicept.

**Table 10. The binding of fusion proteins of anifrolumab and TACI-19-16-BCMA to BAFF**

| BAFF binding | Telitacicept | B637302 | B746201 | B606401 | B805201 |
|---|---|---|---|---|---|
| Emax (nM) | 2.792 | 2.609 | 2.353 | 2.621 | 2.218 |
| EC₅₀ (nM) | 0.2555 | 0.0772 | 0.1290 | 0.0823 | 0.1169 |

Referring to FIG. 18 and Table 11, all 4 fusion proteins bound significantly to APRIL. The EC₅₀ value for the capability of B637302 to bind to APRIL decreased to 1/7 to 1/6 of that of telitacicept. The capabilities of B746201 and B805201 to bind to APRIL are comparable, and their EC₅₀ values decreased to 1/3 of that of telitacicept. The capability of B606401 to bind to APRIL is comparable to that of telitacicept.

**Table 11. The binding of fusion proteins of anifrolumab and TACI-19-16-BCMA to APRIL**

| APRIL binding | Telitacicept | B637302 | B746201 | B606401 | B805201 |
|---|---|---|---|---|---|
| Emax (nM) | 2.984 | 2.899 | 2.798 | 2.814 | 2.855 |
| EC₅₀ (nM) | 0.5356 | 0.0819 | 0.1794 | 0.6072 | 0.1820 |

### Example 15. Plasmacytoid Dendritic Cell (pDC) Functional Experiment

To evaluate the *in vitro* efficacy of fusion proteins of anifrolumab and TACI-19-16-BCMA, a method for testing IFNAR1 antagonist bioactive molecules *in vitro* was established. The pDC functional experiment was as follows: Freshly isolated healthy human PBMCs were cultured *in vitro* in a 1640 basal culture medium containing GlutaMAX (with a sugar concentration of 11 mM), seeded in a 96-well plate at a density of 3 × 10⁶/mL, stimulated with 0.5 µM CpG-A ODN 2216 (InvivoGen cat: tlrl-2216), and treated with gradient concentrations of anifrolumab, B637302, B606401, and IgG1 isotype. After 24 h, the level of IFN-α cytokine secretion in the cell culture supernatant was determined using a human IFN-α kit (Cisbio cat: 62HIFNAPEG).

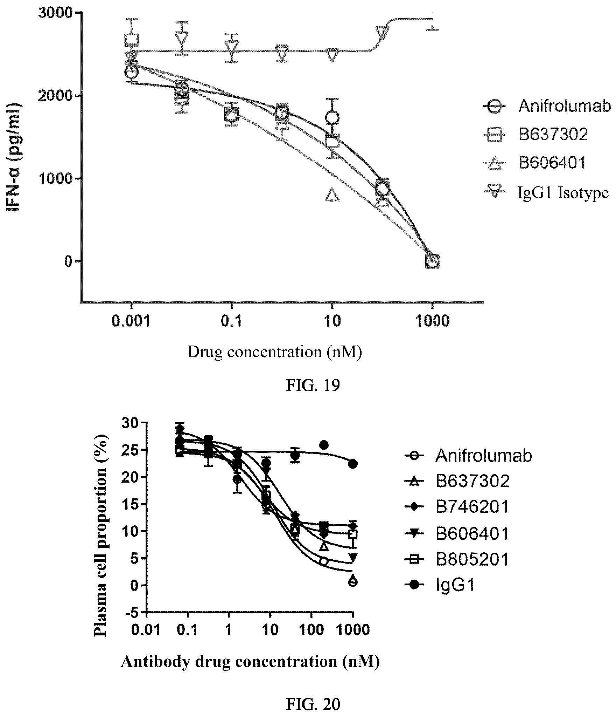

The results show that B637302, B606401, and the control antibody anifrolumab all inhibited IFN-α production in a dose-dependent manner and were comparable in inhibitory activity (FIG. 19).

### Example 16. IFN-α-Induced In Vitro Plasma Cell Differentiation Functional Experiment

To evaluate the antagonistic bioactivity of fusion proteins of anifrolumab and TACI-19-16-BCMA against IFNAR1, an *in vitro* plasma cell differentiation functional experimental method was established. Specifically, the method was as follows: Human B cells were isolated from fresh healthy human PBMCs using a B cell sorting kit (Stemcell cat: 17954), cultured *in vitro* in a 1640 basal culture medium containing GlutaMAX (Gibco cat: 72400-47), seeded in a 96-well plate at a density of 1 × 10⁵/well, stimulated with 2 µg/mL CpG-B 2006 (InvivoGen cat: tlrl-2006) and 250 U/mL IFN-α

(Biolegend cat: 592704), and further treated with gradient concentrations of anifrolumab, B637302, B746201, B606401, B805201, and IgG1 isotype as a control. After 4 days, the plasma cell (CD27⁺ CD38⁺) differentiation proportion was determined by flow cytometry.

Referring to FIG. 20, the results show that B637302, B746201, B606401, B805201, and the control antibody anifrolumab all effectively inhibited IFN-α-induced *in vitro* differentiation of B cells into plasma cells in a dose-dependent manner, with IC₅₀ values of 10.75 nM, 1.895 nM, 17.45 nM, 6.208 nM, and 12.66 nM, respectively, suggesting that the inhibitory activity of the fusion proteins is comparable to that of the control antibody anifrolumab.

### Example 17. BAFF-Induced B Cell Proliferation Functional Experiment

To evaluate the *in vitro* efficacy of fusion proteins of anifrolumab and TACI-19-16-BCMA, a method for testing TACI-BCMA antagonist bioactive molecules *in vitro* was established. The steps of the BAFF-induced *in vitro* B cell proliferation experiment were as follows: Human B cells were isolated from fresh healthy human PBMCs using a B cell sorting kit (Stemcell cat: 17954), labeled with CTV (Invitrogen cat: C34557), cultured *in vitro* in a 1640 basal culture medium containing GlutaMAX (Gibco cat: 72400-47), and seeded in a 96-well plate at a density of 1 × 10⁵/well. 20 µg/mL anti-human IgM antibody (Jacksonimmuno cat: 109-006-129), 10 ng/mL recombinant human IL-4 (PeproTech cat: AF-200-04-20), 100 ng/mL CD40L (R&D cat: 2706-CL-025/CF), 1.5 µg/mL anti-His antibody (R&D cat: MAB050-500), and 1 ng/mL recombinant human IL-17 (SinoBiological cat: 12047-HNAS) were added, and 200 ng/mL recombinant human BAFF (R&D cat: 7537-BF-025/CF) was further added for stimulation. On this basis, the B cells were treated with gradient concentrations of telitacicept, B637302, B606401, B80521, and IgG1 isotype. After 5 days, B cell proliferation signals were detected by flow cytometry.

The results show that, referring to Table 12, B637302, B606401, B805201, and the control telitacicept all effectively inhibited BAFF-induced *in vitro* proliferation of B cells in a dose-dependent manner. Referring to FIG. 21, the inhibitory effects of B637302 and B805201 are significantly stronger than that of telitacicept; under the condition of 10 nM low-concentration drug treatment, telitacicept did not exhibit inhibitory activity, while B637302, B606401, and B805201 all exhibited significant activity of inhibiting plasma cell differentiation.

**Table 12. BAFF-induced B cell proliferation results**

| Drug concentration (nM) | Flow cytometry proliferating B cell proportion (%) | | | | | | | | | |
|---|---|---|---|---|---|---|---|---|---|---|
| | IgG1 | | Telitacicept | | B637302 | | B606401 | | B805201 | |
| | Mean value | Standard deviation | Mean value | Standard deviation | Mean value | Standard deviation | Mean value | Standard deviation | Mean value | Standard deviation |
| 10 | 40.15 | 0.21 | 36.00 | 2.69 | 23.75 | 0.21 | 28.13 | 1.46 | 20.37 | 2.42 |
| 100 | 40.60 | 0.44 | 30.73 | 0.87 | 22.20 | 1.13 | 25.27 | 0.59 | 24.33 | 0.12 |
| 1000 | 33.97 | 3.52 | 24.33 | 0.81 | 10.93 | 0.70 | 17.87 | 0.31 | 14.97 | 1.16 |

### Example 18. APRIL-Induced B Cell Proliferation Functional Experiment

To evaluate the *in vitro* efficacy of fusion proteins of anifrolumab and TACI-19-16-BCMA, a method for testing TACI-BCMA antagonist bioactive molecules *in vitro* was established. The steps of the APRIL-induced *in vitro* B cell proliferation experiment were as follows: Human B cells were isolated from fresh healthy human PBMCs using a B cell sorting kit (Stemcell cat: 17954), labeled with CTV (Invitrogen cat: C34557), cultured *in vitro* in a 1640 basal culture medium containing GlutaMAX (Gibco cat: 72400-47), and seeded in a 96-well plate at a density of 1 × 10⁵/well. 20 µg/mL anti-human IgM antibody (Jacksonimmuno cat: 109-006-129), 10 ng/mL recombinant human IL-4 (PeproTech cat: AF-200-04-20), 100 ng/mL CD40L (R&D cat: 2706-CL-025/CF), 1.5 µg/mL anti-His antibody (R&D cat: MAB050-500), and 1 ng/mL recombinant human IL-17 (SinoBiological cat:12047-HNAS) were added, and 20 ng/mL recombinant human ARPIL (R&D cat: 5860-AP-010/CF) was further added for stimulation. On this basis, the B cells were treated with gradient concentrations of telitacicept, B637302, B606401, and IgG1 isotype. After 5 days, B cell proliferation signals were detected by flow cytometry.

The results show that: referring to Table 13 and FIG. 22, B637302, B606401, and the control telitacicept all effectively inhibited APRIL-induced *in vitro* proliferation of B cells in a dose-dependent manner, and B637302 was significantly superior to telitacicept in efficacy; under the condition of 1000 nM drug treatment, B606401 was also significantly superior to telitacicept in efficacy.

**Table 13. APRIL-induced B cell proliferation results**

| Drug concentration (nM) | Flow cytometry proliferating B cell proportion (%) | | | | | | | |
|---|---|---|---|---|---|---|---|---|
| | IgG1 | | Telitacicept | | B637302 | | B606401 | |
| | Mean value | Standard deviation | Mean value | Standard deviation | Mean value | Standard deviation | Mean value | Standard deviation |
| 4.12 | 55.30 | | 48.07 | 0.42 | 50.60 | 1.23 | 52.00 | 1.56 |
| 12.35 | 54.80 | 1.61 | 49.23 | 0.21 | 50.70 | 1.57 | 52.87 | 1.80 |
| 37.04 | 56.63 | 1.42 | 50.07 | 1.26 | 53.63 | 0.49 | 53.63 | 0.98 |
| 111.11 | 55.83 | 0.72 | 50.33 | 0.98 | 51.53 | 0.90 | 53.07 | 0.74 |
| 333.33 | 56.27 | 1.06 | 49.13 | 0.49 | 47.97 | 1.36 | 50.57 | 0.74 |
| 1000.00 | 53.27 | 1.10 | 46.30 | 1.01 | 33.60 | 0.30 | 40.33 | 1.74 |

### Example 19. BAFF + APRIL-Induced Plasma Cell Production Functional Experiment

To evaluate the TACI-BCMA bioactivity of fusion proteins of anifrolumab and TACI-19-16-BCMA, a BAFF + APRIL-induced *in vitro* plasma cell production experimental method was established. Specifically, the process was as follows: Human B cells were isolated from fresh healthy human PBMCs using a B cell sorting kit (Stemcell cat: 17954), cultured *in vitro* in a 1640 basal culture medium containing GlutaMAX (Gibco cat: 72400-47), seeded in a 96-well plate at a density of 1 × 10⁵/well, and stimulated with 2 µg/mL CpG-B 2006 (InvivoGen cat: tlrl-2006). On day 4, the culture medium was replaced, and 10 ng/mL recombinant human IL-6, 50 ng/mL recombinant human IL-10 (Peprotech cat: 200-10), 50 ng/mL recombinant human IL-21 (Peprotech cat: AF-200-21-10), 500 ng/mL recombinant human BAFF (R&D cat: 7537-BF-025/CF), and 50 ng/mL recombinant human ARPIL (R&D cat: 5860-AP-010/CF) were added for stimulation. After 7 days, half of the culture medium was replaced, the concentrations of BAFF and ARPIL were adjusted to 50 ng/mL and 500 ng/mL, and the concentrations of the other stimulation signals remained unchanged. In this process, the cells were treated with gradient concentrations of telitacicept, B637302, B746201, B606401, B805201, and IgG1 isotype on days 4 to 10, and plasma cells (CD27⁺ CD38⁺) were counted by flow cytometry on day 10.

Referring to Table 14, the plasma cell counts obtained by flow cytometry on day 10 show that BAFF and ARPIL effectively induced *in vitro* production of plasma cells. Referring to FIG. 23, under the condition of 10 nM drug treatment, telitacicept did not inhibit plasma cell production, while B637302, B746201, B606401, and B805201 significantly inhibited plasma cell production, indicating that B637302, B746201, B606401, and B805201 are superior to telitacicept in inhibitory activity against BAFF and ARPIL; under the condition of 100 nM drug treatment, all 4 test drugs and telitacicept significantly inhibited plasma cell production; under the condition of 1000 nM drug treatment, B637302 exhibited the best inhibitory activity against plasma cell production and was superior to telitacicept.

**Table 14. BAFF + APRIL-induced plasma cell production results**

| Drug concentration (nM) | CD27+ CD38+ plasma cell count | | | | | | | | | | | |
|---|---|---|---|---|---|---|---|---|---|---|---|---|
| | IgG1 | | Telitacicept | | B637302 | | B746201 | | B606401 | | B805201 | |
| | Mean value | Standard deviation | Mean value | Standard deviation | Mean value | Standard deviation | Mean value | Standard deviation | Mean value | Standard deviation | Mean value | Standard deviation |
| 10 | 2687 | 432 | 2451 | 110 | 563 | 76 | 575 | 112 | 654 | 34 | 434 | 107 |
| 100 | 2496 | 256 | 510 | 80 | 482 | 60 | 520 | 0 | 549 | 29 | 426 | 32 |
| 1000 | 2279 | 379 | 230 | 82 | 137 | 30 | 410 | 101 | 426 | 27 | 438 | 106 |

### Example 20. IFN-α + BAFF + APRIL-Induced Plasma Cell Production Functional Experiment

To evaluate the synergistic bioactivity of anifrolumab and TACI-BCMA in fusion proteins of anifrolumab and TACI-19-16-BCMA in the process of B cells differentiating into plasma cells and in the process of plasma cell production, an IFN-α + BAFF + APRIL-induced *in vitro* plasma cell production experimental method was established. Specifically, the process was as follows: Human B cells were isolated from fresh healthy human PBMCs using a B cell sorting kit (Stemcell cat: 17954), cultured *in vitro* in a 1640 basal culture medium containing GlutaMAX (Gibco cat: 72400-47), seeded in a 96-well plate at a density of 1 × 10⁵/well, and stimulated with 2 µg/mL CpG-B 2006 (InvivoGen cat: tlrl-2006) and 250 U/mL IFN-α (Biolegend cat: 592704). On day 4, the culture medium was replaced, and 10 ng/mL recombinant human IL-6, 50 ng/mL recombinant human IL-10 (Peprotech cat: 200-10), 15 ng/mL recombinant human IL-21 (Peprotech cat: AF-200-21-10), 500 ng/mL recombinant human BAFF (R&D cat: 7537-BF-025/CF), and 50 ng/mL recombinant human ARPIL (R&D cat: 5860-AP-010/CF) were added for stimulation. After 7 days, half of the culture medium was replaced, the concentrations of BAFF and ARPIL were adjusted to 50 ng/mL and 500 ng/mL, and the concentrations of the other stimulation signals remained unchanged. In this process, the cells were treated with gradient concentrations of anifrolumab, telitacicept, B637302, B606401, and IgG1 isotype on days 0 to 10, and plasma cells (CD27⁺ CD38⁺) were counted by flow cytometry on day 10 (A of FIG. 24).

In the *in vitro* plasma cell production synergistic experiment, through optimization of experimental conditions, IFN-α and BAFF + APRIL were found to be capable of synergistically promoting the differentiation and production of plasma cells under the condition of 15 ng/mL IL-21, and their promoting effects are comparable. Therefore, the inhibitory activity of various protein drugs at different concentrations was compared under that condition. The results show that B637302, B606401, telitacicept, and anifrolumab all inhibited plasma cell production in a dose-dependent manner. See Table 15 and B to D of FIG. 24. Under the condition of 10 nM low-concentration drug treatment, telitacicept or anifrolumab alone did not exhibit significant inhibitory activity against plasma cell production, while B637302 and B606401 did. See B of FIG. 24. Under the condition of 100 nM drug treatment, anifrolumab alone did not exhibit significant inhibitory activity, telitacicept alone exhibited some activity, and B637302 is also significantly superior to telitacicept in activity. See C of FIG. 24. Under the condition of 1000 nM drug treatment, anifrolumab alone exhibited some activity, and fusion proteins B637302 and B606401 are also significantly superior to anifrolumab in activity. See D of FIG. 24.

**Table 15. IFN-α + BAFF + APRIL-induced plasma cell production results**

| Drug concentration (nM) | CD27⁺ CD38⁺ plasma cell count | | | | | | | | | |
|---|---|---|---|---|---|---|---|---|---|---|
| | IgG1 | | Anifrolumab | | Telitacicept | | B637302 | | B606401 | |
| | Mean value | Standard deviation | Mean value | Standard deviation | Mean value | Standard deviation | Mean value | Standard deviation | Mean value | Standard deviation |
| 10 | 3370 | 297 | 4312 | 546 | 3906 | 230 | 1754 | 308 | 2275 | 416 |
| 100 | 3964 | 111 | 3703 | 198 | 2056 | 142 | 1391 | 259 | 1457 | 714 |
| 1000 | 3918 | 111 | 1633 | 297 | 1015 | 113 | 704 | 416 | 1054 | 136 |

### Example 21. PEG-IFN-α-Induced PBMC-Humanized Mouse PD Experiment

To assess the *in vivo* efficacy of the anifrolumab end in fusion proteins of anifrolumab and TACI-19-16-BCMA, B-NDG immunodeficient mice were humanized by injection of 2 × 10⁷ PBMCs into the tail vein and induced by intraperitoneal injection of 0.6 µg of PEG-IFN-α (PEGASYS^{®}, Roche). In this model, the drugs anifrolumab, B637302, and B606401, as well as PBS, at different concentrations, were intraperitoneally injected to treat the mice. PBMCs were collected at 2 h, and pSTAT1 (#9167, Cell Signaling Technology) levels were measured by flow cytometry. Peripheral blood was collected at time points days 1 and 3, PBMCs were separated, and the mRNA expression levels of genes downstream of IFN-α (such as ISG-15, IFI13, MX-1, and HERC5) were measured by QPCR. See A of FIG. 25 and B of FIG. 25.

Referring to C of FIG. 25, PBMCs were collected at 2 h post-induction and assayed for pSTAT1 by flow cytometry, and the results show that B637302, B606401, and anifrolumab are comparable in inhibitory activity against hIFN-α. Referring to D and E of FIG. 25, the QPCR measurements of the mRNA expression levels of ISG on days 1, 3, and 7 also show similar results, which are consistent with the results of the foregoing *in vitro* reporter molecule tests and biological activity experiments.

### Example 22. BAFF + APRIL-Induced Mouse PD Experiment

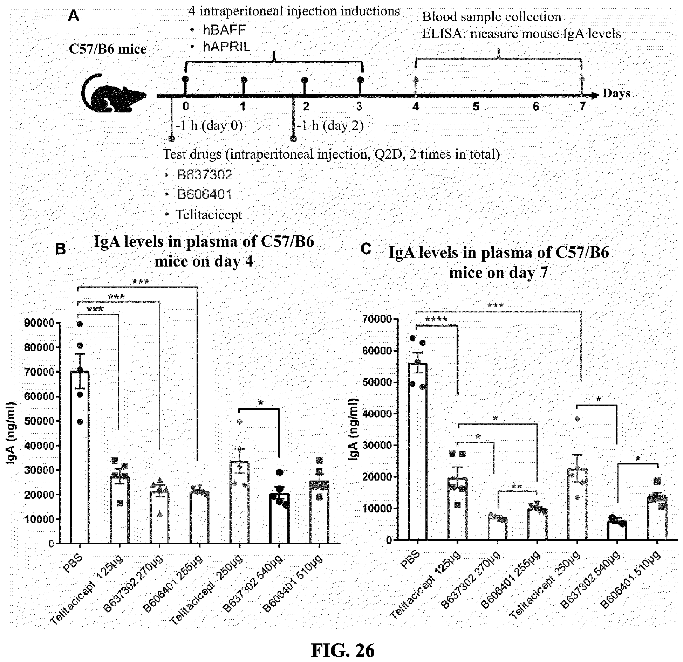

To assess the *in vivo* efficacy of the TACI-19-16-BCMA end in fusion proteins of anifrolumab and TACI-19-16-BCMA, C57/B6 mice were induced by intraperitoneal injection of 3 µg of BAFF (BAF-H52D4-1mg, AcroBio) and 0.5 µg of APRIL (APL-H52D1-1mg, AcroBio). In this model, the drugs telitacicept, B637302, and B606401, as well as PBS, at different concentrations were intraperitoneally injected to treat the mice. Peripheral blood was collected on days 4 and 7, and the IgA levels in the plasma were measured by ELISA (A of FIG. 26).

The results show that there were no significant differences in IgA level between the PBS group and the administration groups on days 0 and 2 (results not shown), while B637302, B606401, and telitacicept significantly inhibited IgA production on days 4 and 7 compared to the PBS control group. See B of FIG. 26 and C of FIG. 26. Moreover, the results from day 7 also show that under equimolar administration conditions, B637302 > B606401 > telitacicept in terms of efficacy. See C of FIG. 26. This is consistent with the results of the foregoing *in vitro* ELISA binding assays for BAFF and APRIL and *in vitro* biological activity experiments.

### Example 23. Establishment of Conditions for In Vitro Differentiation of B Cells

Existing experiments for evaluating B cell-related functions primarily involve using the 3H-labeled thymidine (TdR) method. The present disclosure provides a new *in vitro* experimental system for the differentiation of B cells into plasma cells, and the system can be used for evaluating immunosuppressants, such as IFNAR1 inhibitors and BAFF/ARPIL antagonists. To establish an *in vitro* B cell differentiation experimental system, basal stimulation conditions for effectively inducing *in vitro* differentiation of B cells need to be explored.

The conditions for the sorting and *in vitro* culture of B cells were as follows: Human B cells isolated from fresh healthy human PBMCs using a B cell sorting kit (Stemcell cat: 17954) were cultured *in vitro* in a 1640 GlutaMAX (Gibco cat: 72400-47) basal culture medium containing 50 µM β-mercaptoethanol (Sigma-Aldrich cat: M3148), MEM non-essential amino acids (Gibco cat: 11140050), sodium pyruvate (Gibco cat: 11360070), and GlutaMAXTM (Gibco cat: 35050061).

### 1) Conditions for in vitro differentiation of B cells

B cells were seeded in a 96-well plate at a density of 1 × 10⁵/well. 10 ng/mL recombinant human IL-3 and 0.5 µM CpG-A were added, and 1000 U/mL IFNα or 33 ng/mL recombinant human IL-21 control was further added for stimulation. After 6 days, the plasma cell (CD27⁺ CD38⁺) differentiation proportion and cell viability were determined by flow cytometry.

The results show that some of the B cells were induced to differentiate when IL-3 and CpG-A were used as basal stimuli, and after IL-21 or IFNα was added on this basis, both further stimulated B cell differentiation (A and B of FIG. 27), but none of IL-3, CpG-A, and IL-21 improved overall cell viability; however, after IFNα was added, the cell viability increased (C of FIG. 27).

### 2) Conditions for basal in vitro stimulation of B cells

B cells were seeded in a 96-well plate at a density of 1 × 10⁵/well and stimulated with 1 µg/mL CpG-B, 1 µg/mL R848, 500 U/mL IFNα, 500 ng/mL recombinant human BAFF, 500 ng/mL recombinant human APRIL, and 3 µg/mL retinoic acid (RA). After 6 days, the plasma cell (CD27⁺ CD38⁺) differentiation proportion was determined by flow cytometry.

The results show that CpG-B/R848 + IFNα effectively stimulated B cell differentiation (A and C of FIG. 28), while CpG-B/R848 + BAFF + APRIL did not effectively stimulate B cell differentiation under these conditions but only increased cell viability (B of FIG. 28). Based on CpG-B/R848 + IFNα, CpG-B/R848 + IFNα + BAFF + APRIL + RA showed a tendency to further promote B cell differentiation (A of FIG. 28 and FIG. 28C).

### 3) Detection time and detection window for in vitro differentiation of B cells

Take CpG-B + IFNα-induced differentiation as an example. B cells were seeded in a 96-well plate at a density of 1 × 10⁵/well and stimulated with 1 µg/mL CpG-B and 500 U/mL IFNα for 4 days. In this process, the cells were treated with 100 nM anifrolumab or control IgG on days 0 to 4. After 4 days, the plasma cell (CD27⁺ CD38⁺) differentiation proportion was determined by flow cytometry.

The results show that when the induction time was set to 4 days, IFNα did not induce B cell differentiation by itself, but it promoted B cell differentiation based on CpG-B induction; meanwhile, the control antibody anifrolumab inhibited the effect of IFNα, with a detection window and an inhibition rate of 75% (FIG. 29).

### Example 24. Evaluation Mode for Using In Vitro B Cell Differentiation Experiment to Screening for IFNAR1 Antagonists

Take CpG-B + IFNα-induced differentiation as an example. B cells were seeded in a 96-well plate at a density of 1 × 10⁵/well and stimulated with 2 µg/mL CpG-B and 250 U/mL IFNα for 4 days. In this process, the cells were treated with gradient concentrations of anifrolumab on days 0 to 4. On day 4, the plasma cell (CD27⁺ CD38⁺) differentiation proportion and count were determined by flow cytometry. Repeated experiments showed that the method has good repeatability and good stability. Table 16 shows the results of two of the experiments: experiment 1 and experiment 2.

The results show that in this screening method, anifrolumab inhibited the differentiation of B cells into plasmablasts in a dose-dependent manner (FIG. 30), indicating that this screening method can be used for screening for molecules of such a type as IFNAR1 antagonists. This also indicates that the concentrations of CpG-B and IFNα in this screening method are adjustable within a certain range.

**Table 16. IFNα-induced B cell differentiation results**

| Drug concentration (nM) | CD27+ CD38+ plasma cell proportion (%) | | | |
|---|---|---|---|---|
| | Anifrolumab | | IgG1 | |
| | Experiment 1 | Experiment 2 | Experiment 1 | Experiment 2 |
| 1000 | 0.59 | 0.54 | 24.1 | 23.6 |
| 200 | 2.92 | 2.44 | 23.8 | 23.7 |
| 40 | 4.89 | 4.17 | 22.8 | 23.7 |
| 8 | 9.08 | 6.90 | 24.2 | 24.5 |
| 1.6 | 14.90 | 13.80 | 23.3 | 23.7 |
| 0.32 | 20.00 | 20.00 | 23.4 | 24.8 |
| 0.064 | 22.70 | 23.20 | 24.0 | 24.0 |

### Example 25. Evaluation Mode for Using In Vitro B Cell Differentiation Experiment to Screening for BAFF/ARPIL Antagonists

To determine conditions for BAFF/ARPIL inducing plasma cell production, an *in vitro* plasma cell production functional experimental method was established.

### 1) IL-3 + CpG-A + IFNα

B cells were seeded in a 96-well plate at a density of 1 × 10⁵/well. 10 ng/mL recombinant human IL-3 and 0.5 µM CpG-A were added, and 1000 U/mL IFNα or 4 nM recombinant human BAFF and 3 nM recombinant human APRIL control were further added for stimulation. After 6 days, the plasma cell (CD27⁺ CD38⁺) differentiation proportion was determined by flow cytometry.

The results show that some of the B cells were induced to differentiate into plasma cells when IL-3 and CpG-A were used as basal stimuli, but the proportion was relatively low; after IFNα was added on this basis, B cell differentiation was further stimulated (A and B of FIG. 31). However, on the basis of IL-3, CpG-A or IL-3, CpG-A, and IFNα, adding BAFF + APRIL resulted in a relatively small improvement in the capability to induce B cell differentiation (B in FIG. 31).

### 2) CpG-B + IFNα

B cells were seeded in a 96-well plate at a density of 1 × 10⁵/well and stimulated with 2 µg/mL CpG-B for 4 days. On day 4, 250 U/mL IFNα or 500 ng/mL recombinant human BAFF and 50 ng/mL recombinant human APRIL were added, and the cells were stimulated with them for 3 days. On days 7, 10, 14, and 18, half of the culture medium was replaced, and the concentrations of recombinant human BAFF and recombinant human APRIL in the culture medium were adjusted to 50 ng/mL and 500 ng/mL. On days 7, 10, 14, and 21, plasma cells (CD27⁺ CD38⁺) were counted by flow cytometry. The results show that CpG-B + BAFF + ARPIL cannot induce *in vitro* production of plasma cells under this condition. CpG-B + IFNα induced *in vitro* production of plasma cells, but BAFF + APRIL did not further induce *in vitro* production of plasma cells on this basis (FIG. 32). Meanwhile, the detection window for plasma cell production was relatively large before day 10 and became small after day 14.

### 3) CpG-B + IFNα as early-stage stimulation condition, and IL-6 as late-stage stimulation condition

BAFF/APRIL acts on late stages of plasma cell production. B cells were seeded in a 96-well plate at a density of 1 × 10⁵/well and stimulated under any one of conditions a)-d) below, and plasmablasts (CD27⁺ CD38⁺) were counted by flow cytometry on days 7, 11, and 12.
a) The cells were stimulated with 2 µg/mL CpG-B and 250 U/mL IFNα for 4 days. On day 4, CpG-B and IFNα were removed, and the cells were stimulated with 10 ng/mL recombinant human IL-6, 500 ng/mL recombinant human BAFF, and 50 ng/mL recombinant human APRIL for 3 days. On days 7 and 11, half of the culture medium was replaced, and the concentrations of recombinant human BAFF and recombinant human APRIL in the culture medium were adjusted to 50 ng/mL and 500 ng/mL.
b) The cells were stimulated with 2 µg/mL CpG-B for 4 days. On day 4, CpG-B was removed, and the cells were further stimulated with 10 ng/mL recombinant human IL-6.
c) The cells were stimulated with 2 µg/mL CpG-B and 250 U/mL IFNα for 4 days. On day 4, CpG-B and IFNα were removed, and the cells were further stimulated with 10 ng/mL recombinant human IL-6.
d) The cells were stimulated with 2 µg/mL CpG-B for 4 days. On day 4, CpG-B was removed, and the cells were stimulated with 10 ng/mL recombinant human IL-6, 500 ng/mL recombinant human BAFF, and 50 ng/mL recombinant human APRIL for 3 days. On days 7 and 11, half of the culture medium was replaced, and the concentrations of recombinant human BAFF and recombinant human APRIL in the culture medium were adjusted to 50 ng/mL and 500 ng/mL.

The results show that with CpG-B as an early-stage basal stimulus, IL-6 + BAFF + APRIL did not induce plasma cell production in late stages by themselves under this condition, nor did they further induce plasma cell production on the basis of early-stage IFNα induction (FIG. 33).

### 4) CpG-B as early-stage stimulation condition, and IL-6 + IL-10 + IL-21 as late-stage stimulation condition

B cells were seeded in a 96-well plate at a density of 1 × 10⁵/well and stimulated with 2 µg/mL CpG-B (Invivogen cat: tlrl-2006) for 4 days. On day 4, CpG-B was removed, and the cells were stimulated with 10 ng/mL recombinant human IL-6, 50 ng/mL recombinant human IL-10, 5 ng/mL recombinant human IL-21, 500 ng/mL recombinant human BAFF, and 50 ng/mL recombinant human APRIL for 3 days. On day 7, half of the culture medium was replaced, and the concentrations of recombinant human BAFF and recombinant human APRIL in the culture medium were adjusted to 50 ng/mL and 500 ng/mL. On day 10, plasmablasts (CD27⁺ CD38⁺) were counted by flow cytometry. The results show that with CpG-B as an early-stage basal stimulus, IL-6 + IL-10 + BAFF + APRI and 5 ng/mL IL-21 cannot induce plasma cell production in late stages (FIG. 34).

### 5) CpG-B as early-stage stimulation condition, and IL-6 + IL-10 + IL-21 as late-stage stimulation condition

B cells were seeded in a 96-well plate at a density of 1 × 10⁵/well and stimulated with 2 µg/mL CpG-B for 5 days. On day 5, CpG-B was removed, and the cells were stimulated with 10 ng/mL recombinant human IL-6, 50 ng/mL recombinant human IL-10, 50 ng/mL recombinant human IL-21, 500 ng/mL recombinant human BAFF, and 50 ng/mL recombinant human APRIL for 2 days. On day 7, half of the culture medium was replaced, and the concentrations of recombinant human BAFF and recombinant human APRIL in the culture medium were adjusted to 50 ng/mL and 500 ng/mL. On days 9, 10, and 11, plasmablasts (CD27⁺ CD38⁺) were counted by flow cytometry.

The results show that with CpG-B as an early-stage basal stimulus, IL-6 + IL-10 + BAF + APRIL and 50 ng/mL IL-21 induced plasma cell production in late stages, and there were detection windows on days 9 to 11, with the number of detection windows tending to gradually increase (FIG. 35).

### 6) CpG-B as early-stage stimulation condition, and IL-6 + IL-10 + IL-21 as late-stage stimulation condition

B cells were seeded in a 96-well plate at a density of 1 × 10⁵/well and stimulated with 2 µg/mL CpG-B for 4 days. On day 4, CpG-B was removed, and the cells were stimulated with 10 ng/mL recombinant human IL-6, 50 ng/mL recombinant human IL-10, 50 ng/mL recombinant human IL-21, 500 ng/mL recombinant human BAFF, and 50 ng/mL recombinant human APRIL for 3 days. On day 7, half of the culture medium was replaced, and the concentrations of recombinant human BAFF and recombinant human APRIL in the culture medium were adjusted to 50 ng/mL and 500 ng/mL. On day 10, plasmablasts (CD27⁺ CD38⁺) were counted by flow cytometry.

The *in vitro* cell model was used to screen test fusion proteins. Specifically, test fusion proteins such as telitacicept, at gradient concentrations, were added on days 4 to 10. The results are shown in FIG. 23. The results show that in this screening method, telitacicept inhibited the *in vitro* production of plasma cells in a dose-dependent manner (FIG. 23), indicating that this screening method can be used for screening for BAFF/APRIL-targeting drugs such as TACI-Fc molecules or other similar drugs (e.g., telitacicept, B637302, and B606401).

Repeated experiments showed that the method has good repeatability and good stability. Table 17 shows the results of two of the experiments: experiment 1 and experiment 2.

**Table 17. BAFF + APRIL-induced plasma cell production data**

| Drug concentration (nM) | CD27+ CD38+ plasma cell count | | | | | | | |
|---|---|---|---|---|---|---|---|---|
| | IgG1 | | Telitacicept | | B637302 | | B606401 | |
| | Experiment 1 | Experiment 2 | Experiment 1 | Experiment 2 | Experiment 1 | Experiment 2 | Experiment 1 | Experiment 2 |
| 10 | 6837 | 5738 | 2030 | 1939 | 1939 | 2030 | 2500 | 2448 |
| 100 | 5711 | 5767 | 1854 | 1616 | 1616 | 1854 | 1440 | 1811 |
| 1000 | 5688 | 5174 | 580 | 501 | 501 | 580 | 1166 | 1144 |

### 7) CpG-B + IFNα as early-stage stimulation condition, and IL-6 + IL-10 + IL-21 as late-stage stimulation condition

B cells were seeded in a 96-well plate at a density of 1 × 10⁵/well and stimulated with 2 µg/mL CpG-B and 250 U/mL IFNα for 5 days. On day 5, CpG-B and IFNα were removed, and the cells were stimulated with 10 ng/mL recombinant human IL-6, 50 ng/mL recombinant human IL-10, different concentrations (15, 25, or 40 ng/mL) of recombinant human IL-21, 500 ng/mL recombinant human BAFF, and 50 ng/mL recombinant human APRIL (R&D cat: 5860-AP-010/CF) for 2 days. On day 7, half of the culture medium was replaced, and the concentrations of recombinant human BAFF and recombinant human APRIL in the culture medium were adjusted to 50 ng/mL and 500 ng/mL. On day 10, plasmablasts (CD27⁺ CD38⁺) were counted by flow cytometry. The results show that with CpG-B + IFNα as early-stage basal stimuli, IL-6 + IL-10 + BAF + APRIL and 15 ng/mL IL-21 synergistically induced plasma cell production, while 25 or 40 ng/mL IL-21 did not synergistically induced plasma cell production (FIG. 36).

Information about the reagents used above is shown below: IL-3 (R&D cat: 203-IL-010/CF), CpG-A (Invivogen cat: tlrl-2216), CpG-B (Invivogen cat: tlrl-2006), IFNα (Biolegend cat: 592704), human BAFF (R&D cat: 7537-BF-025/CF), APRIL (R&D cat: 5860-AP-010/CF), IL-10 (Peprotech cat: 200-10), IL-21 (Peprotech cat: AF-200-21-10), R848 (Invivogen cat: tlrl-r848), and retinoic acid (RA, Sigma-Aldrich cat: R2625).

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

## Claims

1. A screening method for an immunomodulator, comprising the following steps:
step 1) administering to B cells a TLR agonist and culturing for M days, wherein M is selected from an integer of 1-28;
step 2) removing the TLR agonist;
step 3) adding one or more interleukins and one or more TNF family members and culturing for N days, wherein N is selected from an integer of 1-28;
step 4) adjusting concentrations of the TNF family members and continuing culturing;
step 5) adding a test sample; and
step 6) determining a B cell differentiation level;
wherein the step 5) is performed concurrently with, during, or after any one of the steps 1) to 4) described above.

2. A screening method for an immunomodulator, comprising the following steps:
step 1) administering to B cells a TLR agonist and an IFN and culturing for M days, wherein M is selected from an integer of 1-28;
step 2) removing the TLR agonist and the IFN;
step 3) adding one or more interleukins and one or more TNF family members and culturing for N days, wherein N is selected from an integer of 1-28;
step 4) adjusting concentrations of the TNF family members and continuing culturing;
step 5) adding a test sample; and
step 6) determining a B cell differentiation level;
wherein the step 5) is performed concurrently with, during, or after any one of the steps 1) to 4) described above.

3. The method according to claim 1 or 2, further comprising:
step 7) selecting a test sample capable of activating or inhibiting B cell differentiation as an immunomodulator, wherein optionally, the step 6) comprises determining a CD27⁺ CD38⁺ B cell percentage to determine the B cell differentiation level.

4. The method according to any one of claims 1-3, wherein:
the TLR agonist is selected from any one or more of TLR7, TLR8, and TLR9 agonists;
preferably, the TLR agonist is selected from any one or more of R848, CpG, and LPS;
preferably, the TLR agonist is CpG-A or CpG-B; more preferably, the TLR agonist is CpG-B;
when present, the IFN is IFNα or IFNβ, preferably IFNα;
the interleukin is selected from one or more of IL-3, IL-6, IL-10, and IL-21; preferably, the interleukin is a combination of IL-6, IL-10, and IL-21;
the TNF family members are selected from one or more of BAFF and APRIL;
preferably, the TNF family members are a combination of BAFF and APRIL.

5. The method according to any one of claims 1 to 3, wherein when the TNF family members are BAFF and APRIL, adjusting the concentrations of the TNF family members comprises reducing a BAFF concentration and/or increasing an APRIL concentration;
preferably, a BAFF concentration in step 4) is reduced to be 0.01-0.9 times a BAFF concentration added in step 3); more preferably, a BAFF concentration in step 4) is reduced to be 0.02-0.5 times a BAFF concentration added in step 3); more preferably, a BAFF concentration in step 4) is reduced to be about 0.02, about 0.04, about 0.05, about 0.06, about 0.07, about 0.08, about 0.09, about 0.1, about 0.11, about 0.12, about 0.13, about 0.14, about 0.15, about 0.2, about 0.3, about 0.4, about 0.5, about 0.6, about 0.7, about 0.8, or about 0.9 times a BAFF concentration added in step 3);
preferably, an APRIL concentration in step 4) is increased to be 1.1-100 times an APRIL concentration added in step 3); more preferably, an APRIL concentration in step 4) is increased to be 5-50 times an APRIL concentration added in step 3); more preferably, an APRIL concentration in step 4) is increased to be about 1.5, about 2, about 3, about 4, about 5, about 6, about 7, about 8, about 9, about 10, about 12, about 15, about 20, about 25, about 30, about 35, about 40, or about 50 times an APRIL concentration added in step 3).

6. The method according to any one of the preceding claims, comprising:
step 1) administering to the B cells 0.1-10 µg/mL CpG-B or CpG-A or R848 and culturing for 2 to 8 days;
step 2) removing CpG-B or CpG-A or R848;
step 3) adding 2-100 ng/mL IL-6, 10-1000 ng/mL IL-10, and 10-200 ng/mL IL-21, as well as 10-2000 ng/mL BAFF and 10-2000 ng/mL APRIL, and culturing for 2-5 days;
step 4) reducing the BAFF concentration and increasing the APRIL concentration;
step 5) adding a test sample on any one day of culturing the B cells and assessing B cell differentiation; and
step 6) selecting a test sample capable of activating or inhibiting B cell differentiation as an immunomodulator;
preferably,
step 1) administering to the B cells 0.5-5 µg/mL CpG-B on day 0;
step 2) removing CpG-B on days 3-5;
step 3) adding 10-50 ng/mL IL-6 and 20-200 ng/mL IL-10 and 20-200 ng/mL IL-21,
adding 200-1000 ng/mL BAFF and 20-200 ng/mL APRIL, and then stimulating for 2-4 days;
step 4) reducing the BAFF concentration to 20-200 ng/mL and increasing the APRIL concentration to 200-1000 ng/mL;
step 5) adding a test sample on days 0-21 and assessing B cell differentiation; and
step 6) selecting a test sample capable of activating or inhibiting B cell differentiation as an immunomodulator;
and more preferably,
step 1) administering to the B cells 1-2 µg/mL CpG-B on day 0;
step 2) removing CpG-B after 4 or 5 days;
step 3) adding about 10 ng/mL IL-6 and about 50 ng/mL IL-10 and about 50 ng/mL IL-21 and adding about 500 ng/mL BAFF and about 50 ng/mL APRIL;
step 4) after 2 or 3 or 4 days, adjusting the BAFF concentration to about 50 ng/mL and
adjusting the APRIL concentration to about 500 ng/mL;
step 5) adding a test sample on days 0-21 and assessing B cell differentiation; and
step 6) selecting a test sample capable of activating or inhibiting B cell differentiation as an immunomodulator.

7. The method according to any one of the preceding claims, comprising:
step 1) administering to the B cells 0.1-10 µg/mL CpG-B or CpG-A or R848 and 50-1000 U/mL IFNα and culturing for 2-8 days;
step 2) removing CpG-B or CpG-A or R848 and removing IFNα;
step 3) adding 2-100 ng/mL IL-6 and 10-1000 ng/mL IL-10 and 10-200 ng/mL IL-21, as well as 10-2000 ng/mL BAFF and 10-2000 ng/mL APRIL, and culturing for 2-5 days;
step 4) reducing the BAFF concentration and increasing the APRIL concentration;
step 5) adding a test sample on days 0-21 and assessing B cell differentiation; and
step 6) selecting a test sample capable of activating or inhibiting B cell differentiation as an immunomodulator;
preferably,
step 1) administering to the B cells 0.5-5 µg/mL CpG-B and 100-500 U/mL IFNα on day 0 to stimulate the B cells;
step 2) removing CpG-B and IFNα on days 3-5;
step 3) adding 10-50 ng/mL IL-6 and 20-200 ng/mL IL-10 and 5-22 ng/mL IL-21, adding 200-1000 ng/mL BAFF and 20-200 ng/mL APRIL, and then stimulating for 2-4 days;
step 4) reducing the BAFF concentration to 20-200 ng/mL and increasing the APRIL concentration to 200-1000 ng/mL;
step 5) adding a test sample on days 0-21 and assessing B cell differentiation; and
step 6) selecting a test sample capable of activating or inhibiting B cell differentiation as an immunomodulator;
and more preferably,
step 1) administering to the B cells about 1-2 µg/mL CpG-B and about 250-500 U/mL IFNα on day 0;
step 2) removing CpG-B and IFNα after 4 or 5 days;
step 3) adding about 10 ng/mL IL-6 and about 50 ng/mL IL-10 and about 15 ng/mL IL-21 and adding about 500 ng/mL BAFF and about 50 ng/mL APRIL;
step 4) after 2 or 3 or 4 days, adjusting the BAFF concentration to about 50 ng/mL and
adjusting the APRIL concentration to about 500 ng/mL;
step 5) adding a test sample on days 0-21 and assessing B cell differentiation; and
step 6) selecting a test sample capable of activating or inhibiting B cell differentiation as an immunomodulator.

8. A screening method for an immunomodulator, comprising:
1) administering to B cells a TLR agonist and an interferon;
2) adding a test sample on any one of days 0-21 and continuing culturing; and
3) determining a B cell differentiation level;
wherein preferably, the method further comprises:
4) selecting a test sample capable of activating or inhibiting B cell differentiation as an immunomodulator.

9. The method according to claim 8, wherein:
the TLR agonist is selected from one or more of TLR7, TLR8, and TLR9 agonists;
preferably, the TLR agonist is selected from one or more of R848 and CpG; preferably, the TLR agonist is CpG-A or CpG-B; more preferably, the TLR agonist is CpG-B;
the IFN is IFNα or IFNβ, preferably IFNα.

10. The method according to claim 8 or 9, comprising:
1) administering to the B cells 0.1-20 µg/mL CpG-B and 50-1000 U/mL IFNα;
2) adding a test sample on any one of days 0-21 and continuing culturing; and
3) determining a B cell differentiation level;
wherein preferably, the method further comprises:
4) selecting a test sample capable of activating or inhibiting B cell differentiation as an immunomodulator;
preferably, the method comprises step 1) administering to the B cells 0.5-5 µg/mL CpG-B and 100-800 U/mL IFNα;
more preferably, the method comprises step 1) administering to the B cells about 1-2 µg/mL CpG-B and 250-500 U/mL IFNα.

11. The method according to any one of the preceding claims, wherein the immunomodulator is a type I interferon pathway modulator and/or a TNF pathway modulator;
preferably, the type I interferon pathway modulator is an IFNAR1 signaling pathway modulator;
preferably, the TNF pathway modulator is a BAFF and/or ARRIL pathway modulator.

12. The method according to any one of the preceding claims, wherein activating or inhibiting B cell differentiation is achieved by determining a number or proportion of plasma cells differentiated from the B cells, and the plasma cells are preferably CD27⁺ CD38⁺ B cells.

13. The method according to any one of the preceding claims, wherein the B cells are at a concentration of 1 × 10⁵/well in a 96-well plate.

14. The method according to any one of the preceding claims, wherein the B cells are obtained by the following steps:
1) separating B cells from PBMCs, and
2) culturing in a 1640 culture medium containing 50 µM β-mercaptoethanol, 100 µM MEM non-essential amino acids, and sodium pyruvate, the 1640 culture medium containing GlutaMAX.

15. The method according to any one of the preceding claims, wherein the immunomodulator is selected from a protein or polypeptide, a nucleic acid, an aptamer, a small-molecule compound, and a molecule comprising the protein or polypeptide, the nucleic acid, the aptamer, or the small-molecule compound; the protein or polypeptide is preferably an antibody or an antigen-binding fragment thereof, a cytokine, a receptor, or a ligand;
preferably, the immunomodulator is used for treating a B cell disorder or an autoimmune disease;
more preferably, the B cell disorder or the autoimmune disease is a disease or disorder associated with TACI and/or BCMA expression;
more preferably, the autoimmune disease is selected from systemic lupus erythematosus, myasthenia gravis, multiple sclerosis, insulin-dependent diabetes mellitus, Crohn's disease, rheumatoid arthritis, polyarticular juvenile rheumatoid arthritis, and psoriatic arthritis; the B cell disorder is selected from tumors, chronic leukocytic leukemia, multiple myeloma, non-Hodgkin lymphoma, post-transplant lymphoproliferative disorder, and light chain gammopathy.

16. A method for preparing or producing an immunomodulator, comprising a step of screening for the immunomodulator by using the method according to any one of claims 1-15.

17. A pharmaceutical composition, comprising the immunomodulator according to any one of claims 1 to 16 and one or more pharmaceutically acceptable carriers, diluents, or excipients.

18. A preparation or production method for a pharmaceutical composition, comprising a step of mixing the immunomodulator according to any one of claims 1-16 with one or more pharmaceutically acceptable carriers, diluents, or excipients, or comprising the steps of screening for an immunomodulator according to any one of claims 1-14, or the step of preparing or producing an immunomodulator according to claim 16, and a step of mixing the resulting immunomodulator with one or more pharmaceutically acceptable carriers, diluents, or excipients.

19. A method for treating or ameliorating a disease or disorder with the immunomodulator in the method according to any one of claims 1 to 16 or the pharmaceutical composition according to claim 17, comprising administering to a subject in need thereof the immunomodulator in the method according to any one of claims 1 to 16 or the pharmaceutical composition according to claim 17, wherein:
preferably, the disease or disorder is a B cell disorder or an autoimmune disease;
more preferably, the B cell disorder or the autoimmune disease is a disease or disorder associated with TACI and/or BCMA expression;
more preferably, the autoimmune disease is selected from systemic lupus erythematosus, myasthenia gravis, multiple sclerosis, insulin-dependent diabetes mellitus, Crohn's disease, rheumatoid arthritis, polyarticular juvenile rheumatoid arthritis, and psoriatic arthritis; the B cell disorder is selected from tumors, chronic leukocytic leukemia, multiple myeloma, non-Hodgkin lymphoma, post-transplant lymphoproliferative disorder, and light chain gammopathy.

20. A method for inducing B cell differentiation, comprising a step of administering to B cells a TLR agonist and an IFN,
wherein the TLR agonist is one or more of TLR7, TLR8, and TLR9 agonists; preferably, the TLR agonist is one or more of R848 or CpG; preferably, the TLR agonist is CpG-A or CpG-B; more preferably, the TLR agonist is CpG-B;
the IFN is IFNα or IFNβ, preferably IFNα.

21. The method according to claim 19, comprising:
administering to B cells CpG-A and IFNα, or
a step of administering to B cells CpG-B or R484 and IFNα,
wherein optionally, the method comprises:
administering to B cells CpG-A and IFNα, or a step of administering to B cells CpG-B or R484 and IFNα, and
a step of administering to the B cells IL-3 and IL-21 simultaneously.

22. The method according to claims 20 to 21, comprising the following step:
administering to the B cells 5-20 ng/mL IL-3, 0.2-1 µM CpG-A, 500-2000 U/mL IFNα, and 16.5-66 ng/mL IL-21, or
administering to the B cells 0.5-5 µg/mL CpG-B or R484 and 100-800 U/mL IFNα;
preferably,
administering to the B cells about 10 ng/mL IL-3, about 0.5 µM CpG-A, about 1000 U/mL IFNα, and about 33 ng/mL IL-21, or
administering to the B cells 1-2 µg/mL CpG-B or R484 and 250-500 U/mL IFNα.

23. The method according to any one of claims 20 to 22, further comprising adding retinoic acid to the B cells at the same time, wherein preferably, the retinoic acid is at a concentration of 2-5 µg/mL; more preferably, the retinoic acid is at a concentration of about 3 µg/mL.

24. A cell model for screening for an immunomodulator, comprising B cells induced to differentiate by the method according to any one of claims 20-23.
